(19) Europäisches Patentamt / European Patent Office / Office européen des brevets

(11) **EP 4 549 013 A1**

## EUROPEAN PATENT APPLICATION

(43) Date of publication:
**07.05.2025 Bulletin 2025/19**

(21) Application number: **23461676.1**

(22) Date of filing: **02.11.2023**

(51) International Patent Classification (IPC):
**B01J 20/281** (2006.01)   **B01D 15/00** (2006.01)
**B01J 20/283** (2006.01)   **B01J 20/292** (2006.01)
**B01J 20/30** (2006.01)   **C12N 15/10** (2006.01)

(52) Cooperative Patent Classification (CPC):
**B01J 20/281; B01D 15/00; B01J 20/283;
B01J 20/292; B01J 20/3085; C12N 15/1013**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Curiosity Diagnostics sp. z o.o
01-796 Warsaw (PL)**

(72) Inventor: **Brzyzek, Grzegorz**
**01-796 Warsaw (PL)**

(74) Representative: **Witek, Rafal**
**WTS Patent Attorneys**
**Witek, Sniezko & Partners**
**Ul. Rudolfa Weigla 12**
**53-114 Wroclaw (PL)**

(54) **STABLE PARTICLE COMPOSITIONS**

(57)    Provided herein are compositions comprising a plurality of particles, methods of preparing compositions comprising a plurality of particles, as well as uses, kits and articles of manufacture related thereto.

EP 4 549 013 A1

**Description**

FIELD

The present disclosure relates to compositions comprising particles, as well as methods, uses, kits and articles of manufacture related thereto.

BACKGROUND

Particles, such as microbeads, are widely used in research, diagnostics, industry, medicine, and other fields to bind ligands, lyse cells, homogenize tissues, localize or isolate molecules (e.g., on arrays), perform on-bead reactions, as well as many other uses. For example, silica beads are commonly used for binding and isolating DNA or RNA.

Particles are typically provided as dried or lyophilized compositions, or in aqueous suspensions. Drying or lyophilization of particles, however, can result in aggregation or degradation of the particles, as well as issues with resuspension. Aqueous suspensions of particles can allow for particle settling, which can lead to aggregation and clumping, require constant mixing to ensure consistency during use, and may lead to bead stability issues.

Accordingly, there is a need in the art for compositions that support and/or enhance particle stability, functionality, storage, and handling.

All references cited herein, including patent applications and publications, are hereby incorporated by reference in their entirety.

SUMMARY

In certain aspects, provided herein are compositions comprising a plurality of particles. In some embodiments, the compositions comprise a plurality of particles and one, two or all three of: a mobile phase, a viscosity enhancing agent, and a solubilizing agent. In some embodiments, the compositions comprise a plurality of particles and a mobile phase. In some embodiments, the compositions comprise a plurality of particles and a viscosity enhancing agent. In some embodiments, the compositions comprise a plurality of particles and a solubilizing agent. In some embodiments, the compositions comprise a plurality of particles, a mobile phase and a viscosity enhancing agent. In some embodiments, the compositions comprise a plurality of particles, a mobile phase, and a solubilizing agent. In some embodiments, the compositions comprise a plurality of particles, a viscosity enhancing agent and a solubilizing agent. In some embodiments, the mobile phase comprises a suspending agent.

In some embodiments, the compositions comprise a plurality of particles and (a) a mobile phase comprising a suspending agent; (b) a viscosity enhancing agent; and (c) a solubilizing agent.

In some embodiments, the mobile phase is at a concentration of between about 60% and about 99%, between about 65% and about 95%, between about 70% and about 90%, between about 75% and about 90%, or between about 80% and about 90% weight by weight (w/w). In some embodiments, the mobile phase is at a concentration of about 70%, about 80%, or about 90% w/w. In some embodiments, the mobile phase is at a concentration of about 80% w/w. In some embodiments, the suspending agent is a sugar alcohol, a glycol, a water-miscible polymer, an aprotic solvent, or any combination thereof. In some embodiments, the sugar alcohol is glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, or any combination thereof. In some embodiments, the sugar alcohol is glycerol. In some embodiments, the glycerol is at a concentration of about 80% w/w in the composition. In some embodiments, the glycol is diethylene glycol, ethylene glycol, hexylene glycol, propylene glycol, dipropylene glycol, triethylene glycol, tripropylene glycol, a glycol ether, or any combination thereof. In some embodiments, the glycol is propylene glycol. In some embodiments, the water-miscible polymer is an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, or any combination thereof. In some embodiments, the water-miscible polymer is a polyethylene glycol (PEG) polymer. In some embodiments, the water-miscible polymer comprises an average molecular weight of about 1000 g/mol or less, about 900 g/mol or less, about 800 g/mol or less, about 700 g/mol or less, about 600 g/mol or less, about 500 g/mol or less, about 400 g/mol or less, about 300 g/mol or less, about 200 g/mol or less, or about 100 g/mol or less. In some embodiments, the water-miscible polymer comprises an average molecular weight of between about 100 g/mol and about 1000 g/mol, between about 200 g/mol and about 600 g/mol, between about 200 g/mol and about 400 g/mol, or between about 200 g/mol and about 300 g/mol. In some embodiments, the water-miscible polymer comprises an average molecular weight of about 200 g/mol or about 300 g/mol. In some embodiments, the water-miscible polymer is PEG 200 and/or PEG 300. In some embodiments, the aprotic solvent is a polar aprotic solvent, optionally a dipolar aprotic solvent. In some embodiments, the aprotic solvent is dimethylsulfoxide (DMSO), dimethylsulfone, diphenylsulfone (DPS), diethylsulfoxide, diethylsulfone, diisopropylsulfone, sulfolane, tetrahydrothiophene-1 -monoxide, N,N-dimethylacetamide (DMAc), N,N-dimethylformamide (DMF), N-methyl-2-pyrrolidinone (NMP), or any combination thereof. In some embodiments, the aprotic solvent is sulfolane.

In some embodiments, the viscosity enhancing agent is at least partially soluble in the mobile phase. In some embodiments, the viscosity enhancing agent is at a concentration of between about 1% and about 40%, between about 1% and about 35%, between about 1% and about 30%, between about 1% and about 25%, between about 1% and about 20%, between about 1% and about 15%, between about 1% and about 10%, or between about 1% and about 5% w/w. In some embodiments, the viscosity enhancing agent is at a concentration of about 5% w/w. In some embodiments, the viscosity enhancing agent comprises a water-miscible polymer. In some embodiments, the water-miscible polymer comprises an average molecular weight of at least about 2000 g/mol, at least about 4000 g/mol, at least about 5000 g/mol, at least about 6000 g/mol, at least about 7000 g/mol, at least about 8000 g/mol, at least about 9000 g/mol, at least about 10000 g/mol, at least about 12000 g/mol, at least about 14000 g/mol, at least about 16000 g/mol, at least about 18000 g/mol, at least about 20000 g/mol, at least about 25000 g/mol, or at least about 30000 g/mol. In some embodiments, the water-miscible polymer comprises an average molecular weight of between about 2000 g/mol and about 30000 g/mol, between about 4000 g/mol and about 20000 g/mol, between about 4000 g/mol and about 10000 g/mol, or between about 4000 g/mol and about 8000 g/mol. In some embodiments, the water-miscible polymer comprises an average molecular weight of about 4000 g/mol, about 8000 g/mol, about 10000 g/mol, or about 20000 g/mol. In some embodiments, said water-miscible polymer is an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, or any combination thereof. In some embodiments, the water-miscible polymer comprises a polyethylene glycol (PEG) polymer. In some embodiments, the PEG polymer is PEG 4000, PEG 8000, PEG 10000, PEG 20000, or any combination thereof. In some embodiments, the PEG polymer is PEG 8000. In some embodiments, the PEG 8000 is at a concentration of about 5% w/w in the composition. In some embodiments, the viscosity enhancing agent comprises polyvinyl alcohol (PVA). In some embodiments, the viscosity enhancing agent comprises sulfone or a sulfone polymer.

In some embodiments, the solubilizing agent is at least partially soluble in the mobile phase. In some embodiments, the solubilizing agent is water-soluble. In some embodiments, the solubilizing agent is at a concentration of between about 1% and about 40%, between about 5% and about 30%, between about 10% and about 25%, or between about 10% and about 20% w/w. In some embodiments, the solubilizing agent is at a concentration of about 15% w/w. In some embodiments, the solubilizing agent comprises a chaotropic agent. In some embodiments, the chaotropic agent is n-butanol, ethanol, a guanidinium salt, lithium perchlorate, lithium acetate, magnesium chloride, phenol, 2-propanol, sodium dodecyl sulfate, thiourea, urea, a water-soluble urea derivative, or any combination thereof. In some embodiments, the chaotropic agent is a guanidinium salt. In some embodiments, the guanidium salt is guanidinium chloride, guanidinium sulfate, guanidine carbonate, guanidinium nitrate, guanidinium isothiocyanate, guanidinium thiocyanate, or any combination thereof. In some embodiments, the chaotropic agent is urea and/or a water-soluble urea derivative. In some embodiments, the urea and/or the water-soluble urea derivative is at a concentration of about 15% w/w in the composition. In some embodiments, the water-soluble urea derivative is thiourea, hydroxyurea, dimethylurea, or any combination thereof. In some embodiments, the solubilizing agent comprises a buffering agent. In some embodiments, the buffering agent is a MES, Bis-Tris, ADA, ACES, PIPES, MOPSO, Bis-Tris Propane, BES, MOPS, TES, HEPES, DIPSO, MOBS, TAPSO, Tris, HEPPSO, POPSO, TEA, EPPS, HEPPS, Tricine, Gly-Gly, Bicine, HEPBS, TAPS, AMPD, TABS, AMPSO, CHES, CAPSO, AMP, CAPS, CABS, phosphate, maleate, glycine, citrate, formate, succinate, acetate, propionate, pyridine, piperazine, cacodylate, histidine, ethanolamine, carbonate, imidazole, pyrophosphate, hydrazine, taurine (AES), borate, ammonium hydroxide, methylamine, piperidine, malate buffer, or any combination thereof. In some embodiments, the buffering agent comprises a TAPS, HEPES, or MOPS buffer, or any combination thereof.

In some embodiments, the suspending agent comprises glycerol, the viscosity enhancing agent comprises PEG 8000, and the solubilizing agent comprises urea. In some embodiments, the glycerol is at a concentration of about 80% w/w, the PEG 8000 is at a concentration of about 5% w/w, and the urea is at a concentration of about 15% w/w. In some embodiments, the ratio of glycerol:PEG 8000:urea in the composition is 16:1:3 w/w. In some embodiments, the pH of the composition is between about 7 and about 8.

In some embodiments, the composition has a viscosity of: between about 7000 millipascal-second (mPa·s) and about 8000 mPa s, between about 7250 mPa s and about 8000 mPa s, between about 7500 mPa s and about 8000 mPa s, or between about 7750 mPa s and about 8000 mPa s, when measured at a temperature of about 15°C; between about 5000 mPa s and about 6000 mPa s, between about 5250 mPa s and about 6000 mPa·s, between about 5500 mPa·s and about 6000 mPa·s, or between about 5500 mPa·s and about 5750 mPa s, when measured at a temperature of about 20°C; between about 5500 mPa s and about 7000 mPa s, between about 5750 mPa s and about 6750 mPa s, or between about 6000 mPa s and about 6500 mPa s, when measured at a temperature of about 25°C; between about 4250 mPa s and about 5500 mPa·s, between about 4500 mPa·s and about 5500 mPa·s, between about 4750 mPa·s and about 5250 mPa·s, or between about 4750 mPa·s and about 5000 mPa·s, when measured at a temperature of about 30°C; between about 2000 mPa·s and about 3000 mPa·s, between about 2250 mPa·s and about 2750 mPa·s, or between about 2250 mPa·s and about 2500 mPa·s, when measured at a temperature of about 35°C; between about 750 mPa·s and about 1750 mPa·s, between about 1000 mPa·s and about 1750 mPa s, or between about 1000 mPa s and about 1500 mPa s, when measured at a temperature of about 40°C; between about 500 mPa·s and about 1500 mPa·s, between about 750 mPa·s and about 1250 mPa·s, or between about 1000 mPa·s and about 1250 mPa·s, when measured at a temperature of about 45°C; between about 250 mPa·s and about 1500 mPa·s, between about 500 mPa·s and about 1250 mPa·s, or between about 500 mPa·s and about 1000 mPa·s, when measured at a temperature of about 50°C; between about 500 mPa·s and about 1500 mPa·s, between about 750 mPa·s and about 1250 mPa·s, or between about 1000 mPa s and about 1250 mPa s, when measured at a temperature of about 55°C; and/or between about 100 mPa s and about 600 mPa s, between about 200 mPa s and about 500 mPa s, or between about 200 mPa·s and about 400 mPa·s, when measured at a temperature of about 60°C. In some embodiments, viscosity of the composition is assessed using a rotational viscometer. In some embodiments, viscosity of the composition is assessed at a spindle rotational speed of about 10 revolutions per minute (RPM).

In some embodiments, the composition has a density of between about 0.5 g/mL and about 2 g/mL, between about 1 g/mL and about 2 g/mL, between about 1 g/mL and about 1.5 g/mL, or between about 1.1 g/mL and about 1.5 g/mL. In some embodiments, the composition has a density of about 1.3 g/mL. In some embodiments, density of the composition is assessed on an analytical balance.

In some embodiments, the mobile phase is non-aqueous or substantially non-aqueous. In some embodiments, the composition is non-aqueous or substantially non-aqueous. In some embodiments, the composition has a water content of about 1% or less. In some embodiments, water content is assessed by Karl Fischer titration.

In some embodiments, the composition is a liquid composition.

In some embodiments, the plurality of particles comprises one or more particles configured to bind a nucleic acid, a polypeptide, a lipid, a carbohydrate, or a small molecule. In some embodiments, the plurality of particles comprises one or more particles configured to bind DNA and/or RNA. In some embodiments, the plurality of particles comprises one or more microparticles, nanoparticles, microspheres, paramagnetic beads, magnetic beads, microbeads, nanobeads, or any combination thereof. In some embodiments, the plurality of particles comprises one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. In some embodiments, one or more particles of the plurality of particles is functionalized. In some embodiments, one or more particles are surface functionalized with a carboxyl, amino, hydroxyl, silica, streptavidin, endopeptidase enzyme moiety, amine, thiol, diol, polymer, an antibody or antibody fragment, a nucleic acid, an oligonucleotide, a peptide, a polypeptide, or any combination thereof. In some embodiments, the plurality of particles comprises magnetic silica beads. In some embodiments, the plurality of particles is present in the composition at a concentration of at least about 0.5%, at least about 1%, at least about 2.5%, at least about 5%, at least about 10%, at least about 15%, at least about 20%, at least about 25%, or at least about 30% weight by volume (w/v). In some embodiments, the plurality of particles is present in the composition at a concentration of between about 0.5% and about 30% w/v, between about 1% and about 25% w/v, between about 2.5% and about 20% w/v, or between about 5% and about 15% w/v. In some embodiments, the plurality of particles is present in the composition at a concentration of about 10% w/v.

In some embodiments, the composition enhances stability of particles in the plurality of particles. In some embodiments, the composition enhances stability of particles in the plurality of particles as compared to corresponding particles in an aqueous solution. In some embodiments, the plurality of particles in the composition is stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months. In some embodiments, particle stability is assessed based on particle integrity, particle aggregation, and/or retention of ability to bind to a ligand. In some embodiments, the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline. In some embodiments, the composition enhances ligand binding of particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution. In some embodiments, the plurality of particles comprises particles capable of binding to nucleic acid ligands. In some embodiments, stability of the particles is assessed based on nucleic acid ligand binding ability compared to baseline and/or compared to corresponding particles in an aqueous solution. In some embodiments, the nucleic acid ligands comprise DNA and/or RNA. In some embodiments, the plurality of particles comprises silica particles. In some embodiments, the plurality of particles comprises magnetic silica particles. In some embodiments, the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of nucleic acid ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline. In some embodiments, the composition enhances nucleic acid ligand binding by particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution. In some embodiments, particle stability is assessed at room temperature. In some embodiments, particle stability is assessed at a temperature of between about 20°C and about 30°C. In some embodiments, particle stability is assessed at a temperature of about 25°C. In some embodiments, particle stability is assessed under normal atmospheric conditions, in reduced oxygen conditions, or in nitrogen gas. In some embodiments, the composition is capable of adhering to a polypropylene surface. In some embodiments, the composition is microbiologically stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months.

In some embodiments, the composition further comprises an agent, such as an antioxidant, a reducing agent, a chelating agent, or any combination thereof. In some embodiments, the composition does not comprise an anti-microbial agent. In some embodiments, the composition comprises less than about 10% of surfactant or detergent. In some embodiments, the composition does not comprise a surfactant or detergent.

In another aspect, provided herein are methods for preparing a composition comprising a plurality of particles, comprising: (a) providing a plurality of particles; and (b) suspending the plurality of particles in a formulation comprising one, two or all three of: a mobile phase (e.g., a mobile phase comprising a suspending agent), a viscosity enhancing agent, and a solubilizing agent.

In another aspect, provided herein are methods for preparing a composition comprising a plurality of particles, comprising: (a) providing a plurality of particles; and (b) suspending the plurality of particles in a formulation comprising: (i) a mobile phase comprising a suspending agent; (ii) a viscosity enhancing agent; and (iii) a solubilizing agent.

In some embodiments, the plurality of particles provided in step (a) is dehydrated or lyophilized. In some embodiments, the method further comprises dehydrating or lyophilizing the plurality of particles prior to step (a).

In some embodiments, the dehydrating comprises suspending the plurality of particles in a first organic solvent. In some embodiments, the dehydrating comprises: (i) separating a plurality of particles stored in an aqueous solution from said aqueous solution, and (ii) suspending the separated plurality of particles in first volume of a first organic solvent. In some embodiments, the dehydrating further comprises: (1) separating the plurality of particles from the first organic solvent, and (2) resuspending the separated plurality of particles in a further volume of said first organic solvent. In some embodiments, the method comprises repeating steps (1) and (2) at least once, at least twice, or more.

In some embodiments, the dehydrating comprises: (i) separating a plurality of particles stored in an aqueous solution from said aqueous solution, and (ii) suspending the separated plurality of particles in a series of volumes of a first organic solvent, wherein the volumes in the series of volumes comprise increasing amounts of the first organic solvent, wherein a first volume of the series of volumes comprises an organic solvent concentration of between about 20% and about 80%, and the final volume of the series of volumes comprises an organic solvent concentration of about 100%. In some embodiments, the first volume of the series of volumes comprises an organic solvent concentration of about 50% and the final volume of the series of volumes comprises an organic alcohol concentration of about 100%. In some embodiments, the series of volumes comprises between about 2 and about 15 volumes, between about 2 and about 10 volumes, or between about 5 and about 7 volumes. In some embodiments, the first organic solvent comprises an alcohol, optionally wherein the alcohol is ethanol and/or isopropanol.

In some embodiments, the first organic solvent comprises an organic solvent miscible with water and/or with high solubility in water. In some embodiments, the first organic solvent comprises: a polar aprotic solvent, optionally wherein the polar aprotic solvent is DMSO, tetrahydrofuran (THF), acetonitrile, propylene carbonate, sulfolane, and any combination thereof; a water miscible P-type or E-type glycol ether, optionally wherein the first organic solvent comprises dipropylene glycol methyl ether and/or diethylene glycol ethyl ether; an alcohol, optionally wherein the alcohol is ethanol and/or isopropanol; tetrahydrothiophene 1-oxide; or any combination thereof. In some embodiments, the first organic solvent comprises a mixture of two or more solvents, optionally wherein the solvents are in about a 1:1 ratio, and further optionally wherein said mixture comprises: DMSO and ethanol; DMSO and isopropanol; or DMSO and THF. In some embodiments, the first organic solvent comprises DMSO. In some embodiments, the first organic solvent comprises an alcohol, optionally wherein the alcohol is ethanol and/or isopropanol.

In some embodiments, step (b) comprises: (i) separating the plurality of particles from a volume of the first organic solvent, and (ii) suspending the separated plurality of particles in a volume of said formulation.

In some embodiments, the method further comprises, prior to step (b), suspending the plurality of particles in a first volume of a solution comprising: a second organic solvent, and a suspending agent. In some embodiments, the method further comprises, prior to step (b): (i) separating the plurality of particles from a volume of the first organic solvent, and (ii) suspending the separated plurality of particles in a first volume of a solution comprising: a second organic solvent, and a suspending agent. In some embodiments, the method further comprises: (1) separating the plurality of particles from said solution, and (2) resuspending the separated plurality of particles in a further volume of said solution. In some embodiments, the method comprises repeating steps (1) and (2) at least once, at least twice, or more.

In some embodiments, the second organic solvent and the suspending agent in the solution are in about a 1:1 ratio.

In some embodiments, the suspending agent is a sugar alcohol, a glycol, a water-miscible polymer, an aprotic solvent, or any combination thereof. In some embodiments, the sugar alcohol is glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, or any combination thereof, optionally wherein the sugar alcohol is glycerol; the glycol is diethylene glycol, ethylene glycol, hexylene glycol, propylene glycol, dipropylene glycol, triethylene glycol, tripropylene glycol, a glycol ether, or any combination thereof; optionally wherein the glycol is propylene glycol; the water-miscible polymer is an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, or any combination thereof, optionally wherein the water-miscible polymer is a PEG 200 and/or PEG 300; and/or the aprotic solvent is a polar aprotic solvent, optionally wherein the aprotic solvent is sulfolane. In some embodiments, the suspending agent comprises glycerol.

In some embodiments, the second organic solvent comprises an organic solvent miscible with water and/or with high solubility in water. In some embodiments, the second organic solvent comprises: a polar aprotic solvent, optionally wherein the polar aprotic solvent is DMSO, tetrahydrofuran (THF), acetonitrile, propylene carbonate, sulfolane, and any combination thereof; a water miscible P-type or E-type glycol ether, optionally wherein the second organic solvent comprises dipropylene glycol methyl ether and/or diethylene glycol ethyl ether; an alcohol, optionally wherein the alcohol is ethanol and/or isopropanol; or tetrahydrothiophene 1-oxide. In some embodiments, the second organic solvent comprises a mixture of two or more solvents, optionally wherein the solvents are in about a 1:1 ratio, and further optionally wherein said mixture comprises: DMSO and ethanol; DMSO and isopropanol; or DMSO and THF. In some embodiments, the second organic solvent comprises DMSO.

In some embodiments, the second organic solvent is the same as the first organic solvent; or the second organic solvent is different from the first organic solvent. In some embodiments, the first and second organic solvents comprise DMSO.

In some embodiments, step (b) comprises: (i) separating the plurality of particles from a volume of said solution comprising the second organic solvent and the suspending agent, and (ii) suspending the separated plurality of particles in a volume of said formulation.

In some embodiments, the mobile phase is at a concentration of between about 60% and about 99%, between about 65% and about 95%, between about 70% and about 90%, between about 75% and about 90%, or between about 80% and about 90% weight by weight (w/w). In some embodiments, the mobile phase is at a concentration of about 70%, about 80%, or about 90% w/w. In some embodiments, the mobile phase is at a concentration of about 80% w/w. In some embodiments, the suspending agent is a sugar alcohol, a glycol, a water-miscible polymer, an aprotic solvent, or any combination thereof. In some embodiments, the sugar alcohol is glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, or any combination thereof. In some embodiments, the sugar alcohol is glycerol. In some embodiments, the glycerol is at a concentration of about 80% w/w in the composition. In some embodiments, the glycol is diethylene glycol, ethylene glycol, hexylene glycol, propylene glycol, dipropylene glycol, triethylene glycol, tripropylene glycol, a glycol ether, or any combination thereof. In some embodiments, the glycol is propylene glycol. In some embodiments, the water-miscible polymer is an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, or any combination thereof. In some embodiments, the water-miscible polymer is a polyethylene glycol (PEG) polymer. In some embodiments, the water-miscible polymer comprises an average molecular weight of about 1000 g/mol or less, about 900 g/mol or less, about 800 g/mol or less, about 700 g/mol or less, about 600 g/mol or less, about 500 g/mol or less, about 400 g/mol or less, about 300 g/mol or less, about 200 g/mol or less, or about 100 g/mol or less. In some embodiments, the water-miscible polymer comprises an average molecular weight of between about 100 g/mol and about 1000 g/mol, between about 200 g/mol and about 600 g/mol, between about 200 g/mol and about 400 g/mol, or between about 200 g/mol and about 300 g/mol. In some embodiments, the water-miscible polymer comprises an average molecular weight of about 200 g/mol or about 300 g/mol. In some embodiments, the water-miscible polymer is PEG 200 and/or PEG 300. In some embodiments, the aprotic solvent is a polar aprotic solvent, optionally a dipolar aprotic solvent. In some embodiments, the aprotic solvent is dimethylsulfoxide (DMSO), dimethylsulfone, diphenylsulfone (DPS), diethylsulfoxide, diethylsulfone, diisopropylsulfone, sulfolane, tetrahydrothiophene-1 -monoxide, N,N-dimethylacetamide (DMAc), N,N-dimethylformamide (DMF), N-methyl-2-pyrrolidinone (NMP), or any combination thereof. In some embodiments, the aprotic solvent is sulfolane.

In some embodiments, the viscosity enhancing agent is at least partially soluble in the mobile phase. In some embodiments, the viscosity enhancing agent is at a concentration of between about 1% and about 40%, between about 1% and about 35%, between about 1% and about 30%, between about 1% and about 25%, between about 1% and about 20%, between about 1% and about 15%, between about 1% and about 10%, or between about 1% and about 5% w/w. In some embodiments, the viscosity enhancing agent is at a concentration of about 5% w/w. In some embodiments, the viscosity enhancing agent comprises a water-miscible polymer. In some embodiments, the water-miscible polymer comprises an average molecular weight of at least about 2000 g/mol, at least about 4000 g/mol, at least about 5000 g/mol, at least about 6000 g/mol, at least about 7000 g/mol, at least about 8000 g/mol, at least about 9000 g/mol, at least about 10000 g/mol, at least about 12000 g/mol, at least about 14000 g/mol, at least about 16000 g/mol, at least about 18000 g/mol, at least about 20000 g/mol, at least about 25000 g/mol, or at least about 30000 g/mol. In some embodiments, the water-miscible polymer comprises an average molecular weight of between about 2000 g/mol and about 30000 g/mol, between about 4000 g/mol and about 20000 g/mol, between about 4000 g/mol and about 10000 g/mol, or between about 4000 g/mol and about 8000 g/mol. In some embodiments, the water-miscible polymer comprises an average molecular weight of about 4000 g/mol, about 8000 g/mol, about 10000 g/mol, or about 20000 g/mol. In some embodiments, said water-miscible polymer is an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, or any combination thereof. In some embodiments, the water-miscible polymer comprises a polyethylene glycol (PEG) polymer. In some embodiments, the PEG polymer is PEG 4000, PEG 8000, PEG 10000, PEG 20000, or any combination thereof. In some embodiments, the PEG polymer is PEG 8000. In some embodiments, the PEG 8000 is at a concentration of about 5% w/w in the composition. In some embodiments, the viscosity enhancing agent comprises polyvinyl alcohol (PVA). In some embodiments, the viscosity enhancing agent comprises sulfone or a sulfone polymer.

In some embodiments, the solubilizing agent is at least partially soluble in the mobile phase. In some embodiments, the solubilizing agent is water-soluble. In some embodiments, the solubilizing agent is at a concentration of between about 1% and about 40%, between about 5% and about 30%, between about 10% and about 25%, or between about 10% and about 20% w/w. In some embodiments, the solubilizing agent is at a concentration of about 15% w/w. In some embodiments, the solubilizing agent comprises a chaotropic agent. In some embodiments, the chaotropic agent is n-butanol, ethanol, a guanidinium salt, lithium perchlorate, lithium acetate, magnesium chloride, phenol, 2-propanol, sodium dodecyl sulfate, thiourea, urea, a water-soluble urea derivative, or any combination thereof. In some embodiments, the chaotropic agent is a guanidinium salt. In some embodiments, the guanidium salt is guanidinium chloride, guanidinium sulfate, guanidine carbonate, guanidinium nitrate, guanidinium isothiocyanate, guanidinium thiocyanate, or any combination thereof. In some embodiments, the chaotropic agent is urea and/or a water-soluble urea derivative. In some embodiments, the urea and/or the water-soluble urea derivative is at a concentration of about 15% w/w in the composition. In some embodiments, the water-soluble urea derivative is thiourea, hydroxyurea, dimethylurea, or any combination thereof. In some embodiments, the solubilizing agent comprises a buffering agent. In some embodiments, the buffering agent is a MES, Bis-Tris, ADA, ACES, PIPES, MOPSO, Bis-Tris Propane, BES, MOPS, TES, HEPES, DIPSO, MOBS, TAPSO, Tris, HEPPSO, POPSO, TEA, EPPS, HEPPS, Tricine, Gly-Gly, Bicine, HEPBS, TAPS, AMPD, TABS, AMPSO, CHES, CAPSO, AMP, CAPS, CABS, phosphate, maleate, glycine, citrate, formate, succinate, acetate, propionate, pyridine, piperazine, cacodylate, histidine, ethanolamine, carbonate, imidazole, pyrophosphate, hydrazine, taurine (AES), borate, ammonium hydroxide, methylamine, piperidine, malate buffer, or any combination thereof. In some embodiments, the buffering agent comprises a TAPS, HEPES, or MOPS buffer, or any combination thereof.

In some embodiments, the suspending agent comprises glycerol, the viscosity enhancing agent comprises PEG 8000, and the solubilizing agent comprises urea. In some embodiments, the glycerol is at a concentration of about 80% w/w, the PEG 8000 is at a concentration of about 5% w/w, and the urea is at a concentration of about 15% w/w. In some embodiments, the ratio of glycerol:PEG 8000:urea in the composition is 16:1:3 w/w. In some embodiments, the pH of the composition is between about 7 and about 8.

In some embodiments, the composition has a viscosity of: between about 7000 millipascal-second (mPa·s) and about 8000 mPa s, between about 7250 mPa s and about 8000 mPa s, between about 7500 mPa s and about 8000 mPa s, or between about 7750 mPa s and about 8000 mPa s, when measured at a temperature of about 15°C; between about 5000 mPa s and about 6000 mPa s, between about 5250 mPa s and about 6000 mPa·s, between about 5500 mPa·s and about 6000 mPa·s, or between about 5500 mPa·s and about 5750 mPa s, when measured at a temperature of about 20°C; between about 5500 mPa s and about 7000 mPa s, between about 5750 mPa s and about 6750 mPa s, or between about 6000 mPa s and about 6500 mPa s, when measured at a temperature of about 25°C; between about 4250 mPa s and about 5500 mPa·s, between about 4500 mPa·s and about 5500 mPa·s, between about 4750 mPa·s and about 5250 mPa·s, or between about 4750 mPa·s and about 5000 mPa·s, when measured at a temperature of about 30°C; between about 2000 mPa·s and about 3000 mPa·s, between about 2250 mPa·s and about 2750 mPa·s, or between about 2250 mPa·s and about 2500 mPa·s, when measured at a temperature of about 35°C; between about 750 mPa·s and about 1750 mPa·s, between about 1000 mPa·s and about 1750 mPa s, or between about 1000 mPa s and about 1500 mPa s, when measured at a temperature of about 40°C; between about 500 mPa·s and about 1500 mPa·s, between about 750 mPa·s and about 1250 mPa·s, or between about 1000 mPa·s and about 1250 mPa·s, when measured at a temperature of about 45°C; between about 250 mPa·s and about 1500 mPa·s, between about 500 mPa·s and about 1250 mPa·s, or between about 500 mPa·s and about 1000 mPa·s, when measured at a temperature of about 50°C; between about 500 mPa·s and about 1500 mPa·s, between about 750 mPa·s and about 1250 mPa·s, or between about 1000 mPa·s and about 1250 mPa·s, when measured at a temperature of about 55°C; and/or between about 100 mPa·s and about 600 mPa·s, between about 200 mPa·s and about 500 mPa·s, or between about 200 mPa·s and about 400 mPa·s, when measured at a temperature of about 60°C. In some embodiments, viscosity of the composition is assessed using a rotational viscometer. In some embodiments, viscosity of the composition is assessed at a spindle rotational speed of about 10 revolutions per minute (RPM).

In some embodiments, the composition has a density of between about 0.5 g/mL and about 2 g/mL, between about 1 g/mL and about 2 g/mL, between about 1 g/mL and about 1.5 g/mL, or between about 1.1 g/mL and about 1.5 g/mL. In some embodiments, the composition has a density of about 1.3 g/mL. In some embodiments, density of the composition is assessed on an analytical balance.

In some embodiments, the mobile phase is non-aqueous or substantially non-aqueous. In some embodiments, the composition is non-aqueous or substantially non-aqueous. In some embodiments, the composition has a water content of about 1% or less. In some embodiments, water content is assessed by Karl Fischer titration.

In some embodiments, the composition is a liquid composition.

In some embodiments, the plurality of particles comprises one or more particles configured to bind a nucleic acid, a polypeptide, a lipid, a carbohydrate, or a small molecule. In some embodiments, the plurality of particles comprises one or more particles configured to bind DNA and/or RNA. In some embodiments, the plurality of particles comprises one or more microparticles, nanoparticles, microspheres, paramagnetic beads, magnetic beads, microbeads, nanobeads, or any combination thereof. In some embodiments, the plurality of particles comprises one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. In some embodiments, one or more particles of the plurality of particles is functionalized. In some embodiments, one or more particles are surface functionalized with a carboxyl, amino, hydroxyl, silica, streptavidin, endopeptidase enzyme moiety, amine, thiol, diol, polymer, an antibody or antibody fragment, a nucleic acid, an oligonucleotide, a peptide, a polypeptide, or any combination thereof. In some embodiments, the plurality of particles comprises magnetic silica beads. In some embodiments, the plurality of particles is present in the composition at a concentration of at least about 0.5%, at least about 1%, at least about 2.5%, at least about 5%, at least about 10%, at least about 15%, at least about 20%, at least about 25%, or at least about 30% weight by volume (w/v). In some embodiments, the plurality of particles is present in the composition at a concentration of between about 0.5% and about 30% w/v, between about 1% and about 25% w/v, between about 2.5% and about 20% w/v, or between about 5% and about 15% w/v. In some embodiments, the plurality of particles is present in the composition at a concentration of about 10% w/v.

In some embodiments, the composition enhances stability of particles in the plurality of particles. In some embodiments, the composition enhances stability of particles in the plurality of particles as compared to corresponding particles in an aqueous solution. In some embodiments, the plurality of particles in the composition is stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months. In some embodiments, particle stability is assessed based on particle integrity, particle aggregation, and/or retention of ability to bind to a ligand. In some embodiments, the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline. In some embodiments, the composition enhances ligand binding of particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution. In some embodiments, the plurality of particles comprises particles capable of binding to nucleic acid ligands. In some embodiments, stability of the particles is assessed based on nucleic acid ligand binding ability compared to baseline and/or compared to corresponding particles in an aqueous solution. In some embodiments, the nucleic acid ligands comprise DNA and/or RNA. In some embodiments, the plurality of particles comprises silica particles. In some embodiments, the plurality of particles comprises magnetic silica particles. In some embodiments, the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of nucleic acid ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline. In some embodiments, the composition enhances nucleic acid ligand binding by particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution. In some embodiments, particle stability is assessed at room temperature. In some embodiments, particle stability is assessed at a temperature of between about 20°C and about 30°C. In some embodiments, particle stability is assessed at a temperature of about 25°C. In some embodiments, particle stability is assessed under normal atmospheric conditions, in reduced oxygen conditions, or in nitrogen gas. In some embodiments, the composition is capable of adhering to a polypropylene surface. In some embodiments, the composition is microbiologically stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months.

In some embodiments, the composition further comprises an agent, such as an antioxidant, a reducing agent, a chelating agent, or any combination thereof. In some embodiments, the composition does not comprise an anti-microbial agent. In some embodiments, the composition comprises less than about 10% of surfactant or detergent. In some embodiments, the composition does not comprise a surfactant or detergent.

In another aspect, provided herein is a composition comprising a plurality of particles produced by any of the methods for preparing a composition provided herein.

In another aspect, provided herein are methods for isolating an analyte from a sample, comprising: (a) providing a sample comprising an analyte (or suspected of comprising an analyte); (b) mixing the sample with a composition comprising a plurality of particles provided herein, or a composition comprising a plurality of particles produced by a method provided herein, to produce a mixture of the sample and the composition, thereby binding said analyte (if present) to one or more particles in the plurality of particles; and (c) separating the plurality of particles from the mixture. In some embodiments, the method further comprises eluting the analyte bound to the one or more particles in the plurality of particles, and optionally separating the plurality of particles from the eluted analyte. In some embodiments, the method further comprises isolating or recovering the eluted analyte. In some embodiments, the analyte is a nucleic acid, a polypeptide, a lipid, a carbohydrate, or a small molecule. In some embodiments, the analyte is DNA and/or RNA. In some embodiments, the plurality of particles comprises one or more particles configured to bind DNA and/or RNA. In some embodiments, the plurality of particles comprises one or more microparticles, nanoparticles, microspheres, paramagnetic beads, magnetic beads, microbeads, nanobeads, or any combination thereof. In some embodiments, the plurality of particles comprises one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. In some embodiments, the plurality of particles is functionalized, optionally wherein the one or more particles are surface functionalized with a carboxyl, amino, hydroxyl, silica, streptavidin, endopeptidase enzyme moiety, amine, thiol, diol, polymer, an antibody or antibody fragment, a nucleic acid, an oligonucleotide, a peptide, a polypeptide, or any combination thereof. In some embodiments, the plurality of particles comprises silica beads, optionally magnetic silica beads, and further optionally wherein the analyte is DNA and/or RNA. In some embodiments, the sample is or is derived from a tissue, primary or cultured cells or cell lines, a cell supernatant, a cell lysate, platelets, serum, plasma, vitreous fluid, lymph fluid, synovial fluid, follicular fluid, seminal fluid, amniotic fluid, milk, whole blood, blood-derived cells, urine, cerebrospinal fluid, saliva, sputum, tears, perspiration, mucus, tumor lysates, tissue culture medium, a tissue extract, homogenized tissue, tumor tissue, cellular extracts, or a combination thereof. In some embodiments, the sample is a liquid biopsy sample, optionally wherein the liquid biopsy sample is a sample of blood or plasma. In some embodiments, the sample is a liquid biopsy sample, and the analyte is cell-free DNA or cell-free RNA. In some embodiments, the sample is or is derived from a nasal swab, a buccal swab, a cervical swab, an ear swab, an eye swab, a vaginal swab, a penile swab, a rectal swab, a throat swab, a urethral swab, a vulval swab, a wound swab, or a skin swab. In some embodiments, the sample is or is derived from an environmental sample such as a water sample, soil sample, surface sample, air sample, rock or mineral sample, plant sample, sediment sample, and the like; a food sample, such as plant samples, meat samples, dairy samples, plant- or animalderived product samples, food additive or component samples, and the like; or any other type of sample that may comprise an analyte of interest.

In another aspect, provided herein is a method for cell lysis, comprising: (a) providing a sample comprising a plurality of cells; (b) mixing the sample with a composition comprising a plurality of particles provided herein, or a composition comprising a plurality of particles produced by a method provided herein, thereby producing a mixture of the sample and the composition; and (c) agitating the mixture, thereby lysing one or more cells in the plurality of cells. In some embodiments, the method further comprises separating the plurality of particles from the mixture. In some embodiments, the one or more cells comprise one or more prokaryotic cells and/or one or more eukaryotic cells. In some embodiments, the one or more cells comprise one or more animal cells, plant cells, mammalian cells, bacterial cells, archaeal cells, fungal cells, protozoan cells, algal cells, or any combination thereof. In some embodiments, the plurality of particles comprises one or more mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. In some embodiments, the method is implemented on a microfluidic device and/or an automated or robotic liquid handling system.

In another aspect, provided herein is a kit comprising a composition provided herein, or a composition produced by a method provided herein. In some embodiments, the kit further comprises instructions for use of the kit or the composition in the kit for isolating one or more analytes from a sample, optionally wherein the instructions are for isolating the one or more analytes from the sample according to a method provided herein. In some embodiments, the kit further comprises instructions for use of the kit or the composition in the kit for lysing one or more cells from a sample, optionally wherein the instructions are for lysing the one or more cells according to a method provided herein.

In another aspect, provided herein is a microfluidic device comprising a composition provided herein, or a composition produced by a method provided herein.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows.

BRIEF DESCRIPTION OF THE DRAWINGS

**FIG.** 1 is a box plot showing the RNA isolation efficiency of silica magnetic beads stored in formulation K (*see* Examples 2-3, herein) at room temperature under normal atmospheric conditions for the indicated amounts of time (from 3 to 15 months). Isolation efficiencies are provided as a percentage relative to the RNA isolation efficiency of the beads when stored in an aqueous solution ("wet beads," set at 100%).

DETAILED DESCRIPTION

The present disclosure relates generally to compositions comprising a plurality of particles, methods of preparing such compositions, as well as uses, kits and articles of manufacture related thereto.

Particles, such as beads, are important tools in various fields, including research (e.g., molecular biology research), diagnostics, medical, and industrial applications. The present disclosure provides compositions that support and/or enhance particle stability and functionality, and facilitate handling and storage of particles, including for extended periods of time in normal ambient conditions. The present disclosure also provides methods for preparing compositions of particles, as well as certain uses for such compositions, and kits and articles of manufacture related thereto.

I. Definitions

As used herein and in the appended claims, the singular forms "a," "an," and "the" include plural referents unless the context clearly dictates otherwise.

The terms "comprising," "having," "including," and "containing" are to be construed as openended terms (i.e., meaning "including, but not limited to,") unless otherwise noted.

Recitations of ranges of values herein are merely intended to serve as a shorthand method of referring individually to each separate value falling within the range, unless otherwise indicated herein, and each separate value is incorporated into the specification as if it were individually recited herein. For example, if the range 10-15 is disclosed, then 11, 12, 13, and 14 are also disclosed.

Reference to "about" a value or parameter herein refers to the usual error range for the respective value readily known to the skilled person in this technical field. For example, reference to "about" a value or parameter herein includes (and describes) aspects that are directed to the stated value or parameter, as well as values or parameters within a variation (error range) of, for example 0-10%, around the stated value or parameter (e.g., X±10%). In one specific example, in the context of compositions containing amounts of ingredients where the term "about" is used, these compositions may contain the stated amount of the ingredient with a variation (error range) of, e.g., 0-10%, around the stated value (e.g., X±10%). Reference to "about" a value or parameter herein includes (and describes) aspects that are directed to that value or parameter per se. For example, description referring to "about X" includes description of "X."

The use of any and all examples, or exemplary language (e.g., "such as," "exemplary" or "for example") provided herein, is intended merely to better illuminate the embodiments of the disclosure and does not pose a limitation on the scope of the disclosure unless otherwise claimed. No language in the specification should be construed as indicating any non-claimed element as essential to the practice of the embodiments of the disclosure. All methods described herein can be performed in any suitable order unless otherwise indicated herein or otherwise clearly contradicted by context.

**II. Compositions of the disclosure**

Certain aspects of the present disclosure relate to compositions comprising one or more particles. As described in greater detail below, compositions of the present disclosure may possess one or more, or all, of the properties of: low or no water content (e.g., may be non-aqueous or substantially non-aqueous), adherence to surfaces (e.g., plastic or glass surfaces), high viscosity, high density, sustained and/or enhanced particle stability and/or particle functionality, sustained and/or enhanced particle ligand binding, and/or microbiological stability.

In some embodiments, a composition of the present disclosure may comprise one or more particles, and any two or all three of: (a) a mobile phase, (b) a viscosity enhancing agent, and (c) a solubilizing agent. In some embodiments, a composition of the disclosure comprises the one or more particles, a mobile phase, and a viscosity enhancing agent. In some embodiments, a composition of the disclosure comprises the one or more particles, a mobile phase, and a solubilizing agent. In some embodiments, a composition of the disclosure comprises the one or more particles, a viscosity enhancing agent, and a solubilizing agent. In some embodiments, a composition of the disclosure comprises the one or more particles, a mobile phase, a viscosity enhancing agent, and a solubilizing agent.

A composition of the disclosure may be a liquid composition. In some cases, compositions of the disclosure have a relatively high viscosity and/or density. Thus, in some embodiments, a composition of the disclosure may be a paste, cream or gel. In some embodiments, compositions of the disclosure are non-aqueous or substantially non-aqueous.

*a. Mobile phases*

In some embodiments, a composition of the present disclosure comprises a mobile phase. A mobile phase is typically a fluid that can serve to suspend the components of a composition in solution.

In some embodiments, a composition of the disclosure comprises a mobile phase in a concentration of at least about 50%, at least about 55%, at least about 60%, at least about 65%, at least about 70%, at least about 75%, at least about 80%, at least about 85%, at least about 90%, at least about 95%, or at least about 99% weight by weight (w/w). In some embodiments, the composition comprises a mobile phase in a concentration of between about 50% and about 99%, between about 60% and about 99%, between about 60% and about 95%, between about 65% and about 90%, between about 70% and about 90%, between about 75% and about 90%, or between about 80% and about 90%, w/w, including any value within each of the recited ranges. In some embodiments, a composition of the disclosure comprises a mobile phase in a concentration of any of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%, w/w. In some embodiments, a composition of the disclosure comprises a mobile phase in a concentration of about 80% w/w.

Any suitable mobile phase may be used in a composition of the disclosure. In some embodiments, the mobile phase may be, for example, a solvent, a detergent, water, and/or suspending agents. In some embodiments, the mobile phase is non-aqueous, or substantially non-aqueous. In some embodiments, the mobile phase is water miscible or water soluble.

In some embodiments, the mobile phase of a composition of the present disclosure comprises one or more suspending agents. Suspending agents are typically excipients that can aid components in a composition, such as particles and other components, in remaining suspended and/or dispersed in the composition, and may inhibit or reduce aggregation, caking, and/or settling. Any suitable suspending agent known in the art may be used in compositions of the disclosure. Exemplary suspending agents that may be used include, without limitation, sugar alcohols, glycols, water-miscible polymers, aprotic solvents, as well as any combination thereof.

In some embodiments, the suspending agent in a composition of the disclosure comprises a sugar alcohol. Any suitable sugar alcohol known in the art may be used in compositions of the disclosure. Exemplary sugar alcohols that may be used include, without limitation, glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, as well as any combination thereof. In some embodiments, the sugar alcohol comprises glycerol. In some embodiments, the sugar alcohol (such as glycerol) is present in the composition in a concentration of at least about 50%, at least about 55%, at least about 60%, at least about 65%, at least about 70%, at least about 75%, at least about 80%, at least about 85%, at least about 90%, at least about 95%, or at least about 99% w/w. In some embodiments, the sugar alcohol (such as glycerol) is present in the composition in a concentration of between about 50% and about 99%, between about 60% and about 99%, between about 60% and about 95%, between about 65% and about 90%, between about 70% and about 90%, between about 75% and about 90%, or between about 80% and about 90%, w/w, including any value within each of the recited ranges. In some embodiments, the sugar alcohol (such as glycerol) is present in the composition in a concentration of any of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%, w/w. In some embodiments, the sugar alcohol (such as glycerol) is present in the composition in a concentration of about 80% w/w. In some embodiments, the sugar alcohol comprises glycerol in a concentration of about 80% w/w in the composition.

In some embodiments, the suspending agent in a composition of the disclosure comprises a glycol. Any suitable glycol known in the art may be used in compositions of the disclosure. Exemplary glycols that may be used include, without limitation, diethylene glycol, ethylene glycol, hexylene glycol, propylene glycol, dipropylene glycol, triethylene glycol, tripropylene glycol, a glycol ether, as well as any combination thereof. In some embodiments, the suspending agent in a composition of the disclosure includes propylene glycol. In some embodiments, the glycol (such as propylene glycol) is present in the composition in a concentration of at least about 50%, at least about 55%, at least about 60%, at least about 65%, at least about 70%, at least about 75%, at least about 80%, at least about 85%, at least about 90%, at least about 95%, or at least about 99% w/w. In some embodiments, the glycol (such as propylene glycol) is present in the composition in a concentration of between about 50% and about 99%, between about 60% and about 99%, between about 60% and about 95%, between about 65% and about 90%, between about 70% and about 90%, between about 75% and about 90%, or between about 80% and about 90%, w/w, including any value within each of the recited ranges. In some embodiments, the glycol (such as propylene glycol) is present in the composition in a concentration of any of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%, w/w. In some embodiments, the glycol (such as propylene glycol) is present in the composition in a concentration of about 80% w/w.

In some embodiments, the suspending agent in a composition of the disclosure comprises a water-miscible polymer. Any suitable water-miscible polymer known in the art may be used in compositions of the disclosure. Exemplary water-miscible polymers that may be used include, without limitation, an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, polyvinyl alcohol (PVA), a polyurethane polymer, as well as any combination thereof. In some embodiments, the water-miscible polymer comprises a polyethylene glycol (PEG) polymer. In some embodiments, the water-miscible polymer has an average molecular weight of about 1000 g/mol or less, about 900 g/mol or less, about 800 g/mol or less, about 700 g/mol or less, about 600 g/mol or less, about 500 g/mol or less, about 400 g/mol or less, about 300 g/mol or less, about 200 g/mol or less, or about 100 g/mol or less. In some embodiments, the water-miscible polymer has an average molecular weight of about 300 g/mol or less, about 200 g/mol or less, or about 100 g/mol or less. In some embodiments, the water-miscible polymer has an average molecular weight of between about 100 g/mol and about 2000 g/mol, between about 100 g/mol and about 1000 g/mol, between about 200 g/mol and about 600 g/mol, between about 200 g/mol and about 400 g/mol, or between about 200 g/mol and about 300 g/mol, including any value within each of the recited ranges. In some embodiments, the water-miscible polymer has an average molecular weight of about 100 g/mol, about 200 g/mol, about 300 g/mol, about 400 g/mol, about 500 g/mol, about 600 g/mol, about 700 g/mol, about 800 g/mol, about 900 g/mol, or about 1000 g/mol. In some embodiments, the water-miscible polymer has an average molecular weight of about 200 g/mol, or about 300 g/mol. In some embodiments, the water-miscible polymer is a PEG polymer, such as PEG 200, PEG 300, PEG 400, PEG 600, or PEG 1000. In some embodiments, the water-miscible polymer is PEG 200 and/or PEG 300. In some embodiments, the water-miscible polymer (such as a PEG polymer, e.g., PEG 200 and/or PEG 300) is present in the composition in a concentration of at least about 50%, at least about 55%, at least about 60%, at least about 65%, at least about 70%, at least about 75%, at least about 80%, at least about 85%, at least about 90%, at least about 95%, or at least about 99% w/w. In some embodiments, the water-miscible polymer (such as a PEG polymer, e.g., PEG 200 and/or PEG 300) is present in the composition in a concentration of between about 50% and about 99%, between about 60% and about 99%, between about 60% and about 95%, between about 65% and about 90%, between about 70% and about 90%, between about 75% and about 90%, or between about 80% and about 90%, w/w, including any value within each of the recited ranges. In some embodiments, the water-miscible polymer (such as a PEG polymer, e.g., PEG 200 and/or PEG 300) is present in the composition in a concentration of any of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%, w/w. In some embodiments, the water-miscible polymer (such as a PEG polymer, e.g., PEG 200 and/or PEG 300) is present in the composition in a concentration of about 80% w/w.

In some embodiments, the suspending agent in a composition of the disclosure comprises an aprotic solvent. In some embodiments, the aprotic solvent is a polar aprotic solvent. In some embodiments, the aprotic solvent is a dipolar aprotic solvent. Any suitable aprotic solvent known in the art may be used in compositions of the disclosure. Exemplary aprotic solvents that may be used include, without limitation, dimethylsulfoxide (DMSO), dimethylsulfone, diphenylsulfone (DPS), diethylsulfoxide, diethylsulfone, diisopropylsulfone, sulfolane, tetrahydrothiophene-1 -monoxide, N,N-dimethylacetamide (DMAc), N,N-dimethylformamide (DMF), N-methyl-2-pyrrolidinone (NMP), as well as any combination thereof. In some embodiments, the aprotic solvent comprises sulfolane. In some embodiments, the aprotic solvent (such as sulfolane) is present in the composition in a concentration of at least about 50%, at least about 55%, at least about 60%, at least about 65%, at least about 70%, at least about 75%, at least about 80%, at least about 85%, at least about 90%, at least about 95%, or at least about 99% w/w. In some embodiments, the aprotic solvent (such as sulfolane) is present in the composition in a concentration of between about 50% and about 99%, between about 60% and about 99%, between about 60% and about 95%, between about 65% and about 90%, between about 70% and about 90%, between about 75% and about 90%, or between about 80% and about 90%, w/w, including any value within each of the recited ranges. In some embodiments, the aprotic solvent (such as sulfolane) is present in the composition in a concentration of any of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%, w/w. In some embodiments, the aprotic solvent (such as sulfolane) is present in the composition in a concentration of about 80% w/w.

### b. Viscosity enhancers

In some embodiments, a composition of the disclosure comprises a viscosity enhancing agent. Such agents can be used to increase the viscosity of a composition, and may include, for example, polymers, thickeners, texturizers, crosslinkers, gelling agents and stiffening agents.

In some embodiments, a composition of the disclosure includes one or more viscosity enhancing agents in an amount sufficient to achieve a viscosity in the final composition of any of at least about 7000 mPa·s, at least about 7100 mPa·s, at least about 7200 mPa·s, at least about 7300 mPa·s, at least about 7400 mPa·s, at least about 7500 mPa·s, at least about 7600 mPa·s, at least about 7700 mPa·s, at least about 7800 mPa s, at least about 7900 mPa s, or at least about 8000 mPa s, when measured at a temperature of about 15°C; or between about 7000 mPa·s and about 8000 mPa·s, between about 7250 mPa·s and about 8000 mPa·s, between about 7500 mPa·s and about 8000 mPa·s, or between about 7750 mPa·s and about 8000 mPa·s, including any value within each of the recited ranges, when measured at a temperature of about 15°C.

In some embodiments, a composition of the disclosure includes one or more viscosity enhancing agents in an amount sufficient to achieve a viscosity in the final composition of any of at least about 5000 mPa s, at least about 5100 mPa·s, at least about 5200 mPa·s, at least about 5300 mPa·s, at least about 5400 mPa·s, at least about 5500 mPa·s, at least about 5600 mPa·s, at least about 5700 mPa·s, at least about 5800 mPa·s, at least about 5900 mPa·s, or at least about 6000 mPa·s, when measured at a temperature of about 20°C; or between about 5000 mPa·s and about 6000 mPa·s, between about 5250 mPa·s and about 6000 mPa·s, between about 5500 mPa·s and about 6000 mPa·s, or between about 5500 mPa·s and about 5750 mPa·s, including any value within each of the recited ranges, when measured at a temperature of about 20°C.

In some embodiments, a composition of the disclosure includes one or more viscosity enhancing agents in an amount sufficient to achieve a viscosity in the final composition of any of at least about 5500 mPa·s, at least about 5600 mPa·s, at least about 5700 mPa·s, at least about 5800 mPa·s, at least about 5900 mPa·s, at least about 6000 mPa·s, at least about 6100 mPa·s, at least about 6200 mPa·s, at least about 6300 mPa·s, at least about 6400 mPa·s, at least about 6500 mPa·s, at least about 6600 mPa·s, at least about 6700 mPa·s, at least about 6800 mPa·s, at least about 6900 mPa·s, or at least about 7000 mPa·s, when measured at a temperature of about 25°C; or between about 5500 mPa·s and about 7000 mPa·s, between about 5750 mPa·s and about 6750 mPa·s, or between about 6000 mPa·s and about 6500 mPa·s, including any value within each of the recited ranges, when measured at a temperature of about 25°C.

In some embodiments, a composition of the disclosure includes one or more viscosity enhancing agents in an amount sufficient to achieve a viscosity in the final composition of any of at least about 4200 mPa·s, at least about 4300 mPa·s, at least about 4400 mPa·s, at least about 4500 mPa·s, at least about 4600 mPa·s, at least about 4700 mPa·s, at least about 4800 mPa·s, at least about 4900 mPa·s, at least about 5000 mPa·s, at least about 5100 mPa·s, at least about 5200 mPa·s, at least about 5300 mPa·s, at least about 5400 mPa·s, or at least about 5500 mPa·s, when measured at a temperature of about 30°C; or between about 4250 mPa·s and about 5500 mPa·s, between about 4500 mPa·s and about 5500 mPa·s, between about 4750 mPa·s and about 5250 mPa·s, or between about 4750 mPa·s and about 5000 mPa·s, including any value within each of the recited ranges, when measured at a temperature of about 30°C.

In some embodiments, a composition of the disclosure includes one or more viscosity enhancing agents in an amount sufficient to achieve a viscosity in the final composition of any of at least about 2000 mPa·s, at least about 2100 mPa·s, at least about 2200 mPa·s, at least about 2300 mPa·s, at least about 2400 mPa·s, at least about 2500 mPa·s, at least about 2600 mPa·s, at least about 2700 mPa·s, at least about 2800 mPa s, at least about 2900 mPa s, or at least about 3000 mPa s, when measured at a temperature of about 35°C; or between about 2000 mPa·s and about 3000 mPa·s, between about 2250 mPa·s and about 2750 mPa·s, or between about 2250 mPa s and about 2500 mPa s, including any value within each of the recited ranges, when measured at a temperature of about 35°C.

In some embodiments, a composition of the disclosure includes one or more viscosity enhancing agents in an amount sufficient to achieve a viscosity in the final composition of any of at least about 750 mPa·s, at least about 850 mPa·s, at least about 950 mPa·s, at least about 1050 mPa·s, at least about 1150 mPa·s, at least about 1250 mPa·s, at least about 1350 mPa·s, at least about 1450 mPa·s, at least about 1550 mPa·s, at least about 1650 mPa·s, or at least about 1750 mPa·s, when measured at a temperature of about 40°C; or between about 750 mPa·s and about 1750 mPa·s, between about 1000 mPa·s and about 1750 mPa·s, or between about 1000 mPa·s and about 1500 mPa·s, including any value within each of the recited ranges, when measured at a temperature of about 40°C.

In some embodiments, a composition of the disclosure includes one or more viscosity enhancing agents in an amount sufficient to achieve a viscosity in the final composition of any of at least about 500 mPa·s, at least about 600 mPa·s, at least about 700 mPa·s, at least about 800 mPa·s, at least about 900 mPa·s, at least about 1000 mPa·s, at least about 1100 mPa·s, at least about 1200 mPa·s, at least about 1300 mPa·s, at least about 1400 mPa·s, or at least about 1500 mPa·s, when measured at a temperature of about 45°C; or between about 500 mPa·s and about 1500 mPa·s, between about 750 mPa·s and about 1250 mPa·s, or between about 1000 mPa·s and about 1250 mPa·s, including any value within each of the recited ranges, when measured at a temperature of about 45°C.

In some embodiments, a composition of the disclosure includes one or more viscosity enhancing agents in an amount sufficient to achieve a viscosity in the final composition of any of at least about 250 mPa·s, at least about 350 mPa·s, at least about 450 mPa·s, at least about 550 mPa·s, at least about 650 mPa·s, at least about 750 mPa·s, at least about 850 mPa·s, at least about 950 mPa·s, at least about 1050 mPa·s, at least about 1150 mPa·s, at least about 1250 mPa·s, at least about 1350 mPa·s, at least about 1450 mPa·s, or at least about 1550 mPa·s, when measured at a temperature of about 50°C; or between about 250 mPa·s and about 1500 mPa·s, between about 500 mPa·s and about 1250 mPa·s, or between about 500 mPa s and about 1000 mPa·s, including any value within each of the recited ranges, when measured at a temperature of about 50°C.

In some embodiments, a composition of the disclosure includes one or more viscosity enhancing agents in an amount sufficient to achieve a viscosity in the final composition of any of at least about 500 mPa s, at least about 600 mPa·s, at least about 700 mPa·s, at least about 800 mPa·s, at least about 900 mPa s, at least about 1000 mPa·s, at least about 1100 mPa·s, at least about 1200 mPa·s, at least about 1300 mPa s, at least about 1400 mPa·s, or at least about 1500 mPa·s, when measured at a temperature of about 55°C; or between about 500 mPa s and about 1500 mPa s, between about 750 mPa s and about 1250 mPa·s, or between about 1000 mPa·s and about 1250 mPa·s, including any value within each of the recited ranges, when measured at a temperature of about 55°C.

In some embodiments, a composition of the disclosure includes one or more viscosity enhancing agents in an amount sufficient to achieve a viscosity in the final composition of any of at least about 100 mPa·s, at least about 150 mPa·s, at least about 200 mPa·s, at least about 250 mPa·s, at least about 300 mPa·s, at least about 350 mPa·s, at least about 400 mPa·s, at least about 450 mPa·s, at least about 500 mPa·s, at least about 550 mPa·s, or at least about 600 mPa·s, when measured at a temperature of about 60°C; or between about 100 mPa·s and about 600 mPa·s, between about 200 mPa·s and about 500 mPa·s, or between about 200 mPa s and about 400 mPa s, including any value within each of the recited ranges, when measured at a temperature of about 60°C.

Viscosity of a composition of the disclosure may be assessed using any suitable technique known in the art, such as using a capillary viscometer, a viscosity cup, a Zahn cup, a falling sphere viscometer, a vibrational viscometer, a rotational viscometer, or a consistometer. In some embodiments, viscosity of a composition of the disclosure is assessed using a rotational viscometer. In some cases, viscosity may be assessed at a spindle rotational speed of about 10 revolutions per minute (RPM).

In some embodiments, a composition of the disclosure comprises a viscosity enhancing agent at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, a composition of the disclosure comprises a viscosity enhancing agent at a concentration of between about 1% and about 50%, between about 1% and about 40%, between about 1% and about 35%, between about 1% and about 30%, between about 1% and about 25%, between about 1% and about 20%, between about 1% and about 15%, between about 1% and about 10%, or between about 1% and about 5%, w/w, including any value within each of the recited ranges. In some embodiments, a composition of the disclosure comprises a viscosity enhancing agent at a concentration of about 1%, about 2%, about 3%, about 4%, about 5%, about 6%, about 7%, about 8%, about 9%, or about 10%, w/w. In some embodiments, the viscosity enhancing agent is at a concentration of about 5% w/w.

Any suitable viscosity enhancing agent may be used in a composition of the disclosure. In some embodiments, a viscosity enhancing agent of the disclosure may be, for example, a polymer, a thickener, a texturizer, a gelling agent, a crosslinker, a stiffening agent, as well as combinations thereof. In some embodiments, the viscosity enhancing agent is at least partially soluble in the mobile phase of the composition.

In some embodiments, the viscosity enhancing agent comprises a water-miscible polymer. Any suitable water-miscible polymer known in the art may be used in compositions of the disclosure. Exemplary water-miscible polymers that may be used include, without limitation, an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, polyvinyl alcohol (PVA), a polyurethane polymer, as well as any combination thereof. In some embodiments, the water-miscible polymer in a composition of the disclosure includes a polyethylene glycol (PEG) polymer.

In some embodiments, the water miscible polymer comprises an average molecular weight of at least about 1000 g/mol, at least about 2000 g/mol, at least about 3000 g/mol, at least about 4000 g/mol, at least about 5000 g/mol, at least about 6000 g/mol, at least about 7000 g/mol, at least about 8000 g/mol, at least about 9000 g/mol, at least about 10000 g/mol, at least about 11000 g/mol, at least about 12000 g/mol, at least about 13000 g/mol, at least about 14000 g/mol, at least about 15000 g/mol, at least about 16000 g/mol, at least about 17000 g/mol, at least about 18000 g/mol, at least about 19000 g/mol, at least about 20000 g/mol, at least about 21000 g/mol, at least about 22000 g/mol, at least about 23000 g/mol, at least about 24000 g/mol, at least about 25000 g/mol, at least about 26000 g/mol, at least about 27000 g/mol, at least about 28000 g/mol, at least about 29000 g/mol, or at least about 30000 g/mol. In some embodiments, the water-miscible polymer comprises an average molecular weight of between about 1000 g/mol and about 30000 g/mol, between about 2000 g/mol and about 30000 g/mol, between about 4000 g/mol and about 20000 g/mol, between about 4000 g/mol and about 10000 g/mol, or between about 4000 g/mol and about 8000 g/mol, including any value within the recited ranges. In some cases, the water-miscible polymer comprises an average molecular weight of about 4000 g/mol, about 8000 g/mol, about 10000 g/mol, or about 20000 g/mol. In some embodiments, the water-miscible polymer is a PEG polymer, such as PEG 4000, PEG 8000, PEG 10000, or PEG 20000, as well as any combination thereof. In some embodiments, the water-miscible polymer is PEG 8000. In some embodiments, the water-miscible polymer is PVA.

In some embodiments, the water-miscible polymer (e.g., a PEG polymer, such as PEG 4000, PEG 8000, PEG 10000, PEG 20000, PVA, or combinations thereof) is at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, the water-miscible polymer (e.g., a PEG polymer, such as PEG 4000, PEG 8000, PEG 10000, PEG 20000, PVA, or combinations thereof) is at a concentration of between about 1% and about 50%, between about 1% and about 40%, between about 1% and about 35%, between about 1% and about 30%, between about 1% and about 25%, between about 1% and about 20%, between about 1% and about 15%, between about 1% and about 10%, or between about 1% and about 5%, w/w, including any value within each of the recited ranges. In some embodiments, the water-miscible polymer (e.g., a PEG polymer, such as PEG 4000, PEG 8000, PEG 10000, PEG 20000, PVA, or combinations thereof) is at a concentration of about 1%, about 2%, about 3%, about 4%, about 5%, about 6%, about 7%, about 8%, about 9%, or about 10%, w/w. In some embodiments, the water-miscible polymer comprises PEG 8000 at a concentration of about 5% w/w in the composition.

In some embodiments, the viscosity enhancing agent comprises sulfone or a sulfone polymer. In some embodiments, the sulfone or sulfone polymer is at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, the sulfone or sulfone polymer is at a concentration of between about 1% and about 50%, between about 1% and about 40%, between about 1% and about 35%, between about 1% and about 30%, between about 1% and about 25%, between about 1% and about 20%, between about 1% and about 15%, between about 1% and about 10%, or between about 1% and about 5%, w/w, including any value within each of the recited ranges. In some embodiments, the sulfone or sulfone polymer is at a concentration of about 1%, about 2%, about 3%, about 4%, about 5%, about 6%, about 7%, about 8%, about 9%, or about 10%, w/w.

### c. Solubilizing agents

In some embodiments, a composition of the disclosure comprises one or more solubilizing agents. Solubilizing agents can increase solubility of components of a composition and/or prevent aggregation or caking, for example, by disrupting interactions between molecules (e.g., disulfide bonds, hydrogen bonds, van der Waals forces, ionic interactions, hydrophobic interactions, hydrophilic interactions, and the like).

In some embodiments, a composition of the disclosure includes one or more solubilizing agents at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, a composition of the disclosure includes one or more solubilizing agents at a concentration of between about 1% and about 50%, between about 5% and about 30%, between about 10% and about 25%, or between about 10% and about 20%, w/w, including any value within each of the recited ranges. In some embodiments, a composition of the disclosure includes one or more solubilizing agents at a concentration of about 5%, about 6%, about 7%, about 8%, about 9%, about 10%, about 11%, about 12%, about 13%, about 14%, about 15%, about 16%, about 17%, about 18%, about 19%, or about 20%, w/w. In some embodiments, a composition of the disclosure includes one or more solubilizing agents at a concentration of about 15% w/w.

Any suitable solubilizing agent may be used in a composition of the disclosure. Exemplary solubilizing agents that may be used include, without limitation, reducing agents, chaotropic agents, surfactants, buffering agents, and salts. In some embodiments, a solubilizing agent for use in a composition of the disclosure is at least partially soluble in the mobile phase of the composition, and/or is water-soluble or water-miscible.

In some embodiments, a composition of the disclosure includes a chaotropic agent as a solubilizing agent component. In some embodiments, the chaotropic agent is at least partially soluble in the mobile phase, and/or is water-soluble or water-miscible. Any suitable chaotropic agent known in the art may be used in compositions of the disclosure. Exemplary chaotropic agents that may be used include, without limitation, n-butanol, ethanol, a guanidinium salt, lithium perchlorate, lithium acetate, magnesium chloride, phenol, 2-propanol, sodium dodecyl sulfate, thiourea, urea, a water-soluble urea derivative, and any combination thereof. In some embodiments, the chaotropic agent in the composition includes urea or a water-soluble urea derivative (e.g., any of thiourea, hydroxyurea, dimethylurea, and any combination thereof). In other embodiments, the chaotropic agent in the composition includes a guanidinium salt (e.g., any of guanidinium chloride, guanidinium sulfate, guanidine carbonate, guanidinium nitrate, guanidinium isothiocyanate, guanidinium thiocyanate, and any combination thereof). In some embodiments, the chaotropic agent (e.g., urea, a urea derivative, and/or a guanidinium salt) is present in the composition at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, the chaotropic agent (e.g., urea, a urea derivative, and/or a guanidinium salt) is present in the composition at a concentration of between about 1% and about 50%, between about 1% and about 40%, between about 5% and about 30%, between about 10% and about 25%, or between about 10% and about 20%, w/w, including any value within each of the recited ranges. In some embodiments, the chaotropic agent (e.g., urea, a urea derivative, and/or a guanidinium salt) is present in the composition at a concentration of about 5%, about 6%, about 7%, about 8%, about 9%, about 10%, about 11%, about 12%, about 13%, about 14%, about 15%, about 16%, about 17%, about 18%, about 19%, or about 20%, w/w. In some embodiments, the chaotropic agent (e.g., urea, a urea derivative, and/or a guanidinium salt) is present in the composition at a concentration of about 15% w/w. In some embodiments, the chaotropic agent comprises urea at a concentration of about 15% w/w in the composition.

In some embodiments, a composition of the disclosure includes a buffering agent as a solubilizing agent component. In some embodiments, the buffering agent is at least partially soluble in the mobile phase, and/or is water-soluble or water-miscible. Any suitable buffering agent known in the art may be used in compositions of the disclosure. Exemplary buffering agents that may be used include, without limitation, a MES, Bis-Tris, ADA, ACES, PIPES, MOPSO, Bis-Tris Propane, BES, MOPS, TES, HEPES, DIPSO, MOBS, TAPSO, Tris, HEPPSO, POPSO, TEA, EPPS, HEPPS, Tricine, Gly-Gly, Bicine, HEPBS, TAPS, AMPD, TABS, AMPSO, CHES, CAPSO, AMP, CAPS, CABS, phosphate, maleate, glycine, citrate, formate, succinate, acetate, propionate, pyridine, piperazine, cacodylate, histidine, ethanolamine, carbonate, imidazole, pyrophosphate, hydrazine, taurine (AES), borate, ammonium hydroxide, methylamine, piperidine, or malate buffers, and any combination thereof. In some embodiments, the buffering agent in the composition includes a TAPS, HEPES, or MOPS buffer, or any combination thereof. In some embodiments, the buffering agent (e.g., a TAPS, HEPES, or MOPS buffer, or any combination thereof) is present in the composition at a concentration of at least about 1%, at least about 5%, at least about 10%, at least about 15%, at least about 20%, at least about 25%, at least about 30%, at least about 35%, at least about 40%, at least about 45%, or at least about 50%, w/w. In some embodiments, the buffering agent (e.g., a TAPS, HEPES, or MOPS buffer, or any combination thereof) is present in the composition at a concentration of between about 1% and about 50%, between about 1% and about 40%, between about 5% and about 30%, between about 10% and about 25%, or between about 10% and about 20%, w/w, including any value within each of the recited ranges. In some embodiments, the buffering agent (e.g., a TAPS, HEPES, or MOPS buffer, or any combination thereof) is present in the composition at a concentration of about 5%, about 6%, about 7%, about 8%, about 9%, about 10%, about 11%, about 12%, about 13%, about 14%, about 15%, about 16%, about 17%, about 18%, about 19%, or about 20%, w/w. In some embodiments, the buffering agent (e.g., a TAPS, HEPES, or MOPS buffer, or any combination thereof) is present in the composition at a concentration of about 15% w/w.

### d. Particles

Compositions of the present disclosure may be suitable for use with any types of particles known in the art, such as any particles that would benefit from any one or all of the properties of the presently disclosed compositions, as described herein (e.g., low or no water content, adherence to surfaces, high viscosity, high density, sustained and/or enhanced stability and/or functionality, sustained and/or enhanced ligand binding, and/or microbiological stability).

In some embodiments, the particles in a composition of the present disclosure may comprise one or more microparticles, nanoparticles, microspheres, granules, fibers, beads, paramagnetic beads, magnetic beads, microbeads, nanobeads, materials in amorphous form, powders, or any combination thereof. Exemplary and non-limiting types of particles that may be included in composition of the present disclosure include silica beads, silica gel beads, controlled pore glass beads, magnetic beads, glass beads, borosilicate beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof.

In some embodiments, the particles in a composition of the present disclosure may be functionalized. Any suitable functional groups or moieties may be appended, attached, bound or otherwise associated with the particles, including, without limitation, a carboxyl, amino, hydroxyl, silica, streptavidin, enzyme, amine, thiol, diol, polymer, an antibody or antibody fragment, a nucleic acid, an oligonucleotide, a peptide, a polypeptide, or any combination thereof.

Particles suitable for use in compositions of the disclosure may have a variety of sizes. For example, particles in a composition of the disclosure may have a dimension, such as a cross-sectional diameter, in the nanometer range, such as between about 1 nm and about 1000 nm, between about 10 nm and about 900 nm, between about 20 nm and about 800 nm, between about 30 nm and about 700 nm, between about 40 nm and about 600 nm, between about 50 nm and about 500 nm, between about 60 nm and about 400 nm, between about 70 nm and about 300 nm, between about 80 nm and about 200 nm, or between about 90 nm and about 100 nm, including any value within each of the recited ranges. In some embodiments, the particles may have a dimension, such as a cross-sectional diameter, between about 100 nm and about 200 nm, including any value within the recited range, such as any of about 105 nm, about 110 nm, about 115 nm, about 120 nm, about 125 nm, about 130 nm, about 135 nm, about 140 nm, about 145 nm, about 150 nm, about 155 nm, about 160 nm, about 165 nm, about 170 nm, about 175 nm, about 180 nm, about 185 nm, about 190 nm, about 195 nm, or about 200 nm. In another example, particles in a composition of the disclosure may have a dimension, such as a cross-sectional diameter, in the micrometer range, such as any of between about 1 $\mu$m and about 1000 $\mu$m, between about 10 $\mu$m and about 900 $\mu$m, between about 20 $\mu$m and about 800 $\mu$m, between about 30 $\mu$m and about 700 $\mu$m, between about 40 $\mu$m and about 600 $\mu$m, between about 50 $\mu$m and about 500 $\mu$m, between about 60 $\mu$m and about 400 $\mu$m, between about 70 $\mu$m and about 300 $\mu$m, between about 80 $\mu$m and about 200 $\mu$m, or between about 90 $\mu$m and about 100 $\mu$m, including any value within each of the recited ranges. In some embodiments, particles in a composition of the disclosure may have a dimension, such as a cross-sectional diameter, of between about 1 $\mu$m and about 10 $\mu$m, including any value within the recited range, such as any of about 1 $\mu$m, about 2 $\mu$m, about 3 $\mu$m, about 4 $\mu$m, about 5 $\mu$m, about 6 $\mu$m, about 7 $\mu$m, about 8 $\mu$m, about 9 $\mu$m, or about 10 $\mu$m. In some embodiments, particles in a composition of the disclosure may have a dimension, such as a cross-sectional diameter, of between about 10 $\mu$m and about 100 $\mu$m, including any value within the recited range, such as any of about 10 $\mu$m, about 20 $\mu$m, about 30 $\mu$m, about 40 $\mu$m, about 50 $\mu$m, about 60 $\mu$m, about 70 $\mu$m, about 80 $\mu$m, about 90 $\mu$m, or about 100 $\mu$m. In some embodiments, particles in a composition of the disclosure may have a dimension, such as a cross-sectional diameter, of between about 100 $\mu$m and about 1000 $\mu$m, including any value within the recited range, such as any of about 100 $\mu$m, about 200 $\mu$m, about 300 $\mu$m, about 400 $\mu$m, about 500 $\mu$m, about 600 $\mu$m, about 700 $\mu$m, about 800 $\mu$m, about 900 $\mu$m, or about 1000 $\mu$m. In another example, particles in a composition of the disclosure may have a dimension, such as a cross-sectional diameter, in the millimeter range, such as any of between about 1 mm and about 20 mm, between about 1 mm and about 15 mm, between about 1 mm about 10 mm, or between about 1 mm and about 5 mm, including any value within each of the recited ranges. In some embodiments, particles in a composition of the disclosure may have a dimension, such as a cross-sectional diameter, of between about 1 mm and about 10 mm, including any value within the recited range, such as any of about 1 mm, about 2 mm, about 3 mm, about 4 mm, about 5 mm, about 6 mm, about 7 mm, about 8 mm, about 9 mm, or about 10 mm. In some embodiments, a composition of the disclosure may include a mixture of one or more particles having different sizes, such as having different dimensions (e.g., different cross-sectional diameters).

In some embodiments, the particles in a composition of the present disclosure may be capable of or be configured to bind a ligand. Exemplary ligands include, without limitation, nucleic acids, cells, viral particles, polypeptides, lipids, carbohydrates, or small molecules.

In some embodiments, the particles in a composition of the present disclosure may be capable of or be configured to bind nucleic acids, such as DNA, cDNA and/or RNA. In such cases, the particles may be silica or silica-coated particles, or particles associated with one or more oligonucleotides. The particles may be beads, microparticles, microspheres, microbeads, nanobeads and the like. The particles may also be magnetic or paramagnetic.

In other embodiments, the particles in a composition of the present disclosure may be capable of or be configured to bind one or more proteins or polypeptides. In such cases, the particles may be associated with protein A/G or be carboxylate modified. The particles may be beads, microparticles, microspheres, microbeads, nanobeads, and the like. The particles may also be magnetic or paramagnetic.

In some embodiments, the particles in a composition of the present disclosure may be used to aid in lysis of cells (e.g., mechanical lysis) and/or in homogenization of tissues or other sample types. In such cases, the particles may be mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof.

Compositions of the present disclosure may include the particles in any suitable amount. In some cases, compositions of the disclosure may comprise particles in a concentration of any of between about 0.1% and about 99%, between about 1% and about 90%, between about 1% and about 80%, between about 1% and about 70%, between about 1% and about 60%, between about 1% and about 50%, between about 1% and about 40%, between about 1% and about 30%, between about 1% and about 20%, or between about 1% and about 10%, weight by volume (w/v), including any value within each of the recited ranges. In some cases, compositions of the disclosure may comprise particles in a concentration of any of at least about 0.25%, at least about 0.5%, at least about 0.75%, at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, at least about 20%, at least about 21%, at least about 22%, at least about 23%, at least about 24%, at least about 25%, at least about 26%, at least about 27%, at least about 28%, at least about 29%, or at least about 30%, w/v. In some cases, compositions of the disclosure may comprise particles in a concentration of any of between about 1% and about 20% w/v, including any value within the recited range such as any of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% w/v. In some embodiments, a composition of the disclosure comprises particles in a concentration of about 10% w/v.

### e. Additional Agents

In some embodiments, a composition of the present disclosure may comprise one or more additional agents, for example, depending on the intended use of the composition, storage conditions or length of time, specific type of particles in the composition, and the like.

Exemplary agents that may be included in compositions of the disclosure include, without limitation, one or more of: buffers such as phosphate buffers, citrate buffers, and buffers based on other organic acids; antioxidants, including ascorbic acid, methionine, glutathione, lipoic acid, uric acid, carotenes, $\alpha$-tocopherol, ubiquinol, tocopherols, propyl gallate, tertiary butylhydroquinone, butylated hydroxyanisole, butylated hydroxytoluene, phenolic antioxidants such as stilbenes, flavonoids, and hydroxycinnamic acid, N,N'-di-2-butyl-1,4-phenylenediamine, N,N'-di-2-butyl-1,4-phenylenediamine, 2,6-di-tert-butyl-4-methylphenol, 2,4-dimethyl-6-tert-butylphenol, 2,4-dimethyl-6-tert-butylphenol, 2,4-dimethyl-6-tert-butylphenol, 2,6-di-tert-butyl-4-methylphenol, 2,6-di-tert-butylphenol, aminic antioxidants, phytic acid, oxalic acid, tannins, and combinations thereof; preservatives such as octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, or m-cresol; low molecular weight polypeptides (e.g., less than about 10 residues); proteins such as serum albumin, gelatin, or immunoglobulins; reducing agents, such as lithium aluminum hydride, Red-Al, hydrogen without or with a suitable catalyst (e.g. a Lindlar catalyst), sodium amalgam, sodium-lead alloy, zinc amalgam, diborane, sodium borohydride, ferrous compounds that contain the Fe2+ ion (e.g., iron(II) sulfate), stannous compounds that contain the Sn2+ ion (e.g., tin(II) chloride), sulfur dioxide, sulfite compounds, dithionates, thiosulfates, iodides, hydrogen peroxide, hydrazine, diisobutylaluminium hydride, oxalic acid, formic acid, ascorbic acid, reducing sugars (such as erythrose), phosphites, hypophosphites, phosphorous acid, dithiothreitol (DTT), carbon monoxide (CO), cyanides, carbon, tris-2-carboxyethylphosphine hydrochloride (TCEP), or any combination thereof; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as ethylenediaminetetraacetic acid disodium salt, ethyleneglycol-O, O'-bis(2-aminoethyl)-N, N, N', N'-tetraacetic acid, N-(2-hydroxyethyl)ethylenediamine-N, N', N'-triacetic acid trisodium salt, nitrilotriacetic acid, 2,2'-bipyridyl, dimercaptopropanol, ethylenediaminotetraacetic acid, ethylenedioxy-diethylene-dinitrilo-tetraacetic acid, ethylene glycol-bis-(2-aminoethyl)-N,N,N', N'-tetraacetic acid, ionophores, nitrilotriacetic acid, orthophenanthroline, salicylic acid, triethanolamine, and combinations thereof; sugars such as sucrose, mannitol, trehalose or sorbitol; salt-forming counter-ions such as sodium; metal complexes (e.g., Znprotein complexes); surfactants such as non-ionic surfactants; or polymers such as polyethylene glycol (PEG).

In some specific embodiments, a composition of the disclosure may comprise an antioxidant, a reducing agent, a chelating agent, or any combination thereof.

In some embodiments, compositions of the present disclosure are microbiologically stable, and may not require use of anti-microbial agents or preservatives. Thus, in some embodiments, a composition of the present disclosure does not comprise an anti-microbial agent and/or preservative.

In some cases, e.g., depending on the intended use of a composition of the disclosure, it may be undesirable to include surfactants or detergents, or high levels of surfactants or detergents, in compositions of the present disclosure due to, for example, foaming. Accordingly, in some cases, compositions of the present disclosure do not contain a surfactant or a detergent, and in other cases, a composition of the present disclosure includes less than about 25%, less than about 20%, less than about 15%, less than about 10%, less than about 5%, less than about 2.5%, or less than about 1% of surfactant or detergent.

### f. Exemplary compositions

In some embodiments, a composition of the disclosure comprises a plurality of particles, a mobile phase, and a viscosity enhancing agent. In some embodiments, the mobile phase is at a concentration of about 70% and the viscosity enhancing agent is at a concentration of about 30%, w/w. In some embodiments, the mobile phase is at a concentration of about 80% and the viscosity enhancing agent is at a concentration of about 20%, w/w. In some embodiments, the mobile phase is at a concentration of about 90% and the viscosity enhancing agent is at a concentration of about 10%, w/w. In some embodiments, the mobile phase and the viscosity enhancing agent are in a ratio of about 2.33: 1, w/w. In some embodiments, the mobile phase and the viscosity enhancing agent are in a ratio of about 4:1, w/w. In some embodiments, the mobile phase and the viscosity enhancing agent are in a ratio of about 9:1, w/w. In some embodiments, the mobile phase comprises a glycerol suspending agent. In some embodiments, the viscosity enhancing agent comprises PEG, such as PEG 4000, PEG 8000, PEG 10000 or PEG 20000. In some embodiments, the viscosity enhancing agent comprises PEG 8000. In some specific embodiments, the composition comprises: (a) a plurality of particles, glycerol at a concentration of about 70%, and PEG 8000 at a concentration of about 30%, w/w; (b) a plurality of particles, glycerol at a concentration of about 80%, and PEG 8000 at a concentration of about 20%, w/w; or (c) a plurality of particles, glycerol at a concentration of about 90%, and PEG 8000 at a concentration of about 10%, w/w. In some specific embodiments, the composition comprises a plurality of particles and: (a) glycerol and PEG 8000 in a ratio of about 2.33:1, w/w; (b) glycerol and PEG 8000 in a ratio of about 4:1, w/w; or (c) glycerol and PEG 8000 in a ratio of about 9:1, w/w.

In some embodiments, a composition of the disclosure comprises a plurality of particles, a mobile phase, and a solubilizing agent. In some embodiments, the mobile phase is at a concentration of about 70% and the solubilizing agent is at a concentration of about 30%, w/w. In some embodiments, the mobile phase is at a concentration of about 80% and the solubilizing agent is at a concentration of about 20%, w/w. In some embodiments, the mobile phase is at a concentration of about 90% and the solubilizing agent is at a concentration of about 10%, w/w. In some embodiments, the mobile phase and the solubilizing agent are in a ratio of about 2.33: 1, w/w. In some embodiments, the mobile phase and the solubilizing agent are in a ratio of about 4:1, w/w. In some embodiments, the mobile phase and the solubilizing agent are in a ratio of 9:1, w/w. In some embodiments, the mobile phase comprises a glycerol suspending agent. In some embodiments, the solubilizing agent comprises a chaotropic agent such as urea, a water-soluble urea derivative, or a guanidinium salt. In some embodiments, the solubilizing agent comprises urea. In some specific embodiments, the composition comprises: (a) a plurality of particles, glycerol at a concentration of about 70%, and urea at a concentration of about 30%, w/w; (b) a plurality of particles, glycerol at a concentration of about 80%, and urea at a concentration of about 20%, w/w; or (c) a plurality of particles, glycerol at a concentration of about 90%, and urea at a concentration of about 10%, w/w. In some specific embodiments, the composition comprises a plurality of particles and: (a) glycerol and urea in a ratio of about 2.33:1, w/w; (b) glycerol and urea in a ratio of about 4:1, w/w; or (c) glycerol and urea in a ratio of about 9:1, w/w.

In some embodiments, a composition of the disclosure comprises a plurality of particles, a mobile phase, a viscosity enhancing agent, and a solubilizing agent. In some embodiments, the mobile phase is at a concentration of about 70%, the viscosity enhancing agent is at a concentration of about 25%, and the solubilizing agent is at a concentration of about 5%, w/w. In some embodiments, the mobile phase is at a concentration of about 70%, the viscosity enhancing agent is at a concentration of about 20%, and the solubilizing agent is at a concentration of about 10%, w/w. In some embodiments, the mobile phase is at a concentration of about 70%, the viscosity enhancing agent is at a concentration of about 15%, and the solubilizing agent is at a concentration of about 15%, w/w. In some embodiments, the mobile phase is at a concentration of about 70%, the viscosity enhancing agent is at a concentration of about 10%, and the solubilizing agent is at a concentration of about 20%, w/w. In some embodiments, the mobile phase is at a concentration of about 70%, the viscosity enhancing agent is at a concentration of about 5%, and the solubilizing agent is at a concentration of about 25%, w/w. In some embodiments, the mobile phase is at a concentration of about 80%, the viscosity enhancing agent is at a concentration of about 15%, and the solubilizing agent is at a concentration of about 5%, w/w. In some embodiments, the mobile phase is at a concentration of about 80%, the viscosity enhancing agent is at a concentration of about 10%, and the solubilizing agent is at a concentration of about 10%, w/w. In some embodiments, the mobile phase is at a concentration of about 80%, the viscosity enhancing agent is at a concentration of about 5%, and the solubilizing agent is at a concentration of about 15%, w/w. In some embodiments, the mobile phase is at a concentration of about 90%, the viscosity enhancing agent is at a concentration of about 5%, and the solubilizing agent is at a concentration of about 5%, w/w. In some embodiments, the mobile phase, the viscosity enhancing agent, and the solubilizing agent are in a ratio of about 14:5:1, w/w. In some embodiments, the mobile phase, the viscosity enhancing agent, and the solubilizing agent are in a ratio of about 7:2:1, w/w. In some embodiments, the mobile phase, the viscosity enhancing agent, and the solubilizing agent are in a ratio of about 4.7:1:1, w/w. In some embodiments, the mobile phase, the viscosity enhancing agent, and the solubilizing agent are in a ratio of about 7:1:2, w/w. In some embodiments, the mobile phase, the viscosity enhancing agent, and the solubilizing agent are in a ratio of about 14:1:5, w/w. In some embodiments, the mobile phase, the viscosity enhancing agent, and the solubilizing agent are in a ratio of about 16:3:1, w/w. In some embodiments, the mobile phase, the viscosity enhancing agent, and the solubilizing agent are in a ratio of about 8:1:1, w/w. In some embodiments, the mobile phase, the viscosity enhancing agent, and the solubilizing agent are in a ratio of about 16:1:3, w/w. In some embodiments, the mobile phase, the viscosity enhancing agent, and the solubilizing agent are in a ratio of about 18:1:1, w/w. In some embodiments, the mobile phase comprises a glycerol suspending agent. In some embodiments, the solubilizing agent comprises a chaotropic agent such as urea, a water-soluble urea derivative, or a guanidinium salt. In some embodiments, the solubilizing agent comprises urea. In some embodiments, the viscosity enhancing agent comprises PEG, such as PEG 4000, PEG 8000, PEG 10000 or PEG 20000. In some embodiments, the viscosity enhancing agent comprises PEG 8000. In some embodiments, the suspending agent of the mobile phase comprises glycerol, the viscosity enhancing agent comprises PEG 8000, and the solubilizing agent comprises urea. In some specific embodiments, the composition comprises: (a) a plurality of particles, glycerol at a concentration of about 70%, PEG 8000 at a concentration of about 25%, and urea at a concentration of about 5%, w/w; (b) a plurality of particles, glycerol at a concentration of about 70%, PEG 8000 at a concentration of about 20%, and urea at a concentration of about 10%, w/w; (c) a plurality of particles, glycerol at a concentration of about 70%, PEG 8000 at a concentration of about 15%, and urea at a concentration of about 15%, w/w; (d) a plurality of particles, glycerol at a concentration of about 70%, PEG 8000 at a concentration of about 10%, and urea at a concentration of about 20%, w/w; (e) a plurality of particles, glycerol at a concentration of about 70%, PEG 8000 at a concentration of about 5%, and urea at a concentration of about 25%, w/w; (f) a plurality of particles, glycerol at a concentration of about 80%, PEG 8000 at a concentration of about 15%, and urea at a concentration of about 5%, w/w; (g) a plurality of particles, glycerol at a concentration of about 80%, PEG 8000 at a concentration of about 10%, and urea at a concentration of about 10%, w/w; (h) a plurality of particles, glycerol at a concentration of about 80%, PEG 8000 at a concentration of about 5%, and urea at a concentration of about 15%, w/w; or (i) a plurality of particles, glycerol at a concentration of about 90%, PEG 8000 at a concentration of about 5%, and urea at a concentration of about 5%, w/w. In some specific embodiments, the composition comprises a plurality of particles and: (a) glycerol, PEG 8000 and urea in a ratio of about 14:5:1, w/w; (b) glycerol, PEG 8000 and urea in a ratio of about 7:2:1, w/w; (c) glycerol, PEG 8000 and urea in a ratio of about 4.7:1:1, w/w; (d) glycerol, PEG 8000 and urea in a ratio of about 7:1:2, w/w; (e) glycerol, PEG 8000 and urea in a ratio of about 14:1:5, w/w; (f) glycerol, PEG 8000 and urea in a ratio of about 16:3:1, w/w; (g) glycerol, PEG 8000 and urea in a ratio of about 8:1:1, w/w; (h) glycerol, PEG 8000 and urea in a ratio of about 16:1:3, w/w; or (i) glycerol, PEG 8000 and urea in a ratio of about 18:1:1, w/w.

### g. Physical properties

In some embodiments, compositions of the disclosure are non-aqueous or substantially non-aqueous. For example, a composition of the disclosure may have a water content of about 5% or less, about 4% or less, about 3% or less, about 2% or less, about 1% or less, about 0.5% or less, or about 0.25%, or less. In some cases, a composition of the disclosure has a water content of about 1% or less. Water content of a composition of the present disclosure may be assessed using any suitable method known in the art, such as by Karl Fischer titration or gas chromatography. In some embodiments, water content is assessed by Karl Fischer titration.

In some embodiments, a composition of the disclosure may be a liquid composition, and may have relatively high viscosity and/or density. In some cases, a composition of the disclosure may be a paste, cream or gel.

In some embodiments, a composition of the disclosure has a viscosity of any of at least about 7000 mPa·s, at least about 7100 mPa·s, at least about 7200 mPa·s, at least about 7300 mPa·s, at least about 7400 mPa·s, at least about 7500 mPa·s, at least about 7600 mPa·s, at least about 7700 mPa·s, at least about 7800 mPa·s, at least about 7900 mPa·s, or at least about 8000 mPa·s, when measured at a temperature of about 15°C; or between about 7000 mPa·s and about 8000 mPa·s, between about 7250 mPa s and about 8000 mPa s, between about 7500 mPa s and about 8000 mPa s, or between about 7750 mPa·s and about 8000 mPa·s, including any value within each of the recited ranges, when measured at a temperature of about 15°C.

In some embodiments, a composition of the disclosure has a viscosity of any of at least about 5000 mPa·s, at least about 5100 mPa·s, at least about 5200 mPa·s, at least about 5300 mPa·s, at least about 5400 mPa s, at least about 5500 mPa s, at least about 5600 mPa s, at least about 5700 mPa s, at least about 5800 mPa·s, at least about 5900 mPa·s, or at least about 6000 mPa·s, when measured at a temperature of about 20°C; or between about 5000 mPa s and about 6000 mPa s, between about 5250 mPa s and about 6000 mPa·s, between about 5500 mPa·s and about 6000 mPa·s, or between about 5500 mPa·s and about 5750 mPa·s, including any value within each of the recited ranges, when measured at a temperature of about 20°C.

In some embodiments, a composition of the disclosure has a viscosity of any of at least about 5500 mPa·s, at least about 5600 mPa·s, at least about 5700 mPa·s, at least about 5800 mPa·s, at least about 5900 mPa·s, at least about 6000 mPa·s, at least about 6100 mPa·s, at least about 6200 mPa·s, at least about 6300 mPa·s, at least about 6400 mPa·s, at least about 6500 mPa·s, at least about 6600 mPa·s, at least about 6700 mPa s, at least about 6800 mPa s, at least about 6900 mPa s, or at least about 7000 mPa·s, when measured at a temperature of about 25°C; or between about 5500 mPa·s and about 7000 mPa·s, between about 5750 mPa·s and about 6750 mPa·s, or between about 6000 mPa·s and about 6500 mPa·s, including any value within each of the recited ranges, when measured at a temperature of about 25°C.

In some embodiments, a composition of the disclosure has a viscosity of any of at least about 4200 mPa·s, at least about 4300 mPa·s, at least about 4400 mPa·s, at least about 4500 mPa·s, at least about 4600 mPa·s, at least about 4700 mPa·s, at least about 4800 mPa·s, at least about 4900 mPa·s, at least about 5000 mPa·s, at least about 5100 mPa·s, at least about 5200 mPa·s, at least about 5300 mPa·s, at least about 5400 mPa·s, or at least about 5500 mPa·s, when measured at a temperature of about 30°C; or between about 4250 mPa·s and about 5500 mPa·s, between about 4500 mPa·s and about 5500 mPa·s, between about 4750 mPa·s and about 5250 mPa·s, or between about 4750 mPa·s and about 5000 mPa·s, including any value within each of the recited ranges, when measured at a temperature of about 30°C.

In some embodiments, a composition of the disclosure has a viscosity of any of at least about 2000 mPa·s, at least about 2100 mPa·s, at least about 2200 mPa·s, at least about 2300 mPa·s, at least about 2400 mPa·s, at least about 2500 mPa·s, at least about 2600 mPa·s, at least about 2700 mPa·s, at least about 2800 mPa·s, at least about 2900 mPa·s, or at least about 3000 mPa·s, when measured at a temperature of about 35°C; or between about 2000 mPa s and about 3000 mPa s, between about 2250 mPa·s and about 2750 mPa·s, or between about 2250 mPa·s and about 2500 mPa·s, including any value within each of the recited ranges, when measured at a temperature of about 35°C.

In some embodiments, a composition of the disclosure has a viscosity of any of at least about 750 mPa s, at least about 850 mPa s, at least about 950 mPa s, at least about 1050 mPa s, at least about 1150 mPa s, at least about 1250 mPa s, at least about 1350 mPa s, at least about 1450 mPa s, at least about 1550 mPa·s, at least about 1650 mPa·s, or at least about 1750 mPa·s, when measured at a temperature of about 40°C; or between about 750 mPa s and about 1750 mPa s, between about 1000 mPa s and about 1750 mPa s, or between about 1000 mPa s and about 1500 mPa s, including any value within each of the recited ranges, when measured at a temperature of about 40°C.

In some embodiments, a composition of the disclosure has a viscosity of any of at least about 500 mPa·s, at least about 600 mPa·s, at least about 700 mPa·s, at least about 800 mPa·s, at least about 900 mPa·s, at least about 1000 mPa·s, at least about 1100 mPa·s, at least about 1200 mPa·s, at least about 1300 mPa·s, at least about 1400 mPa·s, or at least about 1500 mPa·s, when measured at a temperature of about 45°C; or between about 500 mPa·s and about 1500 mPa·s, between about 750 mPa·s and about 1250 mPa·s, or between about 1000 mPa·s and about 1250 mPa·s, including any value within each of the recited ranges, when measured at a temperature of about 45°C.

In some embodiments, a composition of the disclosure has a viscosity of any of at least about 250 mPa s, at least about 350 mPa s, at least about 450 mPa s, at least about 550 mPa s, at least about 650 mPa·s, at least about 750 mPa·s, at least about 850 mPa·s, at least about 950 mPa·s, at least about 1050 mPa·s, at least about 1150 mPa·s, at least about 1250 mPa·s, at least about 1350 mPa·s, at least about 1450 mPa s, or at least about 1550 mPa·s, when measured at a temperature of about 50°C; or between about 250 mPa s and about 1500 mPa s, between about 500 mPa s and about 1250 mPa s, or between about 500 mPa·s and about 1000 mPa·s, including any value within each of the recited ranges, when measured at a temperature of about 50°C.

In some embodiments, a composition of the disclosure has a viscosity of any of at least about 500 mPa·s, at least about 600 mPa·s, at least about 700 mPa·s, at least about 800 mPa·s, at least about 900 mPa·s, at least about 1000 mPa·s, at least about 1100 mPa·s, at least about 1200 mPa·s, at least about 1300 mPa·s, at least about 1400 mPa·s, or at least about 1500 mPa·s, when measured at a temperature of about 55°C; or between about 500 mPa·s and about 1500 mPa·s, between about 750 mPa·s and about 1250 mPa·s, or between about 1000 mPa·s and about 1250 mPa·s, including any value within each of the recited ranges, when measured at a temperature of about 55°C.

In some embodiments, a composition of the disclosure has a viscosity of any of at least about 100 mPa s, at least about 150 mPa s, at least about 200 mPa s, at least about 250 mPa s, at least about 300 mPa s, at least about 350 mPa s, at least about 400 mPa s, at least about 450 mPa s, at least about 500 mPa s, at least about 550 mPa·s, or at least about 600 mPa s, when measured at a temperature of about 60°C; or between about 100 mPa s and about 600 mPa s, between about 200 mPa s and about 500 mPa s, or between about 200 mPa s and about 400 mPa s, including any value within each of the recited ranges, when measured at a temperature of about 60°C.

Viscosity may be assessed using any suitable technique known in the art, such as using a capillary viscometer, a viscosity cup, a Zahn cup, a falling sphere viscometer, a vibrational viscometer, a rotational viscometer, or a consistometer. In some embodiments, viscosity is assessed using a rotational viscometer. In some cases, viscosity may be assessed at a spindle rotational speed of about 10 revolutions per minute (RPM). Viscosity may also be assessed based on adherence of the composition to a surface, such as a plastic (e.g., polypropylene, polyethylene, polypropylene copolymer, polymethylpentene, polyvinyl chloride, polyethylene terephthalate G copolymer, polycarbonate, polysulfone, polystyrene, or teflon) or glass surface. In some cases, viscosity is assessed based on adherence of the composition to a polypropylene surface.

In some embodiments, a composition of the disclosure has a density of at least about 1 g/mL, such as any of about 1 g/mL, about 1.2 g/ml, about 1.3 g/mL, about 1.4 g/mL, about 1.5 g/mL, about 1.6 g/mL, about 1.7 g/mL, about 1.8 g/mL, about 1.9 g/mL, about 2 g/mL, or more. In some embodiments, the composition has a density of between about 0.5 g/mL and about 2 g/mL, between about 1 g/mL and about 2 g/mL, between about 1 g/mL and about 1.5 g/mL, or between about 1.1 g/mL and about 1.5 g/mL, including any value within each of the recited ranges. In some cases, the composition has a density of about 1.3 g/mL. Density of a composition of the disclosure may be assessed using any suitable method known in the art, such as using an analytical balance, a hydrometer, a pycnometer, a specific-gravity bottle, and the like. In some embodiments, density is assessed using an analytical balance.

In some embodiments, a composition of the disclosure may have a pH of between about 5 and about 10, between about 6 and about 9, or between about 7 and about 8, including any value within each of the recited ranges. In some embodiments, a composition of the present disclosure has a pH of between about 7 and about 8, such as any of about 7, about 7.1, about 7.2, about 7.3, about 7.4, about 7.5, about 7.6, about 7.7, about 7.8, about 7.9, or about 8. The pH of a composition may be assessed using any suitable method known in the art, such as using colorimetric methods (e.g., using pH indicator paper or solutions), or using a pH meter.

In some embodiments, compositions of the present disclosure display adherence, at least to some extent, to plastic surfaces (e.g., polypropylene, polyethylene, polypropylene copolymer, polymethylpentene, polyvinyl chloride, polyethylene terephthalate G copolymer, polycarbonate, polysulfone, polystyrene, or teflon), and/or glass surfaces. In some embodiments, compositions of the present disclosure display adherence, at least to some extent, to polypropylene surfaces.

### h. Stability and functionality

In some embodiments, a composition of the present disclosure may support and/or enhance the stability of particles in the composition. Stability of particles in a composition of the disclosure may be assessed based on integrity of the particles (e.g., degree of degradation or fragmentation of the particles), degree of aggregation of the particles, and/or retention of ability to bind to a ligand.

In some cases, particles in a composition of the present disclosure are stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months. In some embodiments, a composition of the disclosure is stable for at least about 1 month. In some embodiments, a composition of the disclosure is stable for at least about 3 months. In some embodiments, a composition of the disclosure is stable for at least about 4.5 months. In some embodiments, a composition of the disclosure is stable for at least about 6 months. In some embodiments, a composition of the disclosure is stable for at least about 9 months. In some embodiments, a composition of the disclosure is stable for at least about 12 months. In some embodiments, a composition of the disclosure is stable for at least about 15 months. In some embodiments, a composition of the disclosure is stable for at least about 18 months. In some embodiments, a composition of the disclosure is stable for at least about 21 months. In some embodiments, a composition of the disclosure is stable for at least about 24 months. In some embodiments, a composition of the disclosure is stable for at least about 36 months.

In some embodiments, less than any of about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, or about 1% of particles in a plurality of particles in a composition of the disclosure are degraded and/or fragmented, for example, as compared to baseline or as compared to corresponding particles in an aqueous solution. In some embodiments, less than any of about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, or about 1% of particles in a plurality of particles in a composition of the disclosure are degraded and/or fragmented following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months, as compared to baseline or as compared to corresponding particles in an aqueous solution.

In some embodiments, a composition of the disclosure may decrease degradation and/or fragmentation of particles in the composition, e.g., as compared to corresponding particles in an aqueous solution, by at least any of about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. In some embodiments, said decrease in degradation and/or fragmentation of the particles is assessed following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months.

In some embodiments, less than any of about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, or about 1% of particles in a plurality of particles in a composition of the disclosure are aggregated, for example, as compared to baseline or as compared to corresponding particles in an aqueous solution. In some embodiments, less than any of about 90%, about 85%, about 80%, about 75%, about 70%, about 65%, about 60%, about 55%, about 50%, about 45%, about 40%, about 35%, about 30%, about 25%, about 20%, about 15%, about 10%, about 5%, or about 1% of particles in a plurality of particles in a composition of the disclosure are aggregated following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months, as compared to baseline or as compared to corresponding particles in an aqueous solution.

In some embodiments, a composition of the disclosure may decrease aggregation of particles in the composition, e.g., as compared to particles in an aqueous solution, by at least any of about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. In some embodiments, said decrease in aggregation of the particles is assessed following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months.

In some embodiments, particles in a composition of the present disclosure retain any of at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of ligand binding ability, as compared to baseline or as compared to corresponding particles in an aqueous solution. In some embodiments, the particles retain any of at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months, as compared to baseline or as compared to corresponding particles in an aqueous solution.

In some embodiments, a composition of the disclosure enhances ligand binding of particles in the composition, e.g., as compared to particles in an aqueous solution, by at least any of about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. In some embodiments, a composition of the disclosure enhances ligand binding of particles in the composition, e.g., as compared to particles in an aqueous solution, by at least any of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%. In some embodiments, said enhancement of ligand binding of the particles is assessed following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months.

In some specific embodiments, the plurality of particles in a composition of the disclosure comprises one or more particles configured to or capable of binding to nucleic acid ligand(s), e.g., DNA, cDNA, and/or RNA. In such cases, stability of the particles in the composition may be assessed based on nucleic acid binding ability of the particles in the composition. In some embodiments, such nucleic acid binding particles are silica particles, such as magnetic or paramagnetic silica particles. In some embodiments, particles in a composition of the present disclosure retain any of at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of nucleic acid binding ability, as compared to baseline or as compared to corresponding particles in an aqueous solution. In some embodiments, the particles retain any of at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99%, or 100% of nucleic acid binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months, as compared to baseline. In some embodiments, a composition of the disclosure may enhance nucleic acid binding of particles in the composition, e.g., as compared to particles in an aqueous solution, by at least any of about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%. In some embodiments, a composition of the disclosure enhances nucleic acid binding of particles in the composition, e.g., as compared to particles in an aqueous solution, by at least any of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%. In some embodiments, said enhancement of nucleic acid binding of the particles is assessed following storage for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months.

Particle stability (e.g., assessed based on integrity of the particles, degree of aggregation of the particles, and/or retention of ability to bind to a ligand) may be assessed under various conditions, such as normal atmospheric conditions, reduced oxygen conditions, in nitrogen gas, and at various temperatures, such as between about -80°C and about 100°C, between about -20°C and about 90°C, between about - 10°C and about 80°C, between about 0°C and about 70°C, between about 2°C and about 60°C, between about 4°C and about 50°C, between about 5°C and about 40°C, between about 10°C and about 30°C, between about 15°C and about 30°C, or between about 20°C and about 30°C, including any value within each of the recited ranges. In some embodiments, particle stability may be assessed at about room temperature, e.g., at any of about 20°C, about 21°C, about 22°C, about 23°C, about 24°C, or about 25°C. In some embodiments, particle stability is assessed under normal atmospheric conditions. In some embodiments, particle stability is assessed under reduced oxygen conditions (e.g., relative to normal atmospheric conditions). In some embodiments, particle stability is assessed in nitrogen gas. In some embodiments, particle stability is assessed at a temperature of about 25°C under normal atmospheric conditions, under reduced oxygen conditions (e.g., relative to normal atmospheric conditions), or in nitrogen gas.

Compositions of the present disclosure have the advantage of being microbiologically stable. In some cases, a composition of the disclosure is microbiologically stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at least about 22 months, at least about 23 months, at least about 24 months, at least about 25 months, at least about 26 months, at least about 27 months, at least about 28 months, at least about 29 months, at least about 30 months, at least about 31 months, at least about 32 months, at least about 33 months, at least about 34 months, at least about 35 months, at least about 36 months, at least about 37 months, at least about 38 months, at least about 39 months, at least about 40 months, at least about 41 months, at least about 42 months, at least about 43 months, at least about 44 months, at least about 45 months, at least about 46 months, at least about 47 months, or at least about 48 months. Microbiological stability (e.g., assessed based on resistance to microbial growth) may be assessed under various conditions, such as normal atmospheric conditions, reduced oxygen conditions, in nitrogen gas, and at various temperatures, such as between about -80°C and about 100°C, between about -20°C and about 90°C, between about -10°C and about 80°C, between about 0°C and about 70°C, between about 2°C and about 60°C, between about 4°C and about 50°C, between about 5°C and about 40°C, between about 10°C and about 30°C, between about 15°C and about 30°C, or between about 20°C and about 30°C, including any value within each of the recited ranges. In some embodiments, microbiological stability may be assessed at about room temperature, e.g., at any of about 20°C, about 21°C, about 22°C, about 23°C, about 24°C, or about 25°C. In some embodiments, microbiological stability may be assessed under normal atmospheric conditions. In some embodiments, microbiological stability may be assessed under reduced oxygen conditions (e.g., relative to normal atmospheric conditions). In some embodiments, microbiological stability may be assessed in nitrogen gas. In some embodiments, microbiological stability is assessed at a temperature of about 25°C under normal atmospheric conditions, under reduced oxygen conditions (e.g., relative to normal atmospheric conditions), or in nitrogen gas.

## III. Methods for preparing compositions

Also provided herein are methods for preparing a composition comprising a plurality of particles. The methods described herein may be used to prepare any of the compositions of the present disclosure.

In some embodiments, a composition comprising a plurality of particles is prepared by suspending the particles in a formulation comprising one, two, or all of: a mobile phase (such as any of the mobile phases provided herein), a viscosity enhancing agent (such as any of the viscosity enhancing agents provided herein), and a solubilizing agent (such as any of the solubilizing agents provided herein). In some embodiments, the particles are suspended in a formulation comprising: a mobile phase (such as any of the mobile phases provided herein) and a solubilizing agent (such as any of the solubilizing agents provided herein). In some embodiments, the particles are suspended in a formulation comprising: a mobile phase (such as any of the mobile phases provided herein), and viscosity enhancing agent (such as any of the viscosity enhancing agents provided herein). In some embodiments, the particles are suspended in a formulation comprising: a viscosity enhancing agent (such as any of the viscosity enhancing agents provided herein), and a solubilizing agent (such as any of the solubilizing agents provided herein). In some embodiments, the particles are suspended in a formulation comprising: a mobile phase (such as any of the mobile phases provided herein), a viscosity enhancing agent (such as any of the viscosity enhancing agents provided herein), and a solubilizing agent (such as any of the solubilizing agents provided herein).

In some embodiments, the particles to be suspended in said formulation are or have been previously dehydrated or lyophilized. In other embodiments, the methods for preparing a composition comprising a plurality of particles comprise a step of dehydrating or lyophilizing the particles prior to suspending the particles in said formulation. Such dehydration or lyophilization may be performed, for example, in cases where the particles are provided in an aqueous suspension. Dehydration or lyophilization of the particles may be carried out using any suitable method known in the art. In some embodiments, the particles are lyophilized by manifold drying, batch drying or bulk drying. In other embodiments, the particles are dehydrated by gradual phase change (e.g., from aqueous phase to organic phase) or spray drying.

In some embodiments, dehydration of the particles comprises suspending the particles in a first organic solvent. In some embodiments, the method comprises performing one or more washes with the organic solvent (e.g., one, two, three, four, or more washes). In some embodiments, the method comprises: (i) separating the particles stored in an aqueous solution from the aqueous solution, and (ii) suspending the separated particles in the first organic solvent. In some embodiments, one or more additional washes with the first organic solvent are performed, for example, by (i) separating the particles from the first organic solvent, and (ii) resuspending the separated particles in a further volume of first organic solvent. In some embodiments, at least 1, at least 2, at least, 3, at least 4, at least 5, at least 6, at least 7, at least 8, at least 9, at least 10, or more, washes with the first organic solvent are performed. In some cases, two washes with organic solvent are performed.

In other embodiments, dehydration of the particles comprises performing a gradual phase change, from aqueous phase to organic phase, by washing the particles with solutions having increasing concentrations of organic solvent. In some embodiments, the dehydrating comprises suspending the particles in a first organic solvent in gradually increasing concentrations of the solvent. For example, in some embodiments, the particles are dehydrated by (i) separating the particles stored in an aqueous solution from said aqueous solution, and (ii) suspending the separated particles in a series of volumes of a mixture comprising the first organic solvent, wherein each volume in the series has increasing amounts of the organic solvent, e.g., ranging from about 20% to about 100%, about 30% to about 100%, about 40% to about 100%, about 50% to about 100%, about 60% to about 100%, about 70% to about 100%, or about 80% to about 100%. For example, a first volume of the series of volumes may comprise an organic solvent concentration of between about 20% and about 80%, including any value within the recited range, and a final volume of the series of volumes may comprise an organic solvent concentration of about 99% or 100%. In another example, the first volume of the series of volumes comprises an organic solvent concentration of about 50% and the final volume of the series of volumes comprises an organic solvent concentration of about 99% or 100%. In some embodiments, the series of volumes includes between about 2 and about 15 volumes, between about 2 and about 10 volumes, or between about 5 and about 7 volumes, including any value within each of the recited ranges. As an example, in a case where the series of volumes includes 5 volumes, the first volume may include an organic solvent concentration of about 52%, the second volume may include an organic solvent concentration of about 64%, the third volume may include an organic solvent concentration of about 76%, the fourth volume may include an organic solvent concentration of about 88%, and the fifth volume may include an organic solvent concentration of about 99% or 100%. In a case where the series of volumes includes 6 volumes, the first volume may include an organic solvent concentration of about 50%, the second volume may include an organic solvent concentration of about 60%, the third volume may include an organic solvent concentration of about 70%, the fourth volume may include an organic solvent concentration of about 80%, the fifth volume may include an organic solvent concentration of about 90%, and the sixth volume may include an organic solvent concentration of about 99% or 100%. In a case where the series of volumes includes 7 volumes, the first volume may include an organic solvent concentration of about 50%, the second volume may include an organic solvent concentration of about 55%, the third volume may include an organic solvent concentration of about 60%, the fourth volume may include an organic solvent concentration of about 65%, the fifth volume may include an organic solvent concentration of about 70%, the sixth volume may include an organic solvent concentration of about 85%, and the seventh volume may include an organic solvent concentration of about 99% or 100%.

In some embodiments, the first organic solvent is an organic solvent that is miscible with water and/or is soluble in water.

In some cases, the first organic solvent comprises an aprotic solvent, such as a polar aprotic solvent. In some embodiments, the polar aprotic solvent is a dipolar aprotic solvent. Any suitable aprotic solvent known in the art may be used. Exemplary aprotic solvents that may be used include, without limitation, dimethylsulfoxide (DMSO), dimethylsulfone, tetrahydrofuran (THF), diphenylsulfone (DPS), diethylsulfoxide, diethylsulfone, diisopropylsulfone, propylene carbonate, acetonitrile, sulfolane, tetrahydrothiophene-1-monoxide, N,N-dimethylacetamide (DMAc), N,N-dimethylformamide (DMF), N-methyl-2-pyrrolidinone (NMP), as well as any combination thereof. In some embodiments, the aprotic solvent comprises sulfolane. In some embodiments, the aprotic solvent comprises DMSO.

In some embodiments, the first organic solvent is a water miscible P-type or E-type glycol ether, such as any of ethylene glycol monomethyl ether, ethylene glycol monoethyl ether, ethylene glycol monopropyl ether, ethylene glycol monoisopropyl ether, ethylene glycol monobutyl ether, ethylene glycol monophenyl ether, ethylene glycol monobenzyl ether, propylene glycol methyl ether, diethylene glycol ethyl ether, diethylene glycol monomethyl ether, diethylene glycol monoethyl ether, diethylene glycol mono-n-butyl ether, dipropyleneglycol methyl ether, ethylene glycol dimethyl ether, ethylene glycol diethyl ether, ethylene glycol dibutyl ether, ethylene glycol methyl ether acetate, ethylene glycol monoethyl ether acetate, ethylene glycol monobutyl ether acetate, propylene glycol methyl ether acetate, or any combination thereof. In some embodiments, the first organic solvent is dipropylene glycol methyl ether and/or diethylene glycol ethyl ether.

In some embodiments, the first organic solvent is an alcohol, such as a monohydric alcohol, a polyhydric alcohol, an unsaturated aliphatic alcohol, or an alicyclic alcohol. In some embodiments, the alcohol is any of tert-amyl alcohol, benzyl alcohol, 1,4-butanediol, 1,2,4-butanetriol, butanol, 1-butanol, 2-butanol, tert-butyl alcohol, denatured alcohol, di(propylene glycol) methyl ether, diethylene glycol, ethanol, ethylene glycol, 2-ethylhexanol, furfuryl alcohol, glycerol, isobutanol, isopropyl alcohol (isopropanol), methanol, 2-(2-methoxyethoxy)ethanol, 2-methyl-1-butanol, 2-methyl-1-pentanol, 3-methyl-2-butanol, neopentyl alcohol, 2-pentanol, 1,3-propanediol, propan-1-ol, propylene glycol, propylene glycol methyl ether, or any combination thereof. In some embodiments, the alcohol is ethanol and/or isopropanol (isopropyl alcohol). In cases where the first organic solvent is an alcohol, dehydration of the particles may comprise suspending the particles in the alcohol in gradually increasing concentrations of the alcohol. For example, in some embodiments, the particles are dehydrated by (i) separating the particles stored in an aqueous solution from said aqueous solution, and (ii) suspending the separated particles in a series of volumes of a mixture comprising the alcohol, wherein each volume in the series has increasing amounts of the alcohol, e.g., ranging from about 20% to about 100%, about 30% to about 100%, about 40% to about 100%, about 50% to about 100%, about 60% to about 100%, about 70% to about 100%, or about 80% to about 100%, including any value within each of the recited ranges. For example, a first volume of the series of volumes comprises an alcohol concentration of between about 20% and about 80%, including any value within the recited range, and a final volume of the series of volumes may comprise an alcohol concentration of about 99% or 100%. In another example, the first volume of the series of volumes comprises an alcohol concentration of about 50% and the final volume of the series of volumes comprises an alcohol concentration of about 99% or 100%. In some embodiments, the series of volumes includes between about 2 and about 15 volumes, between about 2 and about 10 volumes, or between about 5 and about 7 volumes, including any value within each of the recited ranges. As an example, in a case wherein the series of volumes includes 5 volumes, the first volume may include an alcohol concentration of about 52%, the second volume may include an alcohol concentration of about 64%, the third volume may include an alcohol concentration of about 76%, the fourth volume may include an alcohol concentration of about 88%, and the fifth volume may include an alcohol concentration of about 99% or 100%. In a case wherein the series of volumes includes 6 volumes, the first volume may include an alcohol concentration of about 50%, the second volume may include an alcohol concentration of about 60%, the third volume may include an alcohol concentration of about 70%, the fourth volume may include an alcohol concentration of about 80%, the fifth volume may include an alcohol concentration of about 90%, and the sixth volume may include an alcohol concentration of about 99% or 100%. In a case wherein the series of volumes includes 7 volumes, the first volume may include an alcohol concentration of about 50%, the second volume may include an alcohol concentration of about 55%, the third volume may include an alcohol concentration of about 60%, the fourth volume may include an alcohol concentration of about 65%, the fifth volume may include an alcohol concentration of about 70%, the sixth volume may include an alcohol concentration of about 85%, and the seventh volume may include an alcohol concentration of about 99% or 100%.

In some embodiments, the first organic solvent is a mixture of two or more solvents, such as any of the solvents described herein and/or any suitable solvent known in the art. In some embodiments, the first organic solvent comprises a mixture of a polar aprotic solvent and an alcohol, for example a mixture of DMSO and ethanol, or DMSO and isopropanol. In some embodiments, the first organic solvent comprises a mixture of two or more of the same type of solvent, for example two or more aprotic solvents, such as DMSO and THF. In some embodiments, the solvents in a mixture of solvents are in a ratio of about any of 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, or 1:3. In some embodiments, solvents in a mixture of solvents are in a ratio of about any of 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, or in any other suitable ratio.

In some embodiments, dehydrated or lyophilized particles are directly suspended in a formulation comprising one or more, or all of: a mobile phase (such as any of the mobile phases provided herein), a viscosity enhancing agent (such as any of the viscosity enhancing agents provided herein), and a solubilizing agent (such as any of the solubilizing agents provided herein). In other embodiments, dehydrated or lyophilized particles are first suspended in a solution comprising a second organic solvent and/or suspending agent, prior to suspending the particles in said formulation. In such cases, the methods may comprise (i) separating the plurality of particles from a volume of the first organic solvent, and (ii) suspending the separated plurality of particles in a first volume of a solution comprising the second organic solvent and/or the suspending agent. In some embodiments, one or more additional washes with said solution are performed, for example, by (i) separating the plurality of particles from said solution, and (ii) resuspending the separated plurality of particles in a further volume of said solution. In some embodiments, at least 1, at least 2, at least, 3, at least 4, at least 5, at least 6, at least 7, at least 8, at least 9, at least 10, or more, washes with said solution are performed. In some cases, one or two washes with said solution are performed. In some embodiments, the particles are then separated from said solution, and are subsequently suspended in said formulation comprising one or more, or all of: a mobile phase (such as any of the mobile phases provided herein), a viscosity enhancing agent (such as any of the viscosity enhancing agents provided herein), and a solubilizing agent (such as any of the solubilizing agents provided herein). In some embodiments, the particles are suspended in a formulation comprising: a mobile phase (such as any of the mobile phases provided herein) and a solubilizing agent (such as any of the solubilizing agents provided herein). In some embodiments, the particles are suspended in a formulation comprising: a mobile phase (such as any of the mobile phases provided herein), and viscosity enhancing agent (such as any of the viscosity enhancing agents provided herein). In some embodiments, the particles are suspended in a formulation comprising: a viscosity enhancing agent (such as any of the viscosity enhancing agents provided herein), and a solubilizing agent (such as any of the solubilizing agents provided herein). In some embodiments, the particles are suspended in a formulation comprising: a mobile phase (such as any of the mobile phases provided herein), a viscosity enhancing agent (such as any of the viscosity enhancing agents provided herein), and a solubilizing agent (such as any of the solubilizing agents provided herein). In some embodiments, the second organic solvent and the suspending agent in said solution are in a ratio of about any of 1:1, 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, or 1:3, In some embodiments, the second organic solvent and the suspending agent in said solution are in a ratio of about any of 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, or in any other suitable ratio. In some embodiments, the second organic solvent and the suspending agent in said solution are in a ratio of about 1:1.

Exemplary suspending agents that may be used in said solution include, without limitation, a sugar alcohol, a glycol, a water-miscible polymer, an aprotic solvent, as well as any combination thereof. In some embodiments, the suspending agent comprises a sugar alcohol. Any suitable sugar alcohol known in the art may be used. Exemplary sugar alcohols that may be used include, without limitation, glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, as well as any combination thereof. In some embodiments, the sugar alcohol comprises glycerol. In some embodiments, the suspending agent comprises a glycol. Any suitable glycol known in the art may be used. Exemplary glycols that may be used include, without limitation, diethylene glycol, ethylene glycol, hexylene glycol, propylene glycol, dipropylene glycol, triethylene glycol, tripropylene glycol, a glycol ether, as well as any combination thereof. In some embodiments, the suspending agent in a composition of the disclosure includes propylene glycol. In some embodiments, the suspending agent comprises a water-miscible polymer. Any suitable water-miscible polymer known in the art may be used. Exemplary water-miscible polymers that may be used include, without limitation, an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, polyvinyl alcohol (PVA), a polyurethane polymer, as well as any combination thereof. In some embodiments, the water-miscible polymer comprises a polyethylene glycol (PEG) polymer. In some embodiments, the water-miscible polymer has an average molecular weight of about 1000 g/mol or less, about 900 g/mol or less, about 800 g/mol or less, about 700 g/mol or less, about 600 g/mol or less, about 500 g/mol or less, about 400 g/mol or less, about 300 g/mol or less, about 200 g/mol or less, or about 100 g/mol or less. In some embodiments, the water-miscible polymer has an average molecular weight of about 300 g/mol or less, about 200 g/mol or less, or about 100 g/mol or less. In some embodiments, the water-miscible polymer has an average molecular weight of between about 100 g/mol and about 2000 g/mol, between about 100 g/mol and about 1000 g/mol, between about 200 g/mol and about 600 g/mol, between about 200 g/mol and about 400 g/mol, or between about 200 g/mol and about 300 g/mol. In some embodiments, the water-miscible polymer has an average molecular weight of about 100 g/mol, about 200 g/mol, about 300 g/mol, about 400 g/mol, about 500 g/mol, about 600 g/mol, about 700 g/mol, about 800 g/mol, about 900 g/mol, or about 1000 g/mol. In some embodiments, the water-miscible polymer has an average molecular weight of about 200 g/mol, or about 300 g/mol. In some embodiments, the water-miscible polymer is a PEG polymer, such as PEG 200, PEG 300, PEG 400, PEG 600, or PEG 1000. In some embodiments, the water-miscible polymer is PEG 200 and/or PEG 300. In some embodiments, the suspending agent comprises an aprotic solvent. In some embodiments, the aprotic solvent is a polar aprotic solvent. In some embodiments, the aprotic solvent is a dipolar aprotic solvent. Any suitable aprotic solvent known in the art may be used. Exemplary aprotic solvents that may be used include, without limitation, dimethylsulfoxide (DMSO), dimethylsulfone, diphenylsulfone (DPS), diethylsulfoxide, diethylsulfone, diisopropylsulfone, sulfolane, tetrahydrothiophene-1 -monoxide, THF, N,N-dimethylacetamide (DMAc), N,N-dimethylformamide (DMF), N-methyl-2-pyrrolidinone (NMP), as well as any combination thereof. In some embodiments, the aprotic solvent comprises sulfolane.

In some embodiments, the second organic solvent is an organic solvent that is miscible with water and/or is soluble in water. In some cases, the second organic solvent comprises an aprotic solvent, such as a polar aprotic solvent. In some embodiments, the polar aprotic solvent is a dipolar aprotic solvent. Any suitable aprotic solvent known in the art may be used. Exemplary aprotic solvents that may be used include, without limitation, dimethylsulfoxide (DMSO), dimethylsulfone, tetrahydrofuran (THF), acetonitrile, diphenylsulfone (DPS), diethylsulfoxide, diethylsulfone, diisopropylsulfone, propylene carbonate, sulfolane, tetrahydrothiophene-1-monoxide, N,N-dimethylacetamide (DMAc), N,N-dimethylformamide (DMF), N-methyl-2-pyrrolidinone (NMP), as well as any combination thereof. In some embodiments, the aprotic solvent comprises sulfolane. In some embodiments, the aprotic solvent comprises DMSO. In some embodiments, the second organic solvent is a water miscible P-type or E-type glycol ether, such as any of ethylene glycol monomethyl ether, ethylene glycol monoethyl ether, ethylene glycol monopropyl ether, ethylene glycol monoisopropyl ether, ethylene glycol monobutyl ether, ethylene glycol monophenyl ether, ethylene glycol monobenzyl ether, propylene glycol methyl ether, diethylene glycol monomethyl ether, diethylene glycol ethyl ether, diethylene glycol monoethyl ether, diethylene glycol mono-n-butyl ether, dipropyleneglycol methyl ether, ethylene glycol dimethyl ether, ethylene glycol diethyl ether, ethylene glycol dibutyl ether, ethylene glycol methyl ether acetate, ethylene glycol monoethyl ether acetate, ethylene glycol monobutyl ether acetate, propylene glycol methyl ether acetate, or any combination thereof. In some embodiments, the second organic solvent is dipropylene glycol methyl ether and/or diethylene glycol ethyl ether. In some embodiments, the second organic solvent is an alcohol, such as a monohydric alcohol, a polyhydric alcohol, an unsaturated aliphatic alcohol, or an alicyclic alcohol. In some embodiments, the alcohol is any of tert-amyl alcohol, benzyl alcohol, 1,4-butanediol, 1,2,4-butanetriol, butanol, 1-butanol, 2-butanol, tert-butyl alcohol, denatured alcohol, di(propylene glycol) methyl ether, diethylene glycol, ethanol, ethylene glycol, 2-ethylhexanol, furfuryl alcohol, glycerol, isobutanol, isopropyl alcohol, methanol, 2-(2-methoxyethoxy)ethanol, 2-methyl-1-butanol, 2-methyl-1-pentanol, 3-methyl-2-butanol, neopentyl alcohol, 2-pentanol, 1,3-propanediol, propan-1-ol, propylene glycol, propylene glycol methyl ether, or any combination thereof. In some embodiments, the alcohol is ethanol and/or isopropanol (isopropyl alcohol). In some embodiments, the second organic solvent is a mixture of two or more solvents, such as any of the solvents described herein and/or any suitable solvent known in the art. In some embodiments, the second organic solvent comprises a mixture of a polar aprotic solvent and an alcohol, for example a mixture of DMSO and ethanol, or DMSO and isopropanol. In some embodiments, the second organic solvent comprises a mixture of two or more of the same type of solvent, for example two or more aprotic solvents, such as DMSO and THF. In some embodiments, the solvents in a mixture of solvents are in a ratio of about any of 1:1.1, 1:1.2, 1:1.3, 1:1.4, 1:1.5, 1:1.6, 1:1.7, 1:1.8, 1:1.9, 1:2, 1:2.1, 1:2.2, 1:2.3, 1:2.4, 1:2.5, 1:2.6, 1:2.7, 1:2.8, 1:2.9, or 1:3. In some embodiments, solvents in a mixture of solvents are in a ratio of about any of 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, or in any other suitable ratio. In some embodiments, the second organic solvent is the same as the first organic solvent. For example, both the first and second organic solvents may be DMSO. In other embodiments, the second organic solvent is different from the first organic solvent.

In certain aspects, also provided herein is a composition comprising a plurality of particles produced by any of the methods for preparing compositions provided herein.

**IV. Uses**

Further aspects of the present disclosure relate to uses of any of the compositions described herein, for example, in methods for isolating analytes from a sample, methods for lysing cells, methods for masticating or homogenizing tissues or other sample types, and other uses that will be apparent to those of skill in the art.

In one example, a composition of the disclosure may be used in methods for isolating one or more analytes from a sample. In such cases, the method may comprise providing a sample comprising the analyte(s) to be isolated, and mixing the sample, or a portion thereof, with a composition of the disclosure, thereby binding the analyte(s) to be isolated from the sample to the particles in the composition. In some embodiments, the methods further comprise separating the particles from the mixture of the sample, or the portion thereof, and the composition. For example, in cases where the particles are magnetic or paramagnetic, the particles may be separated using a magnetic field. Separation of the particles may also be accomplished by allowing the particles to settle or using a centrifuge. In some cases, the methods further comprise moving the separated particles to a different container (e.g., a fresh tube), or discarding supernatant separated from the particles. In some cases, the methods may include one or more steps of washing the separated particles (e.g., with wash solution or buffer). In some embodiments, the methods further comprise eluting the bound analyte(s) from the particles, for example, using an elution buffer, by heating, by changing the pH, by changing the salt concentration, or any other suitable elution methods known in the art. In some cases, the methods further comprise recovering the eluted analyte(s), e.g., by moving the eluted analyte(s) to a different container (e.g., a fresh tube). The analyte(s) to be isolated may be, for example and without limitation, nucleic acids, cells, viral particles, polypeptides, lipids, carbohydrates, or small molecules.

The particles used in methods for isolating analytes may be any suitable particles known in the art or described herein. In some embodiments, the particles may be beads, microparticles, microspheres, microbeads, nanobeads, and the like. The particles may also be magnetic or paramagnetic. In some embodiments, the particles comprise one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. In some cases, the particles may be functionalized. Any suitable functional groups or moieties may be appended, attached, bound or otherwise associated with the particles, including, without limitation, a carboxyl, amino, hydroxyl, silica, streptavidin, enzyme, amine, thiol, diol, polymer, an antibody or antibody fragment, a nucleic acid, an oligonucleotide, a peptide, a polypeptide, or any combination thereof. In some embodiments, the analyte is DNA, cDNA, and/or RNA. In such cases, the particles may be capable of or be configured to bind nucleic acids, such as DNA, cDNA, and/or RNA. For example, the particles may be silica or silica-coated particles, or particles associated with one or more oligonucleotides.

In another example, a composition of the disclosure may be used for lysing one or more cells from a sample. In such cases, the method may comprise providing a sample comprising one or more cells, and mixing the sample, or a portion thereof, with a composition of the disclosure. In some embodiments, the methods further comprise agitating the mixture of the sample, or the portion thereof, and the composition of the disclosure to lyse the one or more cells. Agitation may be accomplished manually, using a vortex instrument, stirring (e.g., using a magnetic stir bar), using a bead beater instrument, sonication, or other suitable methods. In some embodiments, the methods further comprise separating the particles from the mixture of the sample, or the portion thereof, and the composition. For example, in the case that the particles are magnetic or paramagnetic, the particles may be separated using a magnetic field. Separation of the particles may also be accomplished by allowing the particles to settle or using a centrifuge. In some cases, the methods further comprise moving the supernatant separated from the particles to a separate container (e.g., a fresh tube), and/or discarding the separated beads. The cells to be lysed may be prokaryotic cells, eukaryotic cells, or both. Examples of cells that may be lysed according to the methods provided herein include, without limitation, one or more animal cells, plant cells, mammalian cells, bacterial cells, archaeal cells, fungal cells, protozoan cells, algal cells, or any combination thereof. The particles used in the cell lysis methods of the disclosure may be any type of particle suitable for such a use known in the art or described herein, for example and without limitation, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. The particles may also be magnetic or paramagnetic.

In another example, a composition of the disclosure may be used for homogenization or mastication of a tissue sample or another type of sample. In such cases, the method may comprise providing a sample, such as a tissue sample, and mixing the sample, or a portion thereof, with a composition of the disclosure. In some embodiments, the methods further comprise agitating the mixture of the sample, or the portion thereof, and the composition of the disclosure to masticate and/or homogenize the sample. Agitation may be accomplished manually, using a vortex instrument, stirring (e.g., using a magnetic stir bar), using a bead beater instrument, sonication, or other suitable methods. In some embodiments, the methods further comprise separating the particles from the mixture of the sample, or the portion thereof, and the composition. For example, in the case that the particles are magnetic or paramagnetic, the particles may be separated using a magnetic field. Separation of the particles may also be accomplished by allowing the particles to settle or using a centrifuge. In some cases, the methods further comprise moving the supernatant separated from the particles to a separate container (e.g., a fresh tube), and/or discarding the separated beads. The particles used in the homogenization or mastication methods of the disclosure may be any type of particle suitable for such a use known in the art or described herein, for example and without limitation, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof. The particles may also be magnetic or paramagnetic.

A variety of materials can be the source of, or serve as, samples for use in any of the methods of the disclosure. For example, the sample can be, or be derived from: solid tissue such as from a fresh, frozen and/or preserved organ, tissue sample, biopsy, resection, smear, or aspirate; scrapings; bone marrow or bone marrow specimens; primary or cultured cells or cell lines; a tissue extract; homogenized tissue; tissue culture medium; tumor lysates; a cell lysate or extract; a bone marrow aspirate; blood or any blood constituents such as whole blood, plasma or serum; blood cells; bodily fluids such as cerebrospinal fluid, vitreous fluid, amniotic fluid, lymph fluid, synovial fluid, follicular fluid, seminal fluid, urine, saliva, sputum, milk, tears, perspiration, mucus, peritoneal fluid or interstitial fluid; pleural fluid; ascites; tissue or fine needle biopsy samples; platelets; surgical specimens; cell-containing body fluids; free-floating nucleic acids; feces; lymph; gynecological fluids; skin swabs; vaginal swabs; cervical swabs; oral or buccal swabs; nasal swabs; ear swabs; eye swabs; penile swabs; rectal swabs; throat swabs; urethral swabs; wound swabs; washings or lavages such as ductal lavages or bronchoalveolar lavages; cells from any time in gestation or development of an individual; cells from a cancer or tumor; other body fluids, secretions, and/or excretions, and/or cells therefrom. In some embodiments, a sample is or comprises cells obtained from an individual. In some embodiments, the sample is or is derived from blood or blood constituents, e.g., obtained from a liquid biopsy. In some embodiments, the sample is or is derived from a tumor sample. In some embodiments, the sample is or comprises biological tissue or fluid. In some embodiments, the sample can contain compounds that are not naturally intermixed with the source of the sample in nature, such as preservatives, anticoagulants, buffers, fixatives, nutrients, antibiotics or the like. In some embodiments, the sample is preserved as a frozen sample or as a formaldehyde- or paraformaldehyde- fixed paraffin-embedded (FFPE) tissue preparation. In some embodiments, the sample comprises circulating tumor cells (CTCs). In one embodiment, the sample is or is acquired from a liquid biopsy of blood, plasma, cerebrospinal fluid, sputum, stool, urine, or saliva. In some embodiments, the sample includes cell-free DNA (cfDNA) and/or circulating tumor DNA (ctDNA), e.g., from a biopsy of blood, plasma, cerebrospinal fluid, sputum, stool, urine, or saliva. In another embodiment, the sample includes one or more circulating tumor cells (CTCs) e.g., a CTC acquired from a blood sample. In some embodiments, a sample is a primary sample obtained directly from a source of interest by any appropriate means. For example, in some embodiments, a primary biological sample is obtained by a method chosen from biopsy (e.g., fine needle aspiration or tissue biopsy), surgery, a swab, or collection of body fluid (e.g., blood, lymph, or feces). In some embodiments, as will be clear from context, a sample may be a preparation that is obtained by processing (e.g., by removing one or more components of and/or by adding one or more agents to) a primary sample. Such a processed sample may comprise, for example, nucleic acids or proteins extracted from a sample or obtained by subjecting a primary sample to techniques such as amplification methods, reverse transcription of mRNA, or isolation and/or purification of certain components such as nucleic acids and/or proteins. In some embodiments, the sample comprises nucleic acids, e.g., genomic DNA, cDNA, or mRNA. In some embodiments, the sample comprises cell-free DNA (cfDNA). In some embodiments, the sample comprises cell-free RNA (cfRNA). In some embodiments, the sample comprises circulating tumor DNA (ctDNA). In certain embodiments, the nucleic acids are purified or isolated (e.g., removed from their natural state). In some embodiments, the sample is a control sample or a reference sample.

Any of the methods of the present disclosure may be implemented on automated or robotic systems, such as automated or robotic liquid handling systems. The methods of the present disclosure may also be implemented in microfluidics systems, such as using microfluidic chips or cartridges. Accordingly, in some aspects, also provided herein are microfluidic devices, such as chips or cartridges, that contain or are configured to receive a composition of the present disclosure.

**V. Kits and articles of manufacture**

Further provided herein are kits or articles of manufacture comprising one or more of any of the compositions of the present disclosure. In some embodiments, the kit or article of manufacture includes a microfluidic device, such as chip or cartridge, that contains or is configured to receive a composition of the present disclosure. In some embodiments, a composition in a kit or article of manufacture may be disposed in a suitable container. Suitable containers include, for example, bottles, vials, tubes (e.g., screw top tubes, microcentrifuge tubes, test tubes, conical tubes, and the like), ampules, bags, cartridges (e.g., microfluidic cartridges, for example within a chamber of the cartridge), chips (e.g., microfluidic chips, for example, within a chamber in the chip), syringes, jars, flexible packaging (e.g., sealed Mylar or plastic bags), and the like. The container may be formed from or include a variety of materials such as glass or plastic (e.g., polypropylene, polyethylene, polypropylene copolymer, polymethylpentene, polyvinyl chloride, polyethylene terephthalate G copolymer, polycarbonate, polysulfone, polystyrene, or teflon).

Kits or articles of manufacture of the disclosure may include one or more additional components (e.g., disposed in one or more additional containers). The additional components may include, for example, a diluent or buffer, e.g., water, phosphate-buffered saline, Ringer's solution, and/or dextrose solution. The article of manufacture or kits of the disclosure may further include other materials desirable from a commercial and user standpoint, including other buffers, diluents, filters, needles, magnets, and syringes. The additional components may be selected based on the intended use of the kit or article of manufacture, or the composition contained therein. For example, for cell lysis applications, the kit or article of manufacture may further include buffers (e.g., lysis buffers), surfactants, protease inhibitors, DNase inhibitors, RNase inhibitors, salts, metal ions, sugars, glycerol, metal chelators, reducing agents, and the like, e.g., each disposed in one or more additional containers. In another example, for analyte isolation applications, the kit or article of manufacture may further include buffers (e.g., lysis buffers, binding buffers, wash buffers, elution buffers), sample or specimen preservatives (e.g., Copan eNAT substrate), surfactants, protease inhibitors, DNase inhibitors, RNase inhibitors, salts, metal ions, sugars, glycerol, metal chelators, reducing agents, and reaction mixes or reagents (e.g., PCR reaction mixes or reagents, for example, comprising buffers, salts, oligonucleotides, polymerases, reverse transcriptase, probes or dyes, and the like).

In some embodiments, the kits or articles of manufacture further include information or instructions for use of the kits or articles of manufacture, e.g., according to any of the uses described herein, for example, for isolating one or more analytes from a sample, for masticating or homogenizing tissue or a sample, or for lysing one or more cells. The information or instructions may be in the form of a package insert or label. The information or instructions may take any form, such as paper or on electronic media such as a magnetically recorded medium, a CD-ROM, a Universal Serial Bus (USB) flash drive, a web site, software or application (e.g., a smart phone application) with said information or instructions, and the like.

The specification is considered to be sufficient to enable one skilled in the art to practice the invention. Various modifications of the invention in addition to those shown and described herein will become apparent to those skilled in the art from the foregoing description and fall within the scope of the appended claims. All publications, patents, and patent applications cited herein are hereby incorporated by reference in their entirety for all purposes. To the extent that any reference incorporated by reference conflicts with the instant disclosure, the instant disclosure shall control.

This disclosure provides the following exemplary and non-limiting embodiments:

1. A composition comprising a plurality of particles and

    (a) a mobile phase comprising a suspending agent;
    (b) a viscosity enhancing agent; and
    (c) a solubilizing agent.

2. The composition of embodiment 1, wherein the mobile phase is at a concentration of between about 60% and about 99%, between about 65% and about 95%, between about 70% and about 90%, between about 75% and about 90%, or between about 80% and about 90% weight by weight (w/w).

3. The composition of embodiment 1 or embodiment 2, wherein the mobile phase is at a concentration of about 70%, about 80%, or about 90% w/w.

4. The composition of any one of embodiments 1-3, wherein the mobile phase is at a concentration of about 80% w/w.

5. The composition of any one of embodiments 1-4, wherein the suspending agent is selected from the group consisting of a sugar alcohol, a glycol, a water-miscible polymer, an aprotic solvent, and any combination thereof.

6. The composition of embodiment 5, wherein the sugar alcohol is selected from the group consisting of glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, and any combination thereof.

7. The composition of embodiment 6, wherein the sugar alcohol is glycerol.

8. The composition of embodiment 7, wherein the glycerol is at a concentration of about 80% w/w in the composition.

9. The composition of any one of embodiments 5-8, wherein the glycol is selected from the group consisting of diethylene glycol, ethylene glycol, hexylene glycol, propylene glycol, dipropylene glycol, triethylene glycol, tripropylene glycol, a glycol ether, and any combination thereof.

10. The composition of embodiment 9, wherein the glycol is propylene glycol.

11. The composition of any one of embodiments 5-10, wherein the water-miscible polymer is selected from the group consisting of an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, and any combination thereof.

12. The composition of embodiment 11, wherein the water-miscible polymer is a polyethylene glycol (PEG) polymer.

13. The composition of any one of embodiments 5-12, wherein the water-miscible polymer comprises an average molecular weight of about 1000 g/mol or less, about 900 g/mol or less, about 800 g/mol or less, about 700 g/mol or less, about 600 g/mol or less, about 500 g/mol or less, about 400 g/mol or less, about 300 g/mol or less, about 200 g/mol or less, or about 100 g/mol or less.

14. The composition of any one of embodiments 5-12, wherein the water-miscible polymer comprises an average molecular weight of between about 100 g/mol and about 1000 g/mol, between about 200 g/mol and about 600 g/mol, between about 200 g/mol and about 400 g/mol, or between about 200 g/mol and about 300 g/mol.

15. The composition of any one of embodiments 5-12, wherein the water-miscible polymer comprises an average molecular weight of about 200 g/mol or about 300 g/mol.

16. The composition of any one of embodiments 5-15, wherein the water-miscible polymer is PEG 200 and/or PEG 300.

17. The composition of any one of embodiments 5-16, wherein the aprotic solvent is a polar aprotic solvent, optionally a dipolar aprotic solvent.

18. The composition of embodiment 17, wherein the aprotic solvent is selected from the group consisting of dimethylsulfoxide (DMSO), dimethylsulfone, diphenylsulfone (DPS), diethylsulfoxide, diethylsulfone, diisopropylsulfone, sulfolane, tetrahydrothiophene-1-monoxide, N,N-dimethylacetamide (DMAc), N,N-dimethylformamide (DMF), N-methyl-2-pyrrolidinone (NMP), and any combination thereof.

19. The composition of embodiment 18, wherein the aprotic solvent is sulfolane.

20. The composition of any one of embodiments 1-19, wherein the viscosity enhancing agent is at least partially soluble in the mobile phase.

21. The composition of any one of embodiments 1-20, wherein the viscosity enhancing agent is at a concentration of between about 1% and about 40%, between about 1% and about 35%, between about 1% and about 30%, between about 1% and about 25%, between about 1% and about 20%, between about 1% and about 15%, between about 1% and about 10%, or between about 1% and about 5% w/w.

22. The composition of any one of embodiments 1-20, wherein the viscosity enhancing agent is at a concentration of about 5% w/w.

23. The composition of any one of embodiments 1-22, wherein the viscosity enhancing agent comprises a water-miscible polymer.

24. The composition of embodiment 23, wherein the water-miscible polymer comprises an average molecular weight of at least about 2000 g/mol, at least about 4000 g/mol, at least about 5000 g/mol, at least about 6000 g/mol, at least about 7000 g/mol, at least about 8000 g/mol, at least about 9000 g/mol, at least about 10000 g/mol, at least about 12000 g/mol, at least about 14000 g/mol, at least about 16000 g/mol, at least about 18000 g/mol, at least about 20000 g/mol, at least about 25000 g/mol, or at least about 30000 g/mol.

25. The composition of embodiment 23, wherein the water-miscible polymer comprises an average molecular weight of between about 2000 g/mol and about 30000 g/mol, between about 4000 g/mol and about 20000 g/mol, between about 4000 g/mol and about 10000 g/mol, or between about 4000 g/mol and about 8000 g/mol.

26. The composition of embodiment 23, wherein the water-miscible polymer comprises an average molecular weight of about 4000 g/mol, about 8000 g/mol, about 10000 g/mol, or about 20000 g/mol.

27. The composition of any one of embodiments 23-26, wherein said water-miscible polymer is selected from the group consisting of an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, and any combination thereof.

28. The composition of embodiment 27, wherein the water-miscible polymer comprises a polyethylene glycol (PEG) polymer.

29. The composition of embodiment 28, wherein the PEG polymer is selected from the group consisting of PEG 4000, PEG 8000, PEG 10000, PEG 20000, and any combination thereof.

30. The composition of embodiment 29, wherein the PEG polymer is PEG 8000.

31. The composition of embodiment 30, wherein the PEG 8000 is at a concentration of about 5% w/w in the composition.

32. The composition of any one of embodiments 1-22, wherein the viscosity enhancing agent comprises polyvinyl alcohol (PVA).

33. The composition of any one of embodiments 1-22, wherein the viscosity enhancing agent comprises sulfone or a sulfone polymer.

34. The composition of any one of embodiments 1-33, wherein the solubilizing agent is at least partially soluble in the mobile phase.

35. The composition of any one of embodiments 1-34, wherein the solubilizing agent is water-soluble.

36. The composition of any one of embodiments 1-35, wherein the solubilizing agent is at a concentration of between about 1% and about 40%, between about 5% and about 30%, between about 10% and about 25%, or between about 10% and about 20% w/w.

37. The composition of any one of embodiments 1-36, wherein the solubilizing agent is at a concentration of about 15% w/w.

38. The composition of any one of embodiments 1-37, wherein the solubilizing agent comprises a chaotropic agent.

39. The composition of embodiment 38, wherein the chaotropic agent is selected from the group consisting of n-butanol, ethanol, a guanidinium salt, lithium perchlorate, lithium acetate, magnesium chloride, phenol, 2-propanol, sodium dodecyl sulfate, thiourea, urea, a water-soluble urea derivative, and any combination thereof.

40. The composition of embodiment 39, wherein the chaotropic agent is a guanidinium salt.

41. The composition of embodiment 40, wherein the guanidium salt is selected from the group consisting of guanidinium chloride, guanidinium sulfate, guanidine carbonate, guanidinium nitrate, guanidinium isothiocyanate, guanidinium thiocyanate, and any combination thereof.

42. The composition of embodiment 39, wherein the chaotropic agent is urea and/or a water-soluble urea derivative.

43. The composition of embodiment 42, wherein the urea and/or the water-soluble urea derivative is at a concentration of about 15% w/w in the composition.

44. The composition of any one of embodiments 42-43, wherein the water-soluble urea derivative is thiourea, hydroxyurea, dimethylurea, or any combination thereof.

45. The composition of any one of embodiments 1-44, wherein the solubilizing agent comprises a buffering agent.

46. The composition of embodiment 45, wherein the buffering agent is selected from the group consisting of a MES, Bis-Tris, ADA, ACES, PIPES, MOPSO, Bis-Tris Propane, BES, MOPS, TES, HEPES, DIPSO, MOBS, TAPSO, Tris, HEPPSO, POPSO, TEA, EPPS, HEPPS, Tricine, Gly-Gly, Bicine, HEPBS, TAPS, AMPD, TABS, AMPSO, CHES, CAPSO, AMP, CAPS, CABS, phosphate, maleate, glycine, citrate, formate, succinate, acetate, propionate, pyridine, piperazine, cacodylate, histidine, ethanolamine, carbonate, imidazole, pyrophosphate, hydrazine, taurine (AES), borate, ammonium hydroxide, methylamine, piperidine, malate buffer, and any combination thereof.

47. The composition of embodiment 46, wherein the buffering agent comprises a TAPS, HEPES, or MOPS buffer, or any combination thereof.

48. The composition of any one of embodiments 1-8, 20-31, 34-39, and 42-43, wherein the suspending agent comprises glycerol, the viscosity enhancing agent comprises PEG 8000, and the solubilizing agent comprises urea.

49. The composition of embodiment 48, wherein the glycerol is at a concentration of about 80% w/w, the PEG 8000 is at a concentration of about 5% w/w, and the urea is at a concentration of about 15% w/w.

50. The composition of embodiment 48 or embodiment 49, wherein the ratio of glycerol:PEG 8000:urea in the composition is 16:1:3 w/w.

51. The composition of any one of embodiments 1-50, wherein the pH of the composition is between about 7 and about 8.

52. The composition of any one of embodiments 1-51, wherein the composition has a viscosity of:

(a) between about 7000 millipascal-second (mPa·s) and about 8000 mPa·s, between about 7250 mPa·s and about 8000 mPa·s, between about 7500 mPa·s and about 8000 mPa·s, or between about 7750 mPa·s and about 8000 mPa·s, when measured at a temperature of about 15°C;
(b) between about 5000 mPa's and about 6000 mPa·s, between about 5250 mPa·s and about 6000 mPa·s, between about 5500 mPa·s and about 6000 mPa·s, or between about 5500 mPa s and about 5750 mPa·s, when measured at a temperature of about 20°C;
(c) between about 5500 mPa·s and about 7000 mPa·s, between about 5750 mPa·s and about 6750 mPa·s, or between about 6000 mPa·s and about 6500 mPa·s, when measured at a temperature of about 25°C;
(d) between about 4250 mPa·s and about 5500 mPa·s, between about 4500 mPa·s and about 5500 mPa·s, between about 4750 mPa·s and about 5250 mPa·s, or between about 4750 mPa s and about 5000 mPa s, when measured at a temperature of about 30°C;
(e) between about 2000 mPa·s and about 3000 mPa·s, between about 2250 mPa·s and about 2750 mPa·s, or between about 2250 mPa s and about 2500 mPa·s, when measured at a temperature of about 35°C;
(f) between about 750 mPa·s and about 1750 mPa·s, between about 1000 mPa·s and about 1750 mPa·s, or between about 1000 mPa·s and about 1500 mPa·s, when measured at a temperature of about 40°C;
(g) between about 500 mPa·s and about 1500 mPa s, between about 750 mPa s and about 1250 mPa·s, or between about 1000 mPa·s and about 1250 mPa·s, when measured at a temperature of about 45°C;

(h) between about 250 mPa·s and about 1500 mPa·s, between about 500 mPa·s and about 1250 mPa·s, or between about 500 mPa·s and about 1000 mPa·s, when measured at a temperature of about 50°C;

(i) between about 500 mPa·s and about 1500 mPa s, between about 750 mPa s and about 1250 mPa·s, or between about 1000 mPa·s and about 1250 mPa·s, when measured at a temperature of about 55°C; and/or

(j) between about 100 mPa·s and about 600 mPa·s, between about 200 mPa·s and about 500 mPa s, or between about 200 mPa s and about 400 mPa s, when measured at a temperature of about 60°C.

53. The composition of embodiment 52, wherein viscosity of the composition is assessed using a rotational viscometer.

54. The composition of embodiment 53, wherein viscosity of the composition is assessed at a spindle rotational speed of about 10 revolutions per minute (RPM).

55. The composition of any one of embodiments 1-54, wherein the composition has a density of between about 0.5 g/mL and about 2 g/mL, between about 1 g/mL and about 2 g/mL, between about 1 g/mL and about 1.5 g/mL, or between about 1.1 g/mL and about 1.5 g/mL.

56. The composition of any one of embodiments 1-55, wherein the composition has a density of about 1.3 g/mL.

57. The composition of any one of embodiments 55-56, wherein density of the composition is assessed on an analytical balance.

58. The composition of any one of embodiments 1-57, wherein the mobile phase is non-aqueous or substantially non-aqueous.

59. The composition of any one of embodiments 1-58, wherein the composition is non-aqueous or substantially non-aqueous.

60. The composition of any one of embodiments 1-59, wherein the composition has a water content of about 1% or less.

61. The composition of embodiment 60, wherein water content of the composition is assessed by Karl Fischer titration.

62. The composition of any one of embodiments 1-61, wherein the composition is a liquid composition.

63. The composition of any one of embodiments 1-62, wherein the plurality of particles comprises one or more particles configured to bind a nucleic acid, a polypeptide, a lipid, a carbohydrate, or a small molecule.

64. The composition of embodiment 63, wherein the plurality of particles comprises one or more particles configured to bind DNA and/or RNA.

65. The composition of any one of embodiments 1-64, wherein the plurality of particles comprises one or more microparticles, nanoparticles, microspheres, paramagnetic beads, magnetic beads, microbeads, nanobeads, or any combination thereof.

66. The composition of any one of embodiments 1-65, wherein the plurality of particles comprises one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof.

67. The composition of any one of embodiments 1-66, wherein one or more particles of the plurality of particles is functionalized, optionally wherein the one or more particles are surface functionalized with a carboxyl, amino, hydroxyl, silica, streptavidin, endopeptidase enzyme moiety, amine, thiol, diol, polymer, an antibody or antibody fragment, a nucleic acid, an oligonucleotide, a peptide, a polypeptide, or any combination thereof.

68. The composition of embodiment 66 or embodiment 67, wherein the plurality of particles comprises magnetic silica beads.

69. The composition of any one of embodiments 1-68, wherein the plurality of particles is present in the composition at a concentration of at least about 0.5%, at least about 1%, at least about 2.5%, at least about 5%, at least about 10%, at least about 15%, at least about 20%, at least about 25%, or at least about 30% weight by volume (w/v).

70. The composition of any one of embodiments 1-68, wherein the plurality of particles is present in the composition at a concentration of between about 0.5% and about 30% w/v, between about 1% and about 25% w/v, between about 2.5% and about 20% w/v, or between about 5% and about 15% w/v.

71. The composition of any one of embodiments 1-68, wherein the plurality of particles is present in the composition at a concentration of about 10% w/v.

72. The composition of any one of embodiments 1-71, wherein the composition enhances stability of particles in the plurality of particles as compared to corresponding particles in an aqueous solution.

73. The composition of any one of embodiments 1-72, wherein the plurality of particles in the composition is stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months.

74. The composition of embodiment 72 or embodiment 73, wherein particle stability is assessed based on particle integrity, particle aggregation, and/or retention of ability to bind to a ligand.

75. The composition of any one of embodiments 1-74, wherein the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline.

76. The composition of any one of embodiments 1-75, wherein the composition enhances ligand binding of particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution.

77. The composition of any one of embodiments 1-76, wherein the plurality of particles comprises particles capable of binding to nucleic acid ligands.

78. The composition of embodiment 77, wherein stability of the particles is assessed based on nucleic acid ligand binding ability compared to baseline and/or compared to corresponding particles in an aqueous solution.

79. The composition of embodiment 77 or embodiment 78, wherein the nucleic acid ligands comprise DNA and/or RNA.

80. The composition of any one of embodiments 77-79, wherein the plurality of particles comprises silica particles, optionally magnetic silica particles.

81. The composition of any one of embodiments 77-80, wherein the particles in the plurality of particles retain at least about 50%, at least about 55%, at least about 60%, at least about 65%, at least about 70%, at least about 75%, at least about 80%, at least about 85%, at least about 90%, at least about 95%, or at least about 99% of nucleic acid ligand binding ability for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, as compared to baseline.

82. The composition of any one of embodiments 77-81, wherein the composition enhances nucleic acid ligand binding by particles in the plurality of particles by at least about 1%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 100%, as compared to corresponding particles in an aqueous solution.

83. The composition of any one of embodiments 72-82, wherein particle stability is assessed at room temperature.

84. The composition of any one of embodiments 72-83, wherein particle stability is assessed at a temperature of between about 20°C and about 30°C.

85. The composition of embodiment 84, wherein particle stability is assessed at a temperature of about 25°C.

86. The composition of any one of embodiments 72-85, wherein particle stability is assessed under normal atmospheric conditions, in reduced oxygen conditions, or in nitrogen gas.

87. The composition of any one of embodiments 1-86, wherein the composition is capable of adhering to a polypropylene surface.

88. The composition of any one of embodiments 1-87, wherein the composition is microbiologically stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months.

89. The composition of any one of embodiments 1-88, wherein the composition further comprises an agent selected from the group consisting of: an antioxidant, a reducing agent, a chelating agent, and any combination thereof.

90. The composition of any one of embodiments 1-89, wherein the composition does not comprise an anti-microbial agent.

91. The composition of any one of embodiments 1-90, wherein the composition comprises less than about 10% of surfactant or detergent.

92. The composition of any one of embodiments 1-91, wherein the composition does not comprise a surfactant or detergent.

93. A method for preparing a composition comprising a plurality of particles, the method comprising:

(a) providing a plurality of particles; and
(b) suspending the plurality of particles in a formulation comprising:

i. a mobile phase comprising a suspending agent;
ii. a viscosity enhancing agent; and
iii. a solubilizing agent.

94. The method of embodiment 93, wherein the plurality of particles provided in step (a) is dehydrated or lyophilized.

95. The method of embodiment 94, wherein the method further comprises dehydrating or lyophilizing the plurality of particles prior to step (a).

96. The method of embodiment 95, wherein the dehydrating comprises suspending the plurality of particles in a first organic solvent.

97. The method of embodiment 95 or embodiment 96, wherein the dehydrating comprises: (i) separating a plurality of particles stored in an aqueous solution from said aqueous solution, and (ii) suspending the separated plurality of particles in first volume of a first organic solvent.

98. The method of embodiment 97, wherein the dehydrating further comprises: (1) separating the plurality of particles from the first organic solvent, and (2) resuspending the separated plurality of particles in a further volume of said first organic solvent.

99. The method of embodiment 98, comprising repeating steps (1) and (2) at least once, at least twice, or more.

100. The method of embodiment 95 or embodiment 96, wherein the dehydrating comprises: (i) separating a plurality of particles stored in an aqueous solution from said aqueous solution, and (ii) suspending the separated plurality of particles in a series of volumes of a first organic solvent, wherein the volumes in the series of volumes comprise increasing amounts of the first organic solvent, wherein a first volume of the series of volumes comprises an organic solvent concentration of between about 20% and about 80%, and the final volume of the series of volumes comprises an organic solvent concentration of about 100%.

101. The method of embodiment 100, wherein the first volume of the series of volumes comprises an organic solvent concentration of about 50% and the final volume of the series of volumes comprises an organic alcohol concentration of about 100%.

102. The method of embodiment 100 or embodiment 101, wherein the series of volumes comprises between about 2 and about 15 volumes, between about 2 and about 10 volumes, or between about 5 and about 7 volumes.

103. The method of any one of embodiments 96-102, wherein the first organic solvent comprises an organic solvent miscible with water and/or with high solubility in water.

104. The method of any one of embodiments 96-103, wherein the first organic solvent comprises:

a polar aprotic solvent, optionally wherein the polar aprotic solvent is DMSO, tetrahydrofuran (THF), acetonitrile, propylene carbonate, sulfolane, and any combination thereof;
a water miscible P-type or E-type glycol ether, optionally wherein the first organic solvent comprises dipropylene glycol methyl ether and/or diethylene glycol ethyl ether;
an alcohol, optionally wherein the alcohol is ethanol and/or isopropanol;
tetrahydrothiophene 1-oxide;

or any combination thereof.

105. The method of any one of embodiments 96-104, wherein the first organic solvent comprises a mixture of two or more solvents, optionally wherein the solvents are in about a 1:1 ratio, and further optionally wherein said mixture comprises:

DMSO and ethanol;
DMSO and isopropanol; or
DMSO and THF.

106. The method of any one of embodiments 96-105, wherein the first organic solvent comprises DMSO.

107. The method of any one of embodiments 100-106, wherein the first organic solvent comprises an alcohol, optionally wherein the alcohol is ethanol and/or isopropanol.

108. The method of any one of embodiments 96-107, wherein step (b) comprises: (i) separating the plurality of particles from a volume of the first organic solvent, and (ii) suspending the separated plurality of particles in a volume of said formulation.

109. The method of any one of embodiments 93-107, further comprising, prior to step (b), suspending the plurality of particles in a first volume of a solution comprising:

a second organic solvent, and
a suspending agent.

110. The method of any one of embodiments 96-109, wherein the method further comprises, prior to step (b): (i) separating the plurality of particles from a volume of the first organic solvent, and (ii) suspending the separated plurality of particles in a first volume of a solution comprising:

a second organic solvent, and
a suspending agent.

111. The method of embodiment 109 or embodiment 110, further comprising: (1) separating the plurality of particles from said solution, and (2) resuspending the separated plurality of particles in a further volume of said solution.

112. The method of embodiment 111, comprising repeating steps (1) and (2) at least once, at least twice, or more.

113. The method of any one of embodiments 109-112, wherein the second organic solvent and the suspending agent in the solution are in about a 1:1 ratio.

114. The method of any one of embodiments 109-113, wherein the suspending agent is selected from the group consisting of a sugar alcohol, a glycol, a water-miscible polymer, an aprotic solvent, and any combination thereof.

115. The method of embodiment 114, wherein:

the sugar alcohol is selected from the group consisting of glycerol, sorbitol, erythritol, threitol, arabitol, xylitol, ribitol, mannitol, galactitol, fucitol, iditol, inositol, volemitol, isomalt, maltitol, lactitol, maltotriitol, maltotetraitol, polyglycitol, and any combination thereof, optionally wherein the sugar alcohol is glycerol;
the glycol is selected from the group consisting of diethylene glycol, ethylene glycol, hexylene glycol, propylene glycol, dipropylene glycol, triethylene glycol, tripropylene glycol, a glycol ether, and any combination thereof; optionally wherein the glycol is propylene glycol;
the water-miscible polymer is selected from the group consisting of an acrylic polymer, an ether polymer, an epoxy polymer, a polyethylene polymer, a polyethylene glycol polymer, a polystyrene polymer, a polyvinylchloride polymer, a polytetrafluorethylene polymer, a polydimethylsiloxane polymer, a polyester polymer, a poly(N-vinylpyrrolidone) polymer, a polystyrene copolymer, a polyurethane polymer, and any combination thereof, optionally wherein the water-miscible polymer is a PEG 200 and/or PEG 300; and/or
the aprotic solvent is a polar aprotic solvent, optionally wherein the aprotic solvent is sulfolane.

116. The method of embodiment 115, wherein the suspending agent comprises glycerol.

117. The method of any one of embodiments 109-116, wherein the second organic solvent comprises an organic solvent miscible with water and/or with high solubility in water.

118. The method of any one of embodiments 109-117, wherein the second organic solvent comprises:

a polar aprotic solvent, optionally wherein the polar aprotic solvent is DMSO, tetrahydrofuran (THF), acetonitrile, propylene carbonate, sulfolane, and any combination thereof;
a water miscible P-type or E-type glycol ether, optionally wherein the second organic solvent comprises dipropylene glycol methyl ether and/or diethylene glycol ethyl ether;
an alcohol, optionally wherein the alcohol is ethanol and/or isopropanol; or
tetrahydrothiophene 1-oxide.

119. The method of any one of embodiments 109-118, wherein the second organic solvent comprises a mixture of two or more solvents, optionally wherein the solvents are in about a 1:1 ratio, and further optionally wherein said mixture comprises:

DMSO and ethanol;
DMSO and isopropanol; or
DMSO and THF.

120. The method of any one of embodiments 109-119, wherein the second organic solvent comprises DMSO.

121. The method of any one of embodiments 109-120, wherein: (1) the second organic solvent is the same as the first organic solvent; or (2) the second organic solvent is different from the first organic solvent.

122. The method of embodiment 121, wherein the first and second organic solvents comprise DMSO.

123. The method of any one of embodiments 109-122, wherein step (b) comprises: (i) separating the plurality of particles from a volume of said solution comprising the second organic solvent and the suspending agent, and (ii) suspending the separated plurality of particles in a volume of said formulation.

124. A composition comprising a plurality of particles produced by the method of any one of embodiments 93-123.

125. A method for isolating an analyte from a sample, comprising:

> (a) providing a sample comprising an analyte;
> (b) mixing the sample with the composition comprising a plurality of particles of any one of embodiments 1-92 and 124, or a composition comprising a plurality of particles produced by the method of any one of embodiments 93-123, to produce a mixture of the sample and the composition, thereby binding said analyte to one or more particles in the plurality of particles; and
> (c) separating the plurality of particles from the mixture.

126. The method of embodiment 125, further comprising eluting the analyte bound to the one or more particles in the plurality of particles, and optionally separating the plurality of particles from the eluted analyte.

127. The method of embodiment 126, further comprising isolating or recovering the eluted analyte.

128. The method of any one of embodiments 125-127, wherein the analyte is a nucleic acid, a polypeptide, a lipid, a carbohydrate, or a small molecule, optionally wherein the nucleic acid is DNA and/or RNA.

129. The method of any one of embodiments 125-128, wherein the plurality of particles comprises one or more particles configured to bind DNA and/or RNA.

130. The method of any one of embodiments 125-129, wherein the plurality of particles comprises one or more microparticles, nanoparticles, microspheres, paramagnetic beads, magnetic beads, microbeads, nanobeads, or any combination thereof.

131. The method of any one of embodiments 125-130, wherein the plurality of particles comprises one or more silica beads, silica gel beads, controlled pore glass beads, magnetic beads, glass beads, paramagnetic beads, Dynabeads, Sephadex/Sepharose beads, cellulose beads, polystyrene beads, mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof.

132. The method of any one of embodiments 125-131, wherein one or more particles of the plurality of particles is functionalized, optionally wherein the one or more particles are surface functionalized with a carboxyl, amino, hydroxyl, silica, streptavidin, endopeptidase enzyme moiety, amine, thiol, diol, polymer, an antibody or antibody fragment, a nucleic acid, an oligonucleotide, a peptide, a polypeptide, or any combination thereof.

133. The method of embodiment 131 or embodiment 132, wherein the plurality of particles comprises silica beads, optionally magnetic silica beads, and further optionally wherein the analyte is DNA and/or RNA.

134. The method of any one of embodiments 125-132, wherein the sample is or is derived from a tissue, primary or cultured cells or cell lines, a cell supernatant, a cell lysate, platelets, serum, plasma, vitreous fluid, lymph fluid, synovial fluid, follicular fluid, seminal fluid, amniotic fluid, milk, whole blood, blood-derived cells, urine, cerebrospinal fluid, saliva, sputum, tears, perspiration, mucus, tumor lysates, tissue culture medium, a tissue extract, homogenized tissue, tumor tissue, cellular extracts, or a combination thereof.

135. The method of any one of embodiments 125-132, wherein the sample is a liquid biopsy sample, optionally wherein the liquid biopsy sample is a sample of blood or plasma.

136. The method of embodiment 135, wherein the sample is a liquid biopsy sample, and the analyte is cell-free DNA or cell-free RNA.

137. The method of any one of embodiments 125-132, wherein the sample is or is derived from a nasal swab, a buccal swab, a cervical swab, an ear swab, an eye swab, a vaginal swab, a penile swab, a rectal swab, a throat swab, a urethral swab, a vulval swab, a wound swab, or a skin swab.

138. A method for cell lysis, comprising:

> (a) providing a sample comprising a plurality of cells;
> (b) mixing the sample with the composition comprising a plurality of particles of any one of embodiments 1-92 and 124, or a composition comprising a plurality of particles produced by the method of any one of embodiments 93-123, thereby producing a mixture of the sample and the composition; and
> (c) agitating the mixture, thereby lysing one or more cells in the plurality of cells.

139. The method of embodiment 138, further comprising separating the plurality of particles from the mixture.

140. The method of embodiment 138 or embodiment 139, wherein the one or more cells comprise one or more prokaryotic cells and/or one or more eukaryotic cells.

141. The method of any one of embodiments 138-140, wherein the one or more cells comprise one or more animal cells, plant cells, mammalian cells, bacterial cells, archaeal cells, fungal cells, protozoan cells, algal cells, or any combination thereof.

142. The method of any one of embodiments 138-141, wherein the plurality of particles comprises one or more mineral beads, crystalline particles, zirconium particles, zircon particles, zirconium silicate particles, zirconia particles, zirconium dioxide particles, quartz particles, aluminum oxide particles, silicon carbide particles, ceramic particles, glass particles, silicon dioxide glass particles, silica particles, metal particles, steel particles, stainless steel particles, yttrium particles, chrome-steel particles, or any combination thereof.

143. The method of any one of embodiments 125-142, wherein the method is implemented on a microfluidic device and/or an automated or robotic liquid handling system.

144. A kit comprising the composition of any one of embodiments 1-92 and 124, or a composition produced by the method of any one of embodiments 93-123.

145. The kit of embodiment 144, further comprising instructions for use of the composition for isolating one or more analytes from a sample, optionally wherein the instructions are for isolating the one or more analytes from the sample according to the method of any one of embodiments 125-137 and 143.

146. The kit of embodiment 144, further comprising instructions for use of the composition for lysing one or more cells from a sample, optionally wherein the instructions are for lysing the one or more cells according to the method of any one of embodiments 138-143.

147. A microfluidic device comprising the composition of any one of embodiments 1-92 and 124, or a liquid composition produced by the method of any one of embodiments 93-123.

EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

***Example 1: Development of formulations for enhanced particle stability and handling.***

Particles, such as magnetic beads, are widely used in molecular biology for many applications. For example, magnetic silica beads are frequently used for isolating DNA and RNA. Particles are typically stored as dried compositions or as aqueous suspensions. Drying of particles, however, can result in aggregation or degradation of the particles, as well as issues with resuspension. Aqueous suspensions of particles allow for particle settling, which can lead to aggregation and clumping, and also require constant mixing to ensure consistency during use. This Example describes the development of candidate liquid formulations with high viscosity and density to enhance particle stability and handling.

**Materials and Methods**

*Preparation of candidate formulations*

A three-step process was used to prepare candidate liquid formulations of particles. Magnetic silica beads for nucleic acid isolation were used as exemplary particles in the candidate liquid formulations.

As detailed below, in the first step, the silica beads were dehydrated using an organic solvent to remove the aqueous buffer solution in which the beads were received from the manufacturer. Following dehydration of the beads, the amount of organic solvent was reduced, and the viscosity of the bead mixture was increased. Finally, candidate formulations were prepared by resuspending the dehydrated beads in a solution containing a mobile phase, a viscosity enhancer, and an excipient.

<u>(1) Dehydration of the beads</u>

The exemplary magnetic silica beads used in this example were received from the manufacturer in an aqueous suspension buffer. To remove the buffer and prepare the beads for subsequent steps, the beads were first dehydrated using an organic solvent as follows:

1. The mixture of beads in an aqueous suspension buffer was transferred to a square weigh boat.

2. A magnet was placed under the weigh boat for at least 30 seconds to separate the beads from the suspension buffer.

3. The suspension buffer was removed from the beads by pipetting.

4. Organic solvent was added to the beads on the weigh boat and mixed thoroughly at least 6 times using a glass rod, repeating until a homogenous consistency was obtained.

5. Steps 2-4 were repeated at least two more times.

6. The organic solvent was removed by placing a magnet under the weigh boat for at least 30 seconds to separate the beads from the solvent. The solvent was then removed by pipetting, resulting in dehydrated beads ready for the next step in the process.

Several organic solvents were tested for use during the dehydration step, including the solvents listed in **Table 1.**

*Table 1. Candidate organic solvents for formulating beads.*

| Candidate Organic Solvents |
|---|
| Polar aprotic solvents, such as dimethyl sulfoxide (DMSO), tetrahydrofuran (THF), acetonitrile, and propylene carbonate |
| Alcohols, such as ethanol and isopropanol |
| Water-miscible P and E type glycol ethers, such as dipropylene glycol methyl ether and diethylene glycol ethyl ether |
| Tetrahydrothiophene 1-oxide |
| Sulfolane |
| Dichloromethane |
| Chloroform |
| Toluene |
| Isoamyl alcohol |
| 2-phenoxyethanol |
| Acetic Acid |
| Solvents that are not miscible with water or have low water solubility, such as 2-phenoxyethanol |
| Mixtures of solvents, such as a 1:1 mixture of DMSO and ethanol, a 1:1 mixture of DMSO and isopropanol, and 1:1 mixture of THF and DMSO |

(2) Reduction of solvent content and initial increase in viscosity

To reduce the amount of solvent that could evaporate in the final formulation, and to increase viscosity of the bead mixture, the dehydrated beads were washed in a 1:1 mixture of organic solvent and glycerol as follows:

1. A 1: 1 mixture of organic solvent and glycerol was added to the dehydrated beads on a weigh boat and mixed thoroughly at least 6 times using a glass rod, repeating until a homogenous consistency was obtained.

2. A magnet was placed under the weigh boat for at least 30 seconds to separate the beads from the solvent and glycerol mixture.

3. The solvent and glycerol mixture was removed by pipetting.

4. Steps 1-3 were repeated at least once.

Several organic solvents were tested during this step, including the solvents provided in Table **1,** above.

(3) Candidate formulations

Candidate formulations containing various combinations of a mobile phase, a viscosity enhancer, and an excipient were prepared. The agents listed in **Table 2** were tested.

*Table 2. Agents tested for candidate formulations.*

| | |
|---|---|
| **Mobile Phase** | • Glycerol<br>• Propylene glycol<br>• Polyethylene glycol (PEG), including PEG 200 and PEG 300<br>• Sulfolane<br>• Detergents, including Triton X-100, Tween 20, and Brij-type detergents<br>• Easily evaporating organic solvents, such as alcohols<br>• Water |
| **Viscosity enhancer** | • Hydrophilic polymers, such as PEG compounds (e.g., PEG 4000, PEG 8000, PEG 10000, and PEG 20000) |
| **Excipient** | • Urea<br>• Guanidinium salts (e.g., guanidine thiocyanate and guanidine hydrochloride)<br>• Buffers, including TAPS, HEPES, and MOPS buffers<br>• Di- and tri-carboxylic acids such as citric acid and oxalic acid<br>• Amino acids, such as arginine, glycine, tyrosine, tryptophane, phenylalanine, and proline<br>• Carboxylic acid salts, such as sodium citrate, sodium oxalate, sodium/potassium/ammonium propionate, sodium/potassium/ammonium hexanate, and others<br>• Ethylenediaminetetraacetic acid (EDTA) and its sodium salt<br>• Metal oxides, such as zinc oxide and titanium oxide<br>• Sugars, such as trehalose, sucrose, mannitol, raffinose, glucose, and lactose<br>• Dextran<br>• Polyvinylpyrrolidone 40 (PVP-40)<br>• Polyvinyl acetate (PVA)<br>• Heparin<br>• Polyethylenimine (PEI) |

To prepare the candidate formulations, the beads were resuspended in candidate solutions containing a combination of a mobile phase, a viscosity enhancer, and an excipient as follows:

1. The candidate solution was added to the beads using a sterile Pasteur pipette.

2. The solution and beads were mixed thoroughly at least 6 times using a glass rod, repeating until a homogenous consistency was obtained.

*Stability, functionality, and viscosity testing*

The beads were assessed for stability, functionality and viscosity to determine the suitability of the candidate agents tested in each of steps 1-3, above.

Stability: The stability of the beads was determined based on functional performance of the formulated beads.

Functionality: The functionality of the beads was determined based on retention of their ability to bind nucleic acids using fluorometric analysis. Briefly, known amounts of RNA were mixed with beads, and following washes and elution, the amount of eluted RNA was quantified using a fluorometric assay (Quantus, Promega) according to the manufacturer's instructions. Candidate agents were determined to be incompatible if a decrease of 10% or greater in nucleic acid binding efficiency was observed.

Viscosity: The viscosity of the candidate formulations tested in step 3, above, was assessed based on adherence to a polypropylene surface, and ability to pipette the formulations.

**Results**

*Organic solvents*

Testing of the candidate organic solvents in **Table 1** showed that polar aprotic solvents, such as DMSO, THF, acetonitrile, and propylene carbonate, worked well for dehydration of the beads, and did not result in bead degradation, aggregation, or loss of nucleic acid binding ability, or the need for extensive washing. Alcohols, such as ethanol and isopropanol, also worked well, but required a more elaborate or controlled dehydration process (*see* **Table 3,** below, for details). Water miscible P and E type glycol ethers (e.g., dipropylene glycol methyl ether and diethylene glycol ethyl ether), and 1: 1 mixtures of DMSO and ethanol, DMSO and isopropanol, and THF and DMSO were also suitable. A summary of suitable candidate solvents is provided below, in **Table 3.**

*Table 3: Suitable organic solvents.*

| Candidate Solvent | CAS # | Characteristics |
|---|---|---|
| DMSO | 67-68-5 | Non-toxic, non-flammable, non-volatile, relatively inexpensive. |
| Tetrahydrothiophene 1-oxide | 1600-44-8 | Non-toxic but has offensive smell; not suitable for work outside fume hood because of odor. |
| Sulfolane | 126-33-0 | Works well when melted at 30°C; has high toxicity in comparison to DMSO. |
| Tetrahydrofuran (THF) | 109-99-9 | Inexpensive, slightly more toxic than DMSO, has high evaporation rate, flammable, easy separation of the particles using a magnet. |
| Propylene carbonate | 108-32-7 | Not completely miscible with water, slow evaporation, non-flammable |
| Acetonitrile | 75-05-8 | Strong odor, moderate toxicity |
| Ethanol Isopropanol | 64-17-5 67-63-0 | Dehydration required a stepwise process, starting at 50% ethanol or isopropanol, and going up to 99% ethanol or isopropanol (total 5-7 washes), otherwise beads aggregated; more time-consuming dehydration. |
| Dipropylene Glycol Methyl Ether | 34590-94-8 | No strong smell, slow evaporation, easy separation of the particles using a magnet, common industrial solvent. |
| Diethylene Glycol Ethyl Ether | 112-36-7 | No strong smell, easy separation of the particles using a magnet, for wider use requires drying with molecular sieves to achieve sufficient purity of the solvent; common industrial solvent. |

The following organic solvents were found to result in particle degradation, aggregation, or loss of functionality, or did not result in adequate dehydration under the conditions tested:

- Dichloromethane

- Chloroform

- Toluene

- Isoamyl alcohol

- 2-phenoxyethanol

- Acetic acid (resulted in partial degradation of the beads)

Overall, the results described above showed that solvents miscible with water or highly soluble in water (e.g., propylene carbonate) were suitable for dehydration of the beads. Solvents that are not miscible with water or with low water solubility (e.g., 2-phenoxyethanol) were not able to adequately dehydrate the particles, or led to degradation, aggregation, or loss of bead functionality.

*Components in candidate formulations*

Candidate formulations containing various combinations of the mobile phase, viscosity enhancer, and excipient components listed in **Table 2** were tested.

Mobile phase

Suspending agents, such as glycerol, propylene glycol, PEG 200, PEG 300, and sulfolane (above its melting point or mixed with glycerol, propylene glycol, PEG 200, or PEG 300) worked well as the mobile phase. In contrast, detergents, such as Triton X-100, Tween 20, and Brij-type detergents, easily evaporating organic solvents such as alcohols, and water were found to not be suitable for use as the mobile phase due to issues such as foaming, loss of bead functionality, bead aggregation or degradation, or fast evaporation rate.

Viscosity enhancer

Certain PEG compounds were successfully used as the viscosity enhancing component of the formulations. **Table 4** provides a summary of PEG compounds found to be suitable for use as viscosity enhancers.

*Table 4: Summary of tested viscosity enhancers.*

| PEG Compound | Characteristics |
|---|---|
| PEG 4000 | Beads retained functionality and did not aggregate or degrade; viscosity of formulation was relatively low. |
| PEG 8000 | Beads retained functionality and did not aggregate or degrade; viscosity was adequate. |
| PEG 10000 | Beads retained functionality and did not aggregate or degrade; PEG 10000 was slow to dissolve in final formulation. |
| PEG 20000 | Beads retained functionality and did not aggregate or degrade; PEG 20000 was slow to dissolve in final formulation. |

Excipient

Solubilizing agents, such as urea and guanidinium salts (e.g., guanidine thiocyanate and guanidine hydrochloride), both of which are chaotropic agents, as well as buffering agents, such as TAPS, HEPES and MOPS, worked well as excipients in the candidate formulations. Guanidinium salts worked similarly well to urea, but led to slightly faster bead degradation compared to urea. Buffers, such as TAPS, HEPES and MOPS, can be used to control the pH during use of the beads (e.g., during DNA or RNA isolation). Agents that were found to not be suitable as excipients include those listed in **Table 5,** below. In general, the agents in **Table 5** resulted in issues with bead stability and/or functionality.

*Table 5: Summary of agents not suitable as excipients in formulations.*

| Agent |
|---|
| • Di- and tri-carboxylic acids (citric acid, oxalic acid) |
| • Amino acids (arginine, glycine, tyrosine, tryptophane, phenylalanine, proline) |
| • Carboxylic acids salts (sodium citrate, sodium oxalate, sodium/potassium/ammonium propionate, sodium/potassium/ammonium hexanate) |
| • EDTA and its sodium salt |
| • Metal oxides (zinc oxide, titanium oxide) |
| • Sugars (trehalose, sucrose, mannitol, raffinose, glucose, lactose) |
| • Dextran |
| • PVP-40 |
| • PVA |
| • Heparin |
| • PEI |

Based on the results described above, a formulation containing a mobile phase composed of a suspending agent (e.g., glycerol, propylene glycol, PEG 200, PEG 300, or sulfolane), a viscosity enhancer (e.g., a PEG compound, such as PEG 4000, PEG 8000, PEG 10000, or PEG 20000), and a solubilizing agent (e.g., a chaotropic agent such as urea or guanidium salts, or buffering agents) was selected for further analysis.

***Example 2: Determination of suitable amounts of a mobile phase, a viscosity enhancer, and a solubilizing agent in liquid formulations for storage and handling of particles.***

This Example describes the results of experiments to determine suitable ratios of a mobile phase composed of a suspending agent (e.g., glycerol, propylene glycol, PEG 200, PEG 300, or sulfolane), a viscosity enhancer (e.g., a PEG polymer such as PEG 4000, PEG 8000, PEG 10000, or PEG 20000), and a solubilizing agent (e.g., a chaotropic agent such as urea or guanidium salts, or buffering agents) in liquid formulations for storage and handling of particles, such as magnetic particles.

**Materials and Methods**

*Preparation of candidate formulations*

To assess liquid formulations for storing and facilitating handling of particles, magnetic silica beads for nucleic acid isolation were used as exemplary particles, glycerol was used as an exemplary suspending agent in the mobile phase, the hydrophilic polymer PEG 8000 was used as an exemplary viscosity enhancer, and the chaotropic agent urea was used as an exemplary solubilizing agent.

To determine suitable ratios of each of the components, candidate formulations were prepared with a glycerol concentration ranging from 70-90% (weight by weight; w/w), a PEG 8000 concentration ranging from 0-30% (w/w), and a urea concentration ranging from 0-30% (w/w). *See,* **Table 6.**

*Table 6. Candidate formulations.*

| Formulation | Glycerol concentration (%, w/w) | PEG 8000 concentration (%, w/w) | Urea concentration (%, w/w) |
|---|---|---|---|
| A | 70 | 30 | 0 |
| B | 70 | 25 | 5 |
| C | 70 | 20 | 10 |
| D | 70 | 15 | 15 |
| E | 70 | 10 | 20 |
| F | 70 | 5 | 25 |
| G | 70 | 0 | 30 |
| H | 80 | 20 | 0 |
| I | 80 | 15 | 5 |
| J | 80 | 10 | 10 |
| K | 80 | 5 | 15 |
| L | 80 | 0 | 20 |
| M | 90 | 10 | 0 |
| N | 90 | 5 | 5 |
| O | 90 | 0 | 10 |

The candidate formulations were prepared according to the methods described in Example 1, above, using DMSO as the organic solvent during the dehydration step (1) and during the step of reducing solvent content and increasing viscosity (2). The candidate formulations were prepared with beads at a weight by volume (w/v) concentration of about 10%.

*Assessment of physical properties of candidate formulations*

Candidate formulations A-O (**Table 6**) were assessed for viscosity based on adherence to a polypropylene surface, ability to manipulate the formulations (e.g., by pipetting), and dissolution in water.

**Results**

**Table 7** shows the results of testing of candidate formulations A-O.

*Table 7: Tested candidate liquid formulations.*

| Formulation | Glycerol concentration (%, w/w) | PEG 8000 concentration (%, w/w) | Urea concentration (%, w/w) | Characteristics |
|---|---|---|---|---|
| A | 70 | 30 | 0 | High viscosity leading to difficult manipulation; strong adhesion to polypropylene. |
| B | 70 | 25 | 5 | |
| C | 70 | 20 | 10 | |
| D | 70 | 15 | 15 | Adequate viscosity; relatively slow dissolution in contact with water. |
| E | 70 | 10 | 20 | |
| F | 70 | 5 | 25 | Formulation was too dry, likely due to insufficient glycerol or PEG 8000. |
| G | 70 | 0 | 30 | |
| H | 80 | 20 | 0 | Relatively high viscosity, but manipulation of the formulation was possible. |
| I | 80 | 15 | 5 | Adequate viscosity; relatively slow dissolution; adequate adhesion to polypropylene. |
| J | 80 | 10 | 10 | |
| K | 80 | 5 | 15 | Adequate viscosity, dissolution, adhesion to polypropylene, and manipulabilitv. |
| L | 80 | 0 | 20 | Insufficient viscosity |
| M | 90 | 10 | 0 | Insufficient viscosity likely due to low PEG and/or urea concentrations; adhesion to polypropylene less than optimal. |
| N | 90 | 5 | 5 | |
| O | 90 | 0 | 10 | |

Based on the results in **Table 7,** formulation K, which includes glycerol, PEG 8000 and urea in a 16:1:3 ratio (*i.e.*, 80% glycerol, 5% PEG 8000 and 15% urea, w/w), was selected as an exemplary formulation for further testing.

***Example 3: Characterization of a liquid formulation for enhanced storage and handling of particles.***

This Example describes the results of experiments that characterized the viscosity, density, and stability of exemplary formulation K from **Table 7** in Example 2, above.

**Materials and Methods**

*Preparation of formulation K*

Formulation K, containing glycerol, PEG 8000 and urea in a 16:1:3 ratio (w/w) (*i.e.*, 80% glycerol, 5% PEG 8000 and 15% urea, w/w), was prepared using the method described in Example 1, above, with DMSO as the organic solvent during the dehydration step and during the step of reducing solvent content and increasing viscosity. As formulated, a 5 μL volume of the formulation contained about 0.5 mg ($\pm$ 10%) of exemplary magnetic silica beads.

*Viscosity and density*

Viscosity of formulation K was assessed using a ViscoQC 300-R dynamometric viscosimeter equipped with a CC12-type spindle. Viscosity measurements were conducted at temperatures between 15-60°C in 5°C increments at a constant spindle speed.

To assess density, a one milliliter (mL) volume of the formulation was weighed on an analytical balance. Ten replicates of the density measurements were performed at about 22°C.

*Stability*

The stability of the beads in formulation K was determined based on binding to RNA following storage at room temperature (about 25°C) for 3, 4.5, 6, 9, 12, and 15 months. The beads were stored in a polypropylene chamber either under standard atmosphere or in reduced oxygen conditions following flushing of the chamber with nitrogen.

RNA binding was assessed as follows:

Binding of RNA to beads

400 $\mu$L of eNat substrate (Copan Diagnostics, Italy), 600 $\mu$L of SA1 buffer (0.7 M sodium acetate, pH 4.9) and 500 $\mu$L of SBL buffer (75% isopropanol) were mixed in a microcentrifuge tube. 3 $\mu$L of an exemplary RNA target at $10^7$ copies (cp)/$\mu$L was then added to the tube. 6 $\mu$L of formulation K containing exemplary magnetic silica beads were added to the microcentrifuge tube and mixed. The mixture was vortexed for 30 seconds, and then incubated for 4 minutes on a lab bench.

Wash and elution

The beads were separated from solution using a magnet, and the beads were subsequently washed twice with wash buffer (10 mM glycine pH 2.0), each time adding 500 $\mu$l of wash buffer, stirring on a vortex for 5 seconds, and separating the beads from the wash buffer with a magnet. After the second wash, the samples were centrifuged using a short spin on a microcentrifuge, followed by separation of the beads from the wash buffer using a magnet. As much of the wash buffer supernatant as possible was removed with a pipette. RNA was eluted from the beads by adding 100 $\mu$l of elution buffer (Tris 30mM pH 8.8), mixing on a vortex for 15 seconds, incubating for 3 minutes on a laboratory bench, and separating the particles from the eluate using a magnet. The eluate was then recovered and moved to a fresh tube.

Analysis of RNA recovery by RT-qPCR

The recovery of RNA was assessed using reverse transcription quantitative PCR (RT-qPCR) on a LightCycler 480 II analyzer (Roche). Three PCR replicates were prepared for each eluate sample. A positive control using input RNA ($10^7$ cp/$\mu$L in a final volume of 100 $\mu$L with elution buffer), a negative control with only elution buffer, and beads in the manufacturer's aqueous suspension buffer ('wet beads') were also analyzed. The components of the PCR mixture are shown in **Table 8.**

*Table 8: RT-qPCR reaction master mixture.*

| Component | Amount for 45 reactions ($\mu$l) |
| --- | --- |
| Tris 30mM pH 8.8 | 393.6 |
| 2M KCl | 14.4 |
| Forward primer (100 $\mu$M) | 12 |
| Reverse primer (100 $\mu$M) | 12 |
| Lyophilized enzyme mix & Eva Green | 8 |

The RT-qPCR program was as follows:

- Reverse transcription (RT): 58°C for 30 seconds
- Activation: 99°C for 2 seconds
- Amplification: (50x) 95°C for 1 second, 63°C for 10 seconds
- Melting: 65°C for 5 seconds, 98°C + 5°C for 11 seconds
- Cooling: 37°C for 1 minute

The RT-qPCR reactions were prepared and analyzed as follows:

1. 9 μl of reaction mix was added to wells of a PCR plate.
2. 1 μl of eluate, positive control (input), or negative control was added to the wells.
3. The plate was sealed with PCR film and spun.
4. Reactions were run on a LightCycler 480 II analyzer (Roche).
5. Isolation efficiency was calculated assuming a PCR reaction efficiency of 1.9 according to the following formula:

$$W = 1.9^{(Ct^{wet} - Ct^{sample})} \cdot 100\%,$$

wherein $Ct^{wet}$ is the average cycle threshold (Ct) of beads stored in the manufacturer's aqueous storage solution and $Ct^{sample}$ is the average Ct of the test sample.

**Results**

*Viscosity and density*

High viscosity and density have the desirable effect of preventing settling of particles in a suspension, and may also contribute to stability of the particles, thus facilitating handling, aliquoting and storage of a particle suspension.
The viscosity of formulation K at various ambient temperatures ranging from 15°C to 60°C is provided in **Table 9**.

*Table 9. Viscosity of formulation K.*

| Dynamometric viscosity (mPa·s) | Speed (rpm) | Torque (%) | Duration (hh:mm:ss) | Temperature (°C) | Shearing speed (1/s) | Shear stresses (N/m²) |
|---|---|---|---|---|---|---|
| 7848 | 10.0 | 64.7 | 00:00:30 | 15.0 | 12.91 | 101.3 |
| 5628 | 10.0 | 46.4 | 00:00:30 | 20.0 | 12.91 | 72.65 |
| 6235 | 10.0 | 51.4 | 00:00:30 | 25.0 | 12.91 | 80.48 |
| 4840 | 10.0 | 39.9 | 00:00:30 | 30.0 | 12.91 | 62.47 |
| 2402 | 10.0 | 19.8 | 00:00:30 | 35.0 | 12.91 | 31.00 |
| 1237 | 10.0 | 10.2 | 00:00:30 | 40.0 | 12.91 | 15.97 |
| 1104 | 10.0 | 9.1 | 00:00:30 | 45.0 | 12.91 | 14.25 |
| 800.6 | 10.0 | 6.6 | 00:00:30 | 50.0 | 12.91 | 10.33 |
| 1116 | 10.0 | 9.2 | 00:00:30 | 55.0 | 12.91 | 14.40 |
| 303.3 | 10.0 | 2.5 | 00:00:30 | 60.0 | 12.91 | 3.914 |

The density of formulation K was determined to be about 1.3 g/mL ($\pm$ 0.2 g/mL).

*Stability*

The average Ct values and RNA isolation efficiencies for beads stored in formulation K, either under standard atmosphere conditions or reduced oxygen conditions, for 3, 4.5, 6, 9, 12, and 15 months are provided in **Table 10**. Isolation efficiencies were calculated relative to the beads stored in the manufacturer's aqueous suspension buffer (termed 'wet beads' below).

*Table 10. Ct values and RNA isolation efficiencies at 3, 4.5, 6, 9, 12, and 15 months.*

| 3 months storage at room temperature | | | |
|---|---|---|---|
| | Sample replicates | Average Ct | Isolation efficiency |
| | 1 | 24.76 | 182% |
| | 2 | 25.25 | 129% |
| | 3 | 24.79 | 178% |
| | 4 | 25.15 | 139% |
| Formulation stored in atmospheric conditions | 5 | 24.68 | 192% |
| | Wet beads | 25.62 | 100% |
| 4.5 months storage at room temperature | | | |
| | Sample replicates | Average Ct | Isolation efficiency |
| | 1 | 25.97 | 104% |
| | 2 | 25.59 | 136% |
| | 3 | 25.71 | 125% |
| | 4 | 25.76 | 121% |
| Formulation stored in atmospheric conditions | 5 | 25.9 | 109% |
| | 1 | 25.8 | 117% |
| | 2 | 25.79 | 118% |
| | 3 | 25.9 | 109% |
| | 4 | 25.83 | 115% |
| Formulation stored in oxygen depleted atmosphere | 5 | 25.9 | 109% |
| | Wet beads | 26.03 | 100% |
| 6 months storage at room temperature | | | |
| | Sample replicates | Average Ct | Isolation efficiency |
| | 1 | 24.88 | 127% |
| | 2 | 24.68 | 145% |
| | 3 | 24.84 | 130% |
| | 4 | 25.11 | 108% |
| Formulation stored in atmospheric conditions | 5 | 24.7 | 143% |
| | 1 | 24.66 | 147% |
| | 2 | 24.87 | 127% |
| | 3 | 24.69 | 144% |
| | 4 | 24.81 | 133% |
| Formulation stored in oxygen depleted atmosphere | 5 | 24.91 | 124% |
| | Wet beads | 25.22 | 100% |

(continued)

| 9 months storage at room temperature | | | |
|---|---|---|---|
| | Sample replicates | Average Ct | Isolation efficiency |
| | 1 | 21.62 | 110% |
| | 2 | 21.53 | 117% |
| | 3 | 21.51 | 119% |
| | 4 | 21.37 | 131% |
| Formulation stored in atmospheric conditions | 5 | 21.59 | 113% |
| | 1 | 21.55 | 116% |
| | 2 | 21.16 | 152% |
| | 3 | 21.48 | 121% |
| | 4 | 21.54 | 116% |
| Formulation stored in oxygen depleted atmosphere | 5 | 21.47 | 122% |
| | Wet beads | 21.76 | 100% |
| 12 months storage at room temperature | | | |
| | Sample replicates | Average Ct | Isolation efficiency |
| | 1 | 21.59 | 135% |
| | 2 | 21.38 | 156% |
| | 3 | 21.76 | 120% |
| | 4 | 21.62 | 132% |
| Formulation stored in atmospheric conditions | 5 | 21.72 | 123% |
| | 1 | 21.6 | 134% |
| | 2 | 21.77 | 119% |
| | 3 | 21.47 | 146% |
| Formulation stored in oxygen depleted atmosphere | 4 | 21.67 | 127% |
| | 5 | 21.63 | 131% |
| | Wet beads | 22.02 | 100% |
| 15 months storage at room temperature | | | |
| | Sample replicates | Average Ct | Isolation efficiency |
| | 1 | 21.59 | 135% |
| | 2 | 21.38 | 156% |
| | 3 | 21.76 | 120% |
| | 4 | 21.62 | 132% |
| Formulation stored in atmospheric conditions | 5 | 21.72 | 123% |
| | 1 | 21.6 | 134% |
| | 2 | 21.77 | 119% |
| | 3 | 21.47 | 146% |
| | 4 | 21.67 | 127% |
| Formulation stored in oxygen depleted atmosphere | 5 | 21.63 | 131% |
| | Wet beads | 22.02 | 100% |

As shown in **Table 10,** unexpectedly, beads stored in formulation K were stable and retained RNA-binding ability through at least 15 months of storage under both standard atmosphere and reduced oxygen conditions, even though formulation K provides relatively harsh conditions (e.g., relatively high amounts of urea). Formulation K also surprisingly resulted in improved RNA isolation efficiencies, e.g., by at least about 10%, compared to beads stored in the manufacturer's suspension buffer. *See,* also, **FIG. 1.** Furthermore, formulation K displayed excellent microbiological stability during prolonged storage at room temperature.

## Conclusions

The results described in this Example showed that beads stored in formulation K were stable and functional for unexpectedly long periods of time, e.g., at least 15 months, when stored in standard atmosphere or reduced oxygen conditions at room temperature. Formulation K also surprisingly resulted in improved RNA isolation efficiencies and had excellent microbiological stability. Furthermore, formulation K has high viscosity and density, which can facilitate handling and aliquoting of the beads by inhibiting settling and may also contribute to stability of the beads. Thus, formulation K allows for long-term storage of beads in ambient conditions, while also facilitating the handling and aliquoting of the beads, including by automated systems.

## Claims

1. A composition comprising a plurality of particles and

 (a) a mobile phase comprising a suspending agent;
 (b) a viscosity enhancing agent; and
 (c) a solubilizing agent.

2. The composition of claim 1, wherein:

 (a) the mobile phase is at a concentration of between about 60% and about 99%, between about 65% and about 95%, between about 70% and about 90%, between about 75% and about 90%, or between about 80% and about 90% weight by weight (w/w);
 (b) the suspending agent is selected from the group consisting of a sugar alcohol, a glycol, a water-miscible polymer, an aprotic solvent, and any combination thereof; and/or
 (c) the mobile phase is non-aqueous or substantially non-aqueous.

3. The composition of claim 1 or claim 2, wherein:

 (a) the viscosity enhancing agent is at least partially soluble in the mobile phase;
 (b) the viscosity enhancing agent is at a concentration of between about 1% and about 40%, between about 1% and about 35%, between about 1% and about 30%, between about 1% and about 25%, between about 1% and about 20%, between about 1% and about 15%, between about 1% and about 10%, or between about 1% and about 5% w/w; and/or
 (c) the viscosity enhancing agent comprises a water-miscible polymer.

4. The composition of any one of claims 1-3, wherein:

 (a) the solubilizing agent is at least partially soluble in the mobile phase and/or is water-soluble;
 (b) the solubilizing agent is at a concentration of between about 1% and about 40%, between about 5% and about 30%, between about 10% and about 25%, or between about 10% and about 20% w/w; and/or
 (c) the solubilizing agent comprises a chaotropic agent or a buffering agent.

5. The composition of any one of claims 1-4, wherein the suspending agent comprises glycerol, the viscosity enhancing agent comprises PEG 8000, and the solubilizing agent comprises urea; optionally wherein:

 (i) the glycerol is at a concentration of about 80% w/w, the PEG 8000 is at a concentration of about 5% w/w, and the urea is at a concentration of about 15% w/w, and/or
 (ii) the ratio of glycerol:PEG 8000:urea in the composition is 16:1:3 w/w.

6. The composition of any one of claims 1-5, wherein:

(a) the composition has a viscosity of between about 5500 mPa·s and about 7000 mPa s, between about 5750 mPa·s and about 6750 mPa·s, or between about 6000 mPa·s and about 6500 mPa·s, when measured at room temperature optionally at a temperature of about 25°C, optionally wherein viscosity of the composition is assessed using a rotational viscometer, further optionally wherein viscosity of the composition is assessed at a spindle rotational speed of about 10 revolutions per minute (RPM);

(b) the composition has a density of between about 0.5 g/mL and about 2 g/mL, between about 1 g/mL and about 2 g/mL, between about 1 g/mL and about 1.5 g/mL, or between about 1.1 g/mL and about 1.5 g/mL, optionally wherein density of the composition is assessed on an analytical balance;

(c) the composition is non-aqueous or substantially non-aqueous, optionally wherein the composition has a water content of about 1% or less;

(d) the composition enhances stability of particles in the plurality of particles as compared to corresponding particles in an aqueous solution, optionally wherein particle stability is assessed based on particle integrity, particle aggregation, and/or retention of ability to bind to a ligand;

(e) the plurality of particles in the composition is stable for at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, or at least about 15 months, optionally wherein particle stability is assessed based on particle integrity, particle aggregation, and/or retention of ability to bind to a ligand;

(f) the composition is capable of adhering to a polypropylene surface;

(g) the composition is microbiologically stable;

(h) the composition further comprises an agent selected from the group consisting of: an antioxidant, a reducing agent, a chelating agent, and any combination thereof; and/or

(i) the composition does not comprise an anti-microbial agent, and/or comprises less than about 10% of surfactant or detergent.

7. The composition of any one of claims 1-6, wherein the plurality of particles:

(a) comprises one or more particles configured to bind a nucleic acid, a polypeptide, a lipid, a carbohydrate, or a small molecule;

(b) comprises one or more microparticles, nanoparticles, microspheres, paramagnetic beads, magnetic beads, microbeads, nanobeads, or any combination thereof;

(c) comprises one or more magnetic or paramagnetic particles; and/or

(d) is present in the composition at a concentration of at least about 0.5%, at least about 1%, at least about 2.5%, at least about 5%, at least about 10%, at least about 15%, at least about 20%, at least about 25%, or at least about 30% weight by volume (w/v); or at a concentration of between about 0.5% and about 30% w/v, between about 1% and about 25% w/v, between about 2.5% and about 20% w/v, or between about 5% and about 15% w/v.

8. A method for preparing a composition comprising a plurality of particles, the method comprising:

(c) providing a plurality of particles; and

(d) suspending the plurality of particles in a formulation comprising:

i. a mobile phase comprising a suspending agent;

ii. a viscosity enhancing agent; and

iii. a solubilizing agent.

9. The method of claim 8, wherein:

(1) the plurality of particles provided in step (a) is dehydrated or lyophilized; or

(2) the method further comprises dehydrating or lyophilizing the plurality of particles prior to step (a),

optionally wherein the dehydrating comprises suspending the plurality of particles in a first organic solvent, further optionally wherein the first organic solvent comprises an organic solvent miscible with water and/or with high solubility in water, and further optionally wherein the first organic solvent comprises a polar aprotic solvent, a water miscible P-type or E-type glycol ether, an alcohol, tetrahydrothiophene 1-oxide, or any combination thereof.

10. The method of claim 9, wherein:

(1) step (b) comprises: (i) separating the plurality of particles from a volume of the first organic solvent, and (ii) suspending the separated plurality of particles in a volume of said formulation;
(2) the method further comprises, prior to step (b), suspending the plurality of particles in a solution comprising: a second organic solvent and a suspending agent, optionally wherein step (b) comprises: (i) separating the plurality of particles from said solution comprising the second organic solvent and the suspending agent, and (ii) suspending the separated plurality of particles in a volume of said formulation; and/or
(3) the method further comprises, prior to step (b): (i) separating the plurality of particles from a volume of the first organic solvent, and (ii) suspending the separated plurality of particles in a solution comprising: a second organic solvent and a suspending agent, optionally wherein step (b) comprises (i) separating the plurality of particles from said solution comprising the second organic solvent and the suspending agent, and (ii) suspending the separated plurality of particles in a volume of said formulation;

optionally wherein:

(i) the second organic solvent and the suspending agent in the solution are in about a 1:1 ratio;
(ii) the suspending agent is selected from the group consisting of a sugar alcohol, a glycol, a water-miscible polymer, an aprotic solvent, and any combination thereof;
(iii) the second organic solvent comprises an organic solvent miscible with water and/or with high solubility in water; and/or
(iv) the second organic solvent comprises a polar aprotic solvent, a water miscible P-type or E-type glycol ether, an alcohol, or tetrahydrothiophene 1-oxide.

11. A composition comprising a plurality of particles produced by the method of any one of claims 8-10.

12. A method for isolating an analyte from a sample, comprising:

(a) providing a sample comprising an analyte;
(b) mixing the sample with the composition comprising a plurality of particles of any one of claims 1-7 and 11, or a composition comprising a plurality of particles produced by the method of any one of claims 8-10, to produce a mixture of the sample and the composition, thereby binding said analyte to one or more particles in the plurality of particles; and
optionally (c) separating the plurality of particles from the mixture.

13. A method for cell lysis, comprising:

(a) providing a sample comprising a plurality of cells;
(b) mixing the sample with the composition comprising a plurality of particles of any one of claims 1-7 and 11, or a composition comprising a plurality of particles produced by the method of any one of claims 8-10, thereby producing a mixture of the sample and the composition; and
(c) agitating the mixture, thereby lysing one or more cells in the plurality of cells.

14. A kit comprising the composition of any one of claims 1-7 and 11, or a composition produced by the method of any one of claims 8-10, optionally wherein the kit further comprises:

(a) instructions for use of the composition for isolating one or more analytes from a sample, further optionally wherein the instructions are for isolating the one or more analytes from the sample according to the method of claim 12; or
(b) instructions for use of the composition for lysing one or more cells from a sample, optionally wherein the instructions are for lysing the one or more cells according to the method of claim 13.

15. A microfluidic device comprising the composition of any one of claims 1-7 and 11, or a liquid composition produced by the method of any one of claims 8-10, optionally wherein the device is for use according to the method of claim 12 or claim 13.

## EUROPEAN SEARCH REPORT

Europäisches Patentamt

European Patent Office

Office européen des brevets

Application Number

EP 23 46 1676

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/170893 A1 (FERRY ANNE-LAURE SOPHIE [GB] ET AL) 4 June 2020 (2020-06-04) <br> * in the table, top of page 5.; example 2 * | 1-11,14, 15 | INV. <br> B01J20/281 <br> B01D15/00 <br> B01J20/283 <br> B01J20/292 |
| X | EP 0 978 522 A1 (NAT STARCH CHEM INVEST [US]) 9 February 2000 (2000-02-09) <br> * example 26 * | 1-7,11, 14,15 | B01J20/30 <br> C12N15/10 |
| X | US 8 858 976 B2 (TUOMINEN JUKKA [FI]; LEHTONEN TIMO [FI] ET AL.) 14 October 2014 (2014-10-14) <br> * example 2 * | 1-11,14, 15 | |
| X | EP 0 802 975 B1 (VICAL INC [US]) 26 March 2003 (2003-03-26) <br> * example 1 * | 1-15 | |
| A | EP 1 856 262 B1 (MERCK SHARP & DOHME [US]) 15 August 2012 (2012-08-15) <br> * example 8 * | 1-15 | |

TECHNICAL FIELDS SEARCHED (IPC)

B01J
B01D
C12N

The present search report has been drawn up for all claims

| Place of search | Date of completion of the search | Examiner |
|---|---|---|
| The Hague | 15 May 2024 | Mc Donnell, Shane |

EPO FORM 1503 03.82 (P04C01)

## ANNEX TO THE EUROPEAN SEARCH REPORT
## ON EUROPEAN PATENT APPLICATION NO.

EP 23 46 1676

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2024

| Patent document cited in search report | Publication date | Patent family member(s) | Publication date |
|---|---|---|---|
| US 2020170893 A1 | 04-06-2020 | BR 112020003636 A2 | 01-09-2020 |
| | | EP 3672562 A1 | 01-07-2020 |
| | | US 2020170893 A1 | 04-06-2020 |
| | | WO 2019038101 A1 | 28-02-2019 |
| EP 0978522 A1 | 09-02-2000 | AU 770290 B2 | 19-02-2004 |
| | | CA 2279701 A1 | 06-02-2000 |
| | | EP 0978522 A1 | 09-02-2000 |
| | | JP 2000136228 A | 16-05-2000 |
| | | KR 20000017079 A | 25-03-2000 |
| US 8858976 B2 | 14-10-2014 | AU 2010317959 A1 | 24-05-2012 |
| | | BR 112012011286 A2 | 12-04-2016 |
| | | CN 102711714 A | 03-10-2012 |
| | | EP 2322134 A1 | 18-05-2011 |
| | | JP 5763665 B2 | 12-08-2015 |
| | | JP 2013510611 A | 28-03-2013 |
| | | US 2012276164 A1 | 01-11-2012 |
| | | WO 2011058134 A1 | 19-05-2011 |
| EP 0802975 B1 | 26-03-2003 | AT E235547 T1 | 15-04-2003 |
| | | AT E419341 T1 | 15-01-2009 |
| | | DE 69626957 T2 | 04-03-2004 |
| | | DK 0802975 T3 | 14-07-2003 |
| | | EP 0802975 A1 | 29-10-1997 |
| | | EP 1291421 A1 | 12-03-2003 |
| | | ES 2196140 T3 | 16-12-2003 |
| | | JP 3808506 B2 | 16-08-2006 |
| | | JP H11502403 A | 02-03-1999 |
| | | US 5576196 A | 19-11-1996 |
| | | WO 9621729 A1 | 18-07-1996 |
| EP 1856262 B1 | 15-08-2012 | AU 2006211216 A1 | 10-08-2006 |
| | | CA 2595594 A1 | 10-08-2006 |
| | | CN 101310022 A | 19-11-2008 |
| | | EP 1856262 A2 | 21-11-2007 |
| | | JP 4971997 B2 | 11-07-2012 |
| | | JP 2008528035 A | 31-07-2008 |
| | | US 2008138886 A1 | 12-06-2008 |
| | | WO 2006083721 A2 | 10-08-2006 |

EPO FORM P0459

For more details about this annex : see Official Journal of the European Patent Office, No. 12/82